# EUROPEAN PATENT SPECIFICATION

(11) **EP 0 365 997 B1**
(45) Date of publication and mention of the grant of the patent: **14.09.1994**
(21) Application number: 89119361.7
(22) Date of filing: 18.10.1989
(51) Int. Cl.: C12N 15/13, C12N 5/10, C12P 21/08, A61K 39/395

(54) **A novel family of high affinity, modified antibodies for cancer treatment**
Familie von modifizierten Antikörpern mit hoher Affinität zur Behandlung von Krebs
Famille d'anticorps modifiés à haute affinité pour le traitement du cancer

(30) Priority: 19.10.1988 US 259943
(43) Date of publication of application: 02.05.1990
(73) Proprietor: THE DOW CHEMICAL COMPANY, Midland, Michigan 48640 (US)
(72) Inventor: Mezes, Peter, Midland Michigan 48640 (US); Gourlie, Brian, Midland Michigan 48640 (US); Rixon, Mark, Midland Michigan 48640 (US)
(74) Representative: Huber, Bernhard, Dipl.-Chem.

(56) References cited:
- WO-A-89/01783
- US-A- 7 073 685
- CHEMICAL ABSTRACTS, vol. 110, no. 13, 27th March 1989, page 546, abstract no. 113055e, Columbus, Ohio, US; & US-A-73 685 (UNITED STATES DEPT. OF HEALTH AND HUMAN SERVICES) 01-03-1988 & WO-A-89 00 692 (26-01-1989)
- PROTEIN ENGINEERING, vol. 1, no. 6, 4th April 1987, pages 499-505, IRL Press Ltd, Oxford, GB; N. WHITTLE et al.: "Expression in COS cells of a mouse-human chimaeric B72.3 antibody"
- Cell Engineering (1985) 4(12): 1025-1035
- Nature (1983) 301 : 720-722
- The Journal of Immunology (1985) 134(4) : 2544-2549

## Description

This invention relates to the field of immunoglobulin production and modifications to naturally occurring antibody amino acid sequences. Specifically, the invention relates to using recombinant DNA techniques to produce chimeric genes and to take advantage of these gene modification techniques to construct chimeric antibodies.

Antibodies are specific immunoglobulin (Ig) polypeptides produced by the vertebrate immune system in response to challenges by foreign proteins, glycoproteins, cells or other antigenic foreign substances. The sequence of events which permits the organism to overcome invasion by foreign cells or to rid the system of foreign substances is at least partially understood. An important part of this process is the manufacture of antibodies which bind specifically to a particular foreign substance. The binding specificity of such polypeptides to a particular antigen is highly refined, and the multitude of specificities capable of being generated by the individual vertebrate is remarkable in its complexity and variability. Millions of antigens are capable of eliciting antibody responses, each antibody almost exclusively directed to the particular antigen which elicited it.

Two major sources of vertebrate antibodies are Presently utilized--generation *in situ* by the mammalian B lymphocytes, and generation in cell culture by B-cell hybrids. Antibodies are generated *in situ* as a result of the differentiation of immature B lymphocytes into plasma cells, which occurs in response to stimulation by specific antigens. In the undifferentiated B cells, the portions of DNA coding for the various regions on the immunoglobulin chains are separated in the genomic DNA. The sequences are assembled sequentially prior to expression. A review of this process has been given by Gough, *Trends in Biochem. Sci.* 6. 203 (1981).

The resulting rearranged gene is capable of expression in the mature B lymphocyte to produce the desired antibody. However, even when a particular mammal is exposed to only a single antigen a uniform population of antibodies does not result. The *in situ* immune response to any particular antigen is defined by the mosaic of responses to the various determinants which are present on the antigen. Each subset of homologous antibodies is contributed by a single population of B cells--hence *in situ* generation of antibodies is "polyclonal".

This limited but inherent heterogeneity has been overcome in numerous particular cases by use of hybridoma technology to create "monoclonal" antibodies in cell cultures by B cell hybridomas [See Kohler and Milstein, C., *Nature* 256, 495-497 (1975)].

In this process, the relatively short-lived, or mortal, splenocytes or lymphocytes from a mammal which has been injected with antigen are fused with an immortal tumor cell line, thus producing hybrid cells or "hybridomas" which are both immortal and capable of producing the genetically coded antibody of the B cell. The hybrids thus formed are segregated into single genetic strains by selection, dilution, and regrowth, and each strain thus represents a single genetic line. They therefore produce antibodies which are assured to be homogeneous against a desired antigen. These antibodies, referencing their pure genetic parentage, are called "monoclonal".

Monoclonal antibodies with mono-specificity have greatly influenced immunology, and their usefulness has already been demonstrated in such sciences as biology, pharmacology, chemistry and others. Such monoclonal antibodies have found widespread use not only as diagnostics reagents [see, for example, *Immunology for the 80's*, Eds. Voller, A., Bartlett, A., and Bidwell, D., MTP Press, Lancaster, (1981), but also therapy (see, for example, Ritz, J. and Schlossman, S.F., *Blood* 59, 1-11, (1982)].

Monoclonal antibodies produced by hybridomas, while theoretically effective as discussed above and clearly preferable to polyclonal antibodies because of their specificity, suffer from an important disadvantage. In many applications, the use of monoclonal antibodies produced in non-human animals is severely restricted where the monoclonal antibodies are to be used in humans. Repeated injections of a "foreign" antibody in humans, such as a mouse antibody, may lead to harmful hypersensitivity reactions. Such a non-human derived monoclonal antibody, when injected into humans, causes a anti-nonhuman antibody (ANHA) response. For a discussion of a specific ANHA response caused by using murine-derived antibodies, human anti-mouse antibody (HAMA) response, see Shawler et al., *Journal of Immunology* 135, 1530-1535 (1985).

It is believed that animal immunoglobulins having human constant regions will generate less of a ANHA response when injected into humans than animal immunoglobulins having nonhuman constant regions. As such, monoclonal antibodies having good binding affinities for selected antigens and having human constant regions are thought to possess great potential utility for immunological diagnosis and therapy of human patients with cancer.

Various attempts have so far been made to manufacture human-derived monoclonal antibodies by using human hybridomas. For example, human-human hybridomas [Olsson, L. et al., *Proc. Natl. Acad. Sci. (USA)*, 77, 5429 (1980)]; human-murine hybridomas [(Schlom, J., et al. (*ibid*) 77, 6841 (1980)] and several other xenogenic hybrid combinations have been prepared. Human monoclonal antibodies have also been produced by transformation of lymphocytes using Epstein-Barr virus. However, such hybridomas may potentially harbor pathogenic human viruses. Alternatively, primary, antibody producing B cells have been immortalized *in vitro* by transformation with viral DNA. Unfortunately, yields of monoclonal antibodies from human hybridoma cell lines are relatively low (1 µg/mL in human compared to 100 µg/mL in mouse hybridomas), and production costs are high.

While human immunoglobulins are highly desirable in immunological diagnosis and therapy of human cancer patients, human hybridoma techniques have not yet reached the stage where human monoclonal antibodies with required antigenic specificities can be easily obtained. In addition, for obvious ethical reasons, researchers can not immunize human subjects with selected toxic or otherwise deleterious antigens to generate antibodies against the specific antigen. This imposes great restrictions on immunological diagnosis and therapy of human patients.

The production of human-derived monoclonal antibodies is certainly possible, but is still inefficient in view of its low reproducibility and the other problems noted above. [Additionally, see *Nature* 300, 316-317 (1982)]. Consequently, most monoclonal antibodies are derived from non-human animals.

A monoclonal antibody which reacts with high binding affinity to human tumor antigens, but which is not recognized as a foreign substance by humans is highly desirable. A method to overcome this difficulty is to create artificially an antibody which is very similar to a human antibody and is not recognized as a foreign substance within the human body, i.e., a chimeric, or "humanized" antibody.

Typically in chimeric antibodies, the variable region of both light and heavy chains mimics the variable regions of antibodies derived from one species of mammals, while the constant portions are homologous to the sequences in antibodies derived from humans. One clear advantage to such chimeric forms is that, for example, the variable regions can conveniently be derived from presently known sources using readily available hybridomas of B cells from non-human host organisms in combination with constant regions derived from, for example, human cell preparations. While the specificity of the varible region is not affected by its source, the constant region being human, is less likely to elicit an immune response from a human subject when the antibodies are injected than would the constant region from a non-human source.

One known human tumor antigen is tumor-associated glycoprotein (TAG72). TAG72 is associated with the surface of certain tumor cells of human origin, specifically the LS174T tumor cell line. LS174T [American Type Culture Collection (herein ATCC) No.CL 188] is a variant of the LS180 (ATCC No. CT 187) colon adenocarcinoma line.

The karyotype of LS174T is similar to that of LS180 with a missing X chromosome in a majority of the cells. Data has been presented as described in Johnson, V.G. et al., *Cancer Res.* 46, 850-857 (1986), to characterize the TAG72 molecule as a mucin. This conclusion is based on the following observations: (a) TAG72 has a high molecular weight (>1 x 10⁶) as shown by its exclusion from a Sepharose™ CL-4B column; (b) the density of TAG72 determined by equilibrium centrifugation in CsCl was 1.45 gm/mL, indicating a heavily glycosylated glycoprotein; (c) TAG72 demonstrates a change in migration after neuraminidase digestion, indicating that it is a heavily sialylated molecule with an abundance of O-glycosidically linked oligosaccharides characteristic of mucins; (d) blood group antigens commonly found on mucins are found on affinity-purified TAG72; and (e) Chondroitinase ABC digestion had no effect on TAG72, thus demonstrating that the TAG72 epitope is not expressed on a chondroitin sulfate proteoglycan.

Numerous murine monoclonal antibodies have been developed which have binding specificity for TAG72. One of these monoclonal antibodies, designated B72.3, is a murine IgGl produced by hybridoma B72.3 (ATCC No. HB-8108). B72.3 is a first generation monoclonal antibody developed using a human breast carcinoma extract as the immunogen (see Colcher, D. et al., *Proc. Natl. Acad. Sci. (USA)* 78, 3199-3203 (1981); and U.S. Patents 4,522,918 and 4,612,282). As used herein, the expression "first generation monoclonal antibody" means a monoclonal antibody produced using, as the immunogen, a crude cell extract.

Other monoclonal antibodies directed against TAG72 are designated "CC" (colon cancer). CC monoclonal antibodies are a family of second generation murine monoclonal antibodies. As used herein, the expression "second generation monoclonal antibody" means a monoclonal antibody produced using, as the immunogen, an antigen purified with a first generation monoclonal antibody. CC monoclonal antibodies were prepared using TAG72 purified with B72.3. A discussion of the method for Producing the CC antibodies is set forth in United States Patent Application 7-073,685 (USPA 7-073,685), the application was filed by Schlom et al. on July 15, 1987 and is available to the public from the National Technical Information Service. Because of their relatively good binding affinities to TAG72, the following CC antibodies have been deposited at the ATCC, with restricted access having been requested: CC49 (ATCC No. HB 9459); CC83 (ATCC No. HB 9453); CC46 (ATCC No. HB 9458); CC92 (ATCC No. HB 9454); CC30 (ATCC NO. HB 9457); CC11 (ATCC No. 9455); and CC15 (ATCC No. HB 9460).

In the known art, no human antibody has been isolated which relatively strongly binds to TAG72. Consequently, suitable antibodies must be engineered.

It is known that the function of an Ig molecule is dependent on its three dimensional structure, which in turn is dependent on its primary amino acid sequence. Thus, changing the amino acid sequence of an Ig may adversely affect its activity. Moreover, a change in the DNA sequence coding for the Ig may affect the ability of the cell containing the DNA sequence to express, secrete or assemble Ig.

USPA 7-073,685 teaches that the CC antibodies may be altered into their chimeric form by substituting, e.g., human constant regions (Fc) domains for mouse constant regions by recombinant DNA techniques known in the art. It is believed that the proposals set out in USPA 7-073,685 did not lead to an actual attempt to express any chimeric Ig polypeptide chains, nor to produce Ig activity, nor to secrete and assemble Ig chains into the desired chimeric Igs.

An article by Whittle et al. in Protein Engineering 1 (1987), 499-505, describes the expressions in COS-1 cells of a mouse-human chimeric antibody, which recognizes an epitope on a tumor-associated antigene TAG-72. Chimeric immunoglobulin genes with a specificity against TAG-72 have been constructed by joining the mouse- variable regions from cDNA clones to human genomic constant regions using recombinant DNA techniques. The chimeric heavy and light chain immunoglobulin genes were placed under the control of the strong viral promoter and cotransfected into C0S-1 cells. The specific binding characteristics of the parent antibody were retained by the chimeric antibody in an antigene-based ELISA.

It is therefore not at all clear from the art that known recombinant DNA techniques will routinely produce a chimeric animal-human antibody from selected DNA sources that generate functional chimeric antibodies which bind specifically to selected human carcinomas and which reduce the initiation of ANHA side-effects when injected into humans.

Suprisingly, the present invention is able to meet many of these above mentioned needs and provides a new method for supplying the desired antibodies.

The present invention provides novel antibodies for use in *in vivo* diagnostic assays; *in vivo* therapy; and radioimmunoguided surgery.

The subject matter of the present invention is an antibody produced by one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875) or a fragment thereof, which is capable of selectively reacting with a particular antigene or antigene family.

A further subject matter of the present invention is a DNA-sequence encoding at least a portion of an antibody, which is capable of selectively reacting with a particular antigene or antigene family and being contained in one of the above-mentioned cell lines.

This invention also concerns a DNA sequence encoding at least a portion of an antibody heavy chain, said sequence comprising a DNA sequence segment as defined above being effectively homologous to the V_{H}αTAG germline gene (V_{H}αTAG), wherein the DNA sequence segment encodes at least a portion of a heavy chain variable region (V_{H}).

Further, the invention concerns a DNA sequence comprising:
(A) a sequence segment encoding for a heavy chain, said sequence segment having
   (1) a sequence subsegment being effectively homologous to the V_{H}αTAG germline gene (V_{H}αTAG), wherein the DNA sequence segment encodes at least a portion of a V_{H}, and
   (2) a sequence subsegment encoding for at least a portion of a C_{H}; and
(B) a sequence segment encoding for a light chain, said sequence segment having
   (1) a sequence subsegment encoding for at least a portion of an animal light chain variable region (V_{L}), and
   (2) a sequence subsegment encoding for at least a portion of a human light chain constant region (C_{L}),
   wherein the antibody encoded by the DNA is as defined above.

The invention further includes the aforementioned antibody alone or conjugated to an imaging marker or therapeutic agent. The invention also includes a composition comprising the aforementioned antibody in unconjugated or conjugated form in a pharmaceutically acceptable, non-toxic, sterile carrier.

Additionally, the invention also concerns process for preparing the various antibodies or antibody fragments, their conjugates, a suitable recombinant expression vehicle, and the insertion into a suitable host. Some of these processes are expressed as follows. A process for preparing an antibody or antibody fragment comprising contacting a V_{H} region with a V_{L} region to form a variable region of the antibody or antibody fragment. A process for preparing an antibody or antibody fragment conjugate comprising contacting an antibody or antibody fragment with an imaging marker or therapeutic agent. A process for preparing a recombinant expression vehicle comprising inserting a DNA sequence into an expression vehicle. A process for preparing a transformed host comprising inserting the plasmid into a suitable host.

In other aspects, the invention is directed to DNA which encodes the aforementioned antibodies and fragments thereof, as well as expression vectors or plasmids capable of effecting the production of such immunoglobulins in suitable host cells. It includes the host cells and cell cultures which result from transformation with these vectors.

### Description of the Drawings

Figure 1 illustrates a basic immunoglobulin structure, with the enzymatic cleavage sites being indicated.

Figure 2 illustrates the nucleotide sequences of V_{H}αTAG V_{H}, CC46 V_{H}, CC49 V_{H}, CC83 V_{H} and CC92 V_{H}.

Figure 3 illustrates the amino acid sequences of V_{H}αTAG V_{H}, CC46 V_{H}, CC49 V_{H}, CC83 V_{H} and CC92 V_{H}.

Figure 4a illustrates the nucleotide sequence and Figure 4b illustrates the corresponding amino acid sequence of the CC49 V_{L}.

Figure 5a illustrates the nucleotide sequence and Figure 5b illustrates the corresponding amino acid sequence of the CC83 V_{L}.

Figure 6a illustrates the nucleotide sequence and Figure 6b illustrates the corresponding amino acid sequence of the CC92 V_{L}.

Figure 7 illustrates the nucleotide sequence of the *Hind* III-*Pst* I fragment isolated from the plasmid pGD1.

Figure 8 illustrates the plasmid map of the pBLUESCRIPT SK(-).

Figure 9 illustrates the plasmid map of the pRL101.

Figure 10 illustrates a restriction enzyme map of the CC49 L chain genomic DNA insert in pRL101.

Figure 11 illustrates the plasmid map of the pRL200.

Figure 12 illustrates a restriction enzyme map of the CC83 L chain genomic DNA insert in pRL200.

Figure 13 illustrates the nucleotide sequence of the *Eco* RI-*Bam* HI fragment isolated from the plasmid pNP9.

Figure 14 illustrates the plasmid map of the pHH49.

Figure 15 illustrates the plasmid map of the pHS83.

Figure 16 shows the nucleotide sequence of CC49 V_{H}, with the underlined segments showing the sequences derived using oligonucleotide primers on mRNA.

Figure 17 shows the nucleotide sequence of CC83 V_{H}, with the underlined segments show the sequences derived using oligonucleotide primers on mRNA.

Figure 18 shows the amino acid sequence of CC49 V_{H}, with the underlined segments show the sequences determined by protein sequencing.

Figure 19 shows the amino acid sequence of CC83 V_{H}, with the underlined segments show the sequences determined by protein sequencing.

Figure 20 shows the results of a SDS polyacrylamide gel, with the results of PNGase F treatment of CC83 antibody.

Figure 21 illustrates the restriction enzyme map of human gamma 1, gamma 2, gamma 3, and gamma 4.

Figure 22 illustrates the plasmid map of pSV2gpt/R/B.

Figure 23 illustrates the plasmid map of pSV2gpt-γ1-7.8.

Figure 24 illustrates the plasmid map of pSV2gpt-γ1-2.3.

Figure 25 illustrates the plasmid map of pSV2gpt-γ2.

Figure 26 illustrates the plasmid map of pSV2gpt-γ3.

Figure 27 illustrates the plasmid map of pSV2gpt-γ4.

Figure 28 illustrates the plasmid map of p49γ1-7.8.

Figure 29 illustrates the plasmid map of p49γ1-2.3.

Figure 30 illustrates the plasmid map of p49-γ2.

Figure 31 illustrates the plasmid map of p49-γ3.

Figure 32 illustrates the plasmid map of p49-γ4.

Figure 33 illustrates the plasmid map of p83γ1-7.8.

Figure 34 illustrates the plasmid map of p83γ1-2.3.

Figure 35 illustrates the plasmid map of p83-γ2.

Figure 36 illustrates the plasmid map of p83-γ3.

Figure 37 illustrates the plasmid map of p83-γ4.

Figure 38 illustrates the overall reaction for the engineering of hybrid genes based on the method of Horton et al., *Gene* 77, 61 (1989).

Figures 39A, 39B, and 39C show the biodistribution and whole body retention of CH44-1.

Figures 40A and 40B show the biodistribution and whole body retention of CH84-1.

The immunoglobulin of this invention have been developed to address the problems of murine monoclonal antibodies disclosed in the prior art. It is characterized by having a chimeric structure composed of a heavy chain variable region encoded by DNA derived from the V_{H}αTAG.

### Definitions

As used herein, "immunoglobulin" refers to a tetramer or aggregate thereof whether or not specific immunoreactive activity is a property. "Antibodies" refers to such assemblies which have significant known specific immunoreactive activity to an antigen. comprising light and heavy chains, with or without covalent linkage between them: "Non-specific immunoglobulin" ("NSI") means those immunoglobulins which do not possess known specificity to an antigen.

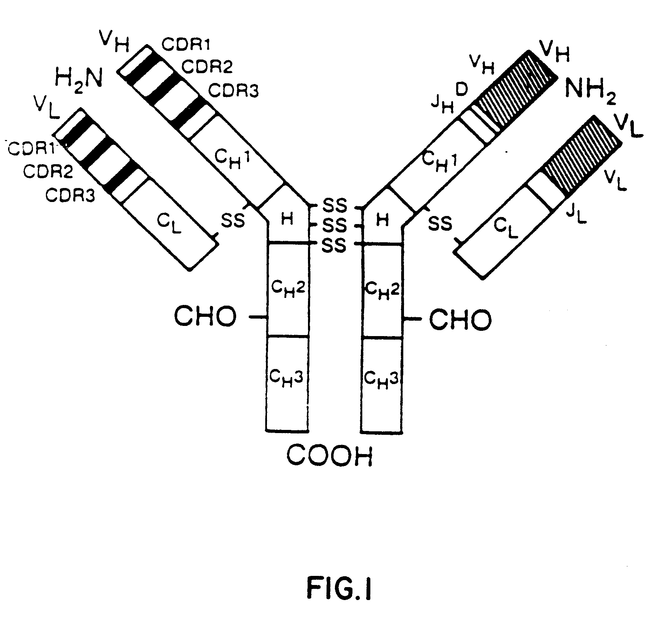

The basic immunoglobulin structural unit in vertebrate systems is relatively well understood [Edelman, G.M., *Ann. N.Y. Acad. Sci.*, 190, 5 (1971)]. As seen in Figure 1, the units are composed of two identical light polypeptide chains of molecular weight approximately 23,000 daltons, and two identical heavy chains of molecular weight 53,000-70,000. The four chains are joined by disulfide bonds in a "Y" configuration wherein the light chains bracket the heavy chains starting at the mouth of the Y and continuing through the diversity region.

Heavy chains are classified as gamma, mu, alpha, delta, or epsilon, with some subclasses among them. The nature of this chain, as it has a long constant region, determines the "class" of the antibody as IgA, IgD, IgE, IgG or IgM.

Light chains are classified as either kappa (κ) or lambda (λ). Each heavy chain class may be bound with either a kappa or lambda light chain. In general, the light and heavy chains are covalently bonded to each other, and the "tail" portions of the two heavy chains are bonded to each other by covalent disulfide linkages when the immunoglobulins are generated either by hybridomas or by B cells. However, if non-covalent association of the chains can be effected in the correct geometry, the aggregate of non-disulfide-linked chains will still be capable of reaction with antigen.

The amino acid sequences run from an N-terminus at the forked edges of the Y to the C-terminus at the bottom of each chain. At the N-terminus is a variable region and at the C-terminus is a constant region.

The terms "constant" and "variable" are used functionally. The variable regions of both light (V_{L}) and heavy (V_{H}) chains determine binding recognition and specificity to the antigen. The constant region domains of light (C_{L}) and heavy (C_{H}) chains confer important biological properties such as antibody chain association, secretion, transplacental mobility, and complement binding.

The variable region is linked in each chain to the constant region by a linkage linking the V gene sequence and the C gene sequence. The linkage occurs at the genomic level, combining nucleotide sequences via recombination sites. The linking sequence is known currently as a "J" sequence in the light chain gene, which encodes about 12 amino acids, and as a combination of a "D" sequence and a "J" sequence in the heavy chain gene, which together encode approximately 25 amino acids.

"Chimeric antibody" for purposes of this invention refers to an antibody having in the heavy chain a variable region amino acid sequence encoded by a nucleotide sequence derived from a murine germline gene and a constant region amino acid sequences encoded by a nucleotide sequence derived from a human gene.

However, the present invention is not intended to be narrowly limited to merely substituting human C genes sequences encoding immunoglobulin constant regions for murine C gene sequences encoding immunoglobulin constant regions. Thus the present invention is not limited to whether or not the fusion point is at the variable/constant boundary.

Through various techniques, it is now possible to produce altered chimeric antibodies, composite chimeric antibodies, and fragmented chimeric antibodies encoded by nucleotide sequences disclosed herein.

"Composite" immunoglobulins comprise polypeptide variable regions not hitherto found associated with each other in nature. It is not critical whether any of the above are covalently or noncovalently aggregated, so long as the aggregation is capable of selectively reacting with a particular antigen or antigen family.

"Altered antibodies" means antibodies wherein the amino acid sequences, particularly in the variable region, has been varied. Because of the relevance of recombinant DNA techniques to this invention, one need not be confined to the amino acid sequences of antibodies selected from natural sources; amino acid sequences of the antibodies can be redesigned to obtain desired characteristics. The possible variations are many and range from the changing of just one or a few amino acids to the complete redesign of an antibody variable and/or constant region.

Changes in the variable region will be made in order to improve the antigen binding characteristics. Changes in the constant region will, in general, be made in order to improve the cellular process characteristics, such as complement fixation, interaction with membranes, and other effector functions. Alterations, can be made by standard recombinant techniques and also by oligonucleotide-directed mutagenesis techniques [Dalbadie-McFarland, et al. *Proc. Natl. Acad. Sci. (USA)* 79, 6409 (1982)].

"Fragments" of immunoglobulins include segments of proteolytically-cleaved or recombinantly-prepared portions of an antibody molecule that are capable of selectively reacting with a particular antigen or antigen family. Nonlimiting examples of such proteolytic and/or recombinant fragments include "Fab", "F(ab′)₂", and "Fab′", with their proteolytic cleavage sites being shown in Figure 1; as well as "Fv". Recombinant techniques for producing Fv fragments are set forth in WO 88/01649, WO 88/06630, WO 88/07085, WO 88/07086 and WO 88/09344.

In this invention, "animals" is meant to include bovines, Porcine, rodents, and primates, including humans, and others.

"Expression vector" is given a functional definition of any DNA sequence which is capable of effecting expression of a specified DNA code in a suitable host is included in this term. As at present, such vectors are frequently in the form of plasmids; thus "plasmid" and "expression vector" are often used interchangeably. However, the invention is intended to include such other forms of expression vectors which serve equivalent functions and which may, from time to time, become known in the art.

By "transformation" is meant the introduction of DNA into a recipient host cell that changes the genotype and consequently results in a change in the recipient cell.

"Host cells" refers to cells which have been recombinantly transformed with vectors constructed using recombinant DNA techniques. As defined herein, the antibody or modification thereof produced by a host cell is by virtue of this transformation.

In descriptions of processes for isolation of antibodies from recombinant hosts, the terms "cell" and "cell culture" are used interchangeably to denote the source of antibody unless it is clearly specified otherwise. In other words, recovery of antibody from the "cells" may mean either from spun down whole cells, or from the cell culture containing both the medium and the suspended cells.

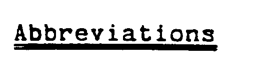

Nucleic acids, amino acids, peptides, protective groups, active groups and similar moieties, when abbreviated, are abbreviated according to the IUPACIUB (Commission on Biological Nomenclature) or the practice in the fields concerned. The following are examples.

### Reagents

- EDTA:: Ethylenediaminetetraacetic acid
- SDS:: Sodium dodecylsulfate

### Nucleic Acids

- RNA:: Ribonucleic acid
- DNA:: Deoxyribonucleic acid

### Nitrogenous Bases

| Purines | Pyrimidines |
|---|---|
| A: Adenine | T: Thymine |
| G: Guanine | C: Cytosine |
| | U: Uracil |

Both DNA and RNA contain long chains of phosphoric acid, a sugar, and nitrogenous bases. DNA is a double stranded helix, wherein the sugar is 2-deoxyribose, whereas RNA is single stranded, wherein the sugar is D-ribose. The four nitrogenous bases which characterize DNA nucleotides are linked in complementary pairs by hydrogen bonds to form the double helix of DNA: adenine is linked to thymine; guanine is linked to cytosine. In RNA, uracil is substituted for thymine in the listed DNA pairs.

### Amino Acids

| | |
|---|---|
| Gly: glycine | Phe: phenylalanine |
| Ala: alanine | Tyr: tyrosine |
| Val: valine | Thr: threonine |
| Leu: leucine | Cys: cysteine |
| Ile: isoleucine | Met: methionine |
| Ser: serine | Glu: glutamic acid |
| Asp: aspartic acid | Trp: tryptophan |
| Lys: lysine | Pro: proline |
| Arg: arginine | Asn: asparagine |
| His: histidine | Gln: glutamine |

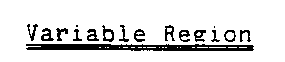

The DNA encoding the heavy chain consists of a V_{H} gene sequence, a D_{H} gene sequence, and a J_{H} gene sequence. The DNA encoding the light chain consists of a V_{L} gene sequence, and a J_{L} gene sequence.

### V_{H} Gene Sequence

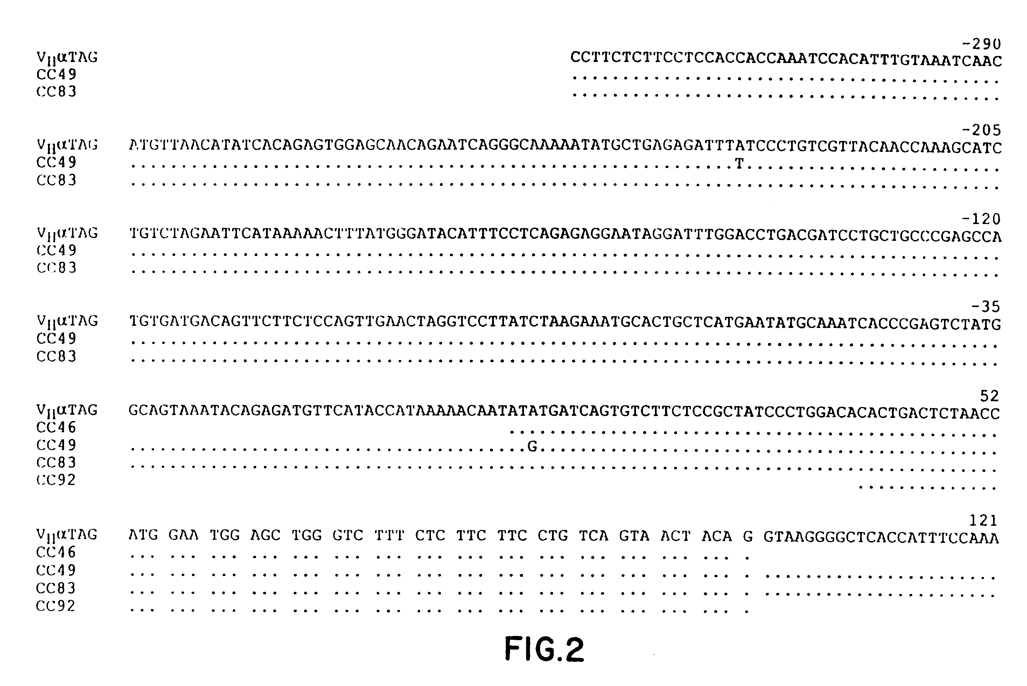

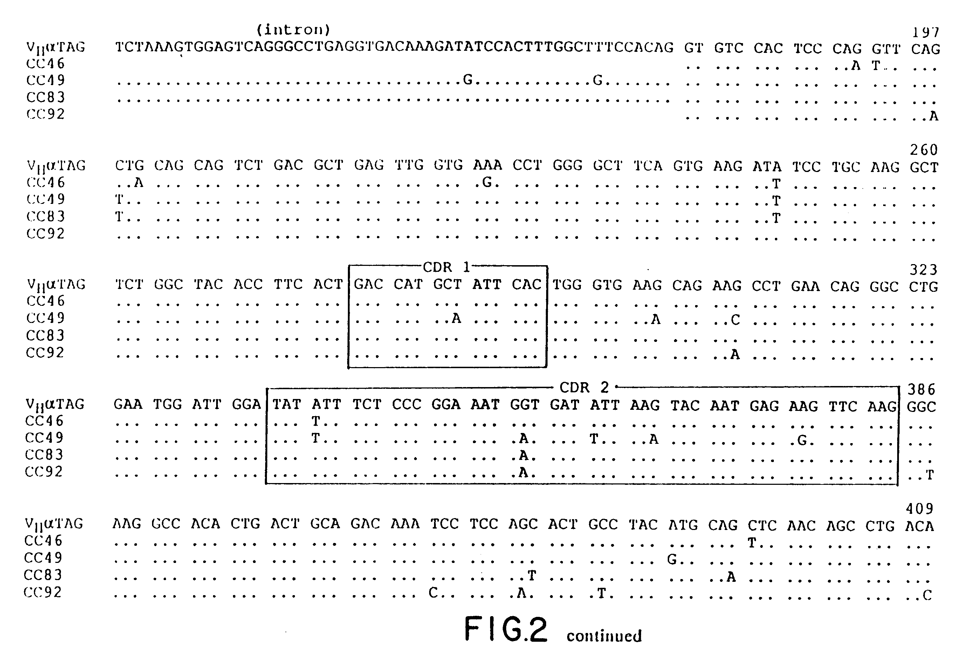

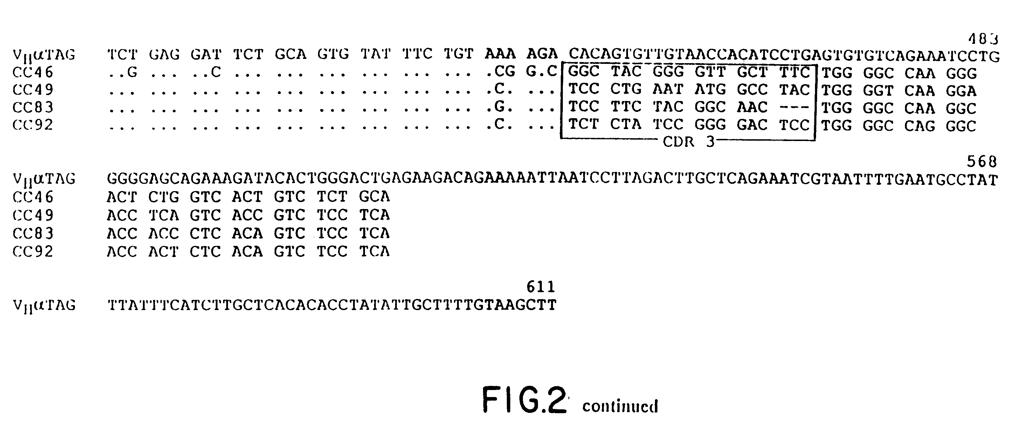

The present invention is directed to selected chimeric antibodies having the V_{H} region encoded by a DNA sequence derived from a germline gene that is specifically reactive against TAG72 (V_{H}αTAG), the sequence of which is set forth in Figure 2. The chimeric antibodies are selected on the basis of their ability to bind TAG72, namely wherein the variable region binds to TAG72 at least 25 percent greater than the variable region of B72.3 binds to TAG72. Generally, the binding affinities of the chimeric antibody and B72.3 are measured by the same technique. Exemplary techniques for measuring antibody binding affinity are set forth in the following references: Scatchard G., *Annals of the N.Y. Acad. of Sciences* 51, 660 (1949); Steward, M.W., and Petty, R.E., *Immunology* 23, 881 (1972); Muraro, R., et al., *Cancer Research* 48, 4588 (1988); and Heyman. B., *J. of Immunol. Methods* 68, 193-204 (1984).

A skilled artsan will appreciate that, as a result of the present invention, namely the nucleotide sequence of (and amino acid sequences encoded by) the V_{H}αTAG, the present invention is intended to include effectively homologous nucleotide sequences and corresponding amino acid sequences. "Effectively homologous" refers to identity or near identify of nucleotide or amino acid sequences. Thus, in this disclosure it will be understood that minor sequence variation can exist within homologous sequences and that any sequences exhibiting at least 80% homology are deemed equivalent.

Homology is expressed at the fraction or percentage of matching bases (or amino acids) after two sequences (possibly of unequal length) have been aligned. The term alignment is used in the sense defined by Sankoff and Kruskal in Chapter One of their book, The Time Warps, String Edits, and Macromolecules: The Theory and Practice of Sequence Comparison, Addison-Wesley, Reading, MA, (1983). Roughly, two sequences are aligned by maximizing the number of matching bases (or amino acids) between the two sequences with the insertion of a minimal number of "blank" or "null" bases into either sequence to bring about the maximum overlap.

As is understood in the art, nucleotide mismatches can occur at the third or wobble base in the codon without causing amino acid substitutions in the final polypeptide sequence. Also, minor nucleotide modifications (e.g., substitutions, insertions or deletions) in certain regions of the gene sequence can be tolerated and considered insignificant whenever such modifications result in changes in amino acid sequence that do not alter functionality of the final product. It has been shown that chemically synthesized copies of whole, or parts of, gene sequences can replace the corresponding regions in the natural gene without loss of gene function.

Homologs of specific DNA sequences may be identified by those skilled in the art using the test of cross-hybridization of nucleic acids under conditions of stringency as is well understood in the art [as described in Nucleic Acid Hybridization, Hames and Higgens (eds.), IRL Press, Oxford, UK (1985)]. Given two sequences, algorithms are available for computing their homology: e.g. Needleham and Wunsch, *J. Mol. Biol.*, 48, 443-453 (1970); and Sankoff and Kruskal (cited above) pgs. 23-29. Also, commercial services are available for performing such comparisons, e.g. Intelligenetics, Inc. (Palo Alto, CA).

### D_{H} and J_{H} Gene Sequences

The D_{H} and J_{H} gene segments exist in various types, although the type of D or J gene segment selected is not critical to the invention. That is the D_{H} and J_{H} may be derived from any animal. Preferred animals include mice and humans. Obviously, human _{DH} and/or J_{H} gene segments are particularly preferred, but the invention is not so limited if a D or J gene segment from another animal species provides an important property, i.e., increased binding to TAG72.

Exemplary murine D_{H} and J_{H} sequences are set forth in "Organization, Structure, and Assembly of Immunoglobulin Heavy Chain Diversity DNA Segments", Kurosawa and Tonegawa, *J. Exp. Med.* 155, 201 (1982); and "Sequences of the Joining Region Genes for Immunoglobulin Heavy Chains and Their Role in Generation of Antibody Diversity" Gough and Bernard, *Proc. Natl. Acad. Sci. (USA)*, 78, 509 (1981).

Exemplary human D_{H} and J_{H} sequences are set forth in an article titled "Human Immunoglobulin D Segments encoded in Tandem Multigenic Families" by Siebenlist et al. in *Nature* 294, 631 (1981); and exemplary human J_{H} sequences are set forth in "Structure of the Human Immunoglobulin µ Locus: Characterization of Embryonic and Rearranged J and D Genes" by Ravetch et al., *Cell*, 27, 583 (1981).

### V_{L} and J_{L} Gene Sequences

Generally, any V_{L} and J_{L} gene sequences may be employed that encodes a portion of a V_{L} which is complementary to the V_{H} encoded by a nucleotide sequence effectively homologous to V_{H}αTAG. By "complementary" means a V_{L} that binds to the V_{H} and which yields an antibody variable region having a binding affinity of at least 25% more than B72.3, as measured by any standard technique for measuring binding affinity constants.

The type of V_{L} and J_{L} gene segment selected is not critical to the invention. That is the V_{L} and J_{L} may be derived from any animal. Preferred animals include mice and humans. Obviously, human V_{L} and/or J_{L} gene segments are particularly preferred, but the invention is not so limited if a J_{L} gene segment from another species provides an important property, i.e., increased binding to TAG72.

Murine J_{L} sequences are set forth in an article titled "The nucleotide Sequence of a 5,5-kilobase DNA Segment Containing the Mouse Kappa Immunoglobulin J and C Region Genes" by Max. et al. in *J. Biol. Chem.* 256, 5116-5120 (1981). Human J_{L} sequences are set forth in an article titled "Evolution of Human Immunoglobulin K J Region Genes" by Heiter et al. in *The Journal of Biological Chemistry* 357(2), 1516-1522 (1982).

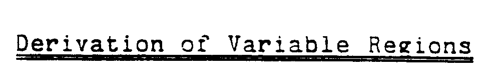

Given the above teachings, it now becomes possible to derive numerous specific embodiments of antibody variable regions within the scope of the present invention, i.e., having effectively homologous V_{H} sequences to V_{H}αTAG and binding to TAG72 at least 25% greater than the variable region of B72.3 binds to TAG72, with the binding affinities of the antibody and B72.3 being measured by the same technique. Several possible techniques are set forth below.

### Naturally-Produced Variable Regions

In response to an immunogen, TAG72, an immunized animal will expand selected antibody producing B cells. The variable region of antibodies produced by the B cells will be encoded by rearranged germline heavy and light chain DNA. For example, the rearranged germline heavy chain will include the V, D, and J gene segments including the leader sequence, as well as any introns which may be subsequently removed. The light chain coding DNA will include the V and J gene segments including the leader sequence, as well as any introns which may be subsequently removed.

Variability may result from somatic mutations occurring in a B cell during productive rearrangement of the V_{H}αTAG. These somatic mutations are nucleotide changes that may or may not result in an amino acid change that alters the activity toward TAG72 of the productively rearranged V_{H}.

### Screening Techniques

Monoclonal or polyclonal antibodies may be Screened to determine which of said antibodies Selectively bind to TAG72. Such screening may be accomplished by any of a number of well-known procedures, such as solid-phase radioimmunoassay, enzyme-linked immunosorbent assays, rosetting assays and blocking assays. The above-described procedures are well-known in the art.

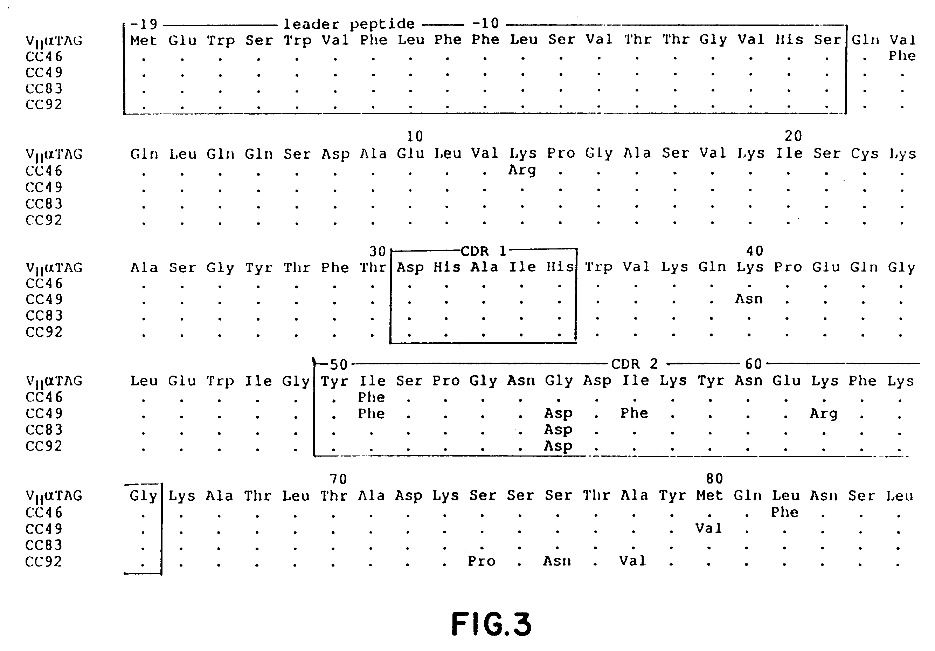

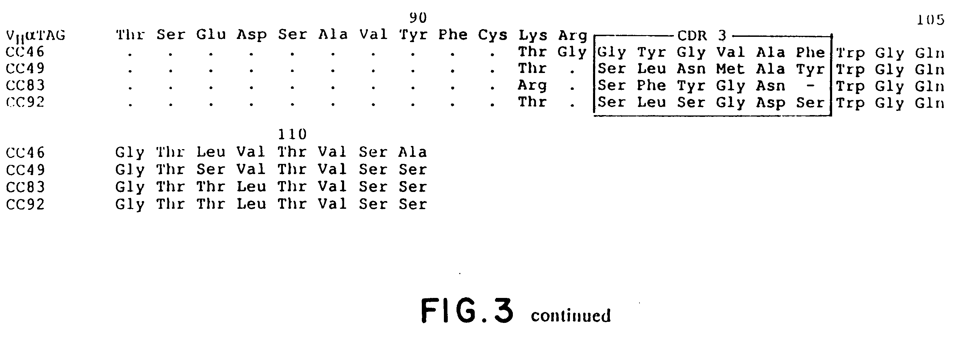

The nucleotide sequences of encoding variable regions of antibodies produced from the productive rearrangement of the V_{H}αTAG have now been obtained. In addition to the nucleotide sequence of V_{H}αTAG, Figure 2 also shows the nucleotide sequences encoding the heavy chain variable regions of CC46, CC49, CC83 and CC92 antibodies, respectively. Figure 3 shows the amino acid sequences of V_{H}αTAG V_{H}, CC46 V_{H}, CC49 V_{H}, CC83 V_{H}, and CC92 V_{H}, corresponding to the nucleotide sequences set forth in Figure 2. A comparison of the nucleotide and amino acid sequences of V_{H}αTAG V_{H}, CC46 V_{H}, CC49 V_{H}, CC83 V_{H} and CC92 V_{H} shows a most striking feature, namely that the chains have extraordinary similarity.

The relative similarity of the DNA encoding the CC46 V_{H}, CC49 V_{H}, CC83 V_{H}, and CC92 V_{H} regions, Particularly in the 5′ flanking segment, proves that those DNA sequences are derived from V_{H}αTAG. Somatic mutations occuring during productive rearrangement of the V_{H} region gene to be expressed in a B cell give rise to some nucleotide changes that may or may not result in a homologous amino acid change between two productively rearranged V_{H}αTAG producing hybridomas.

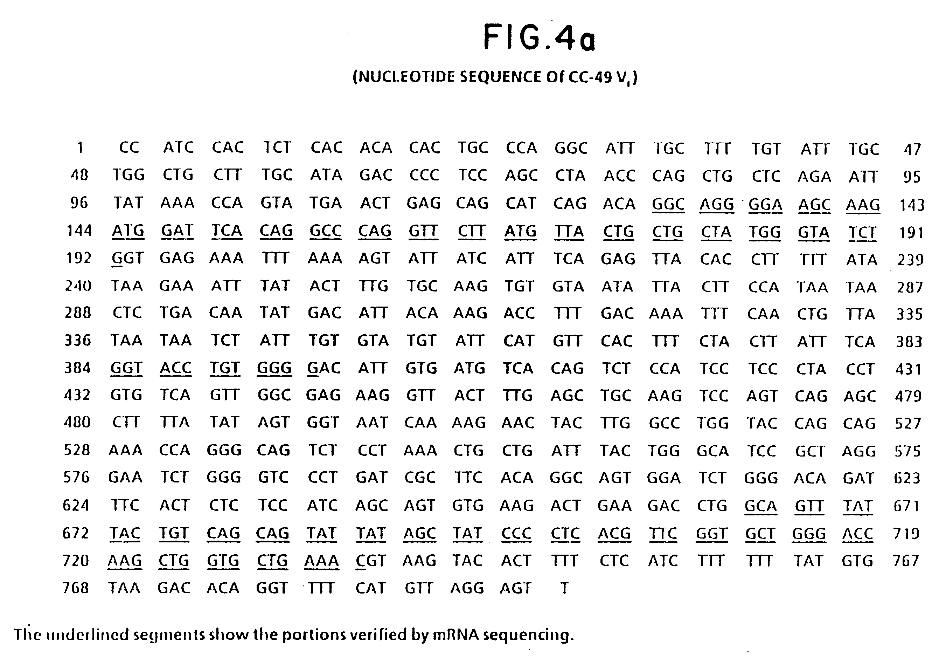

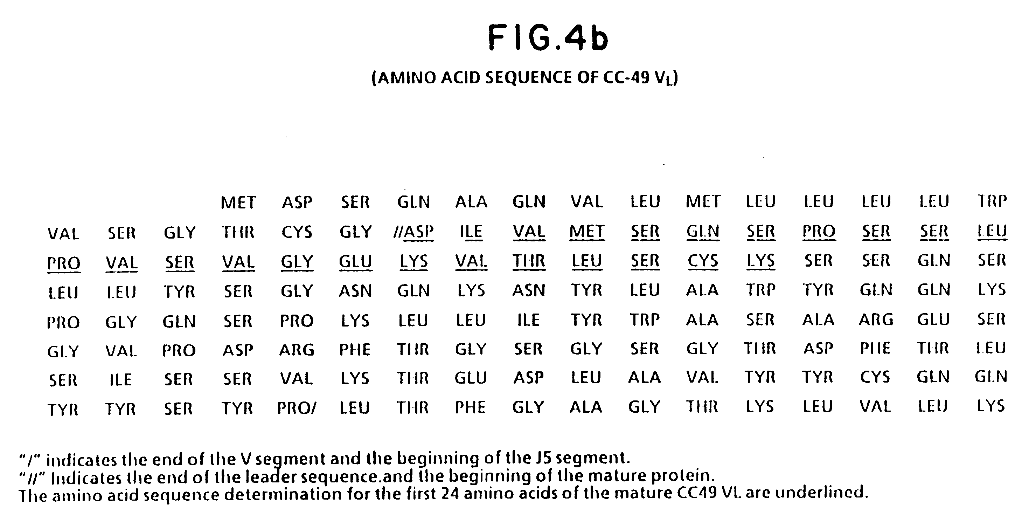

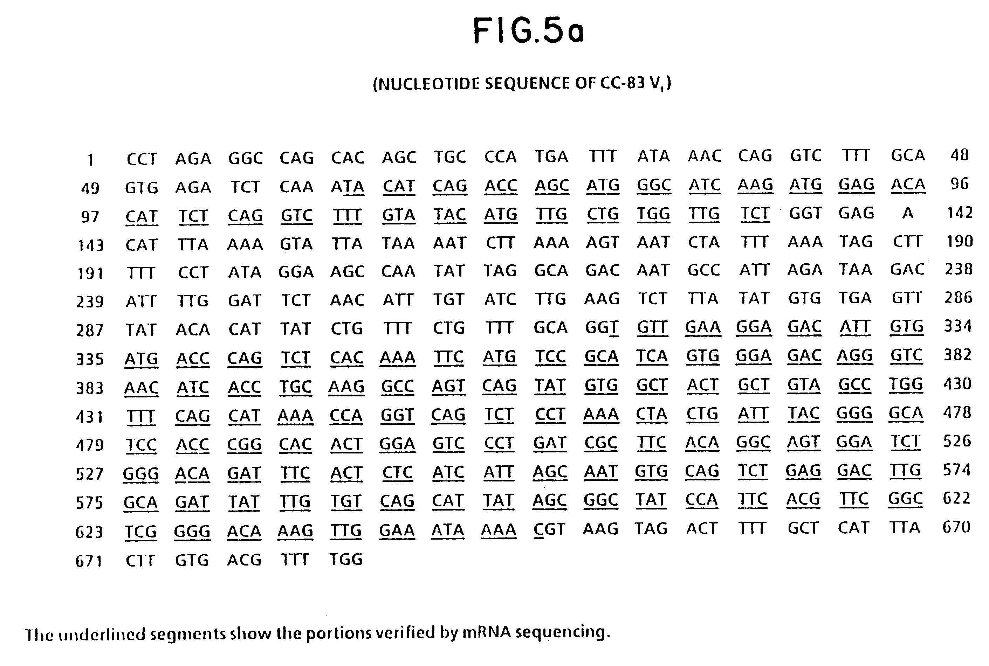

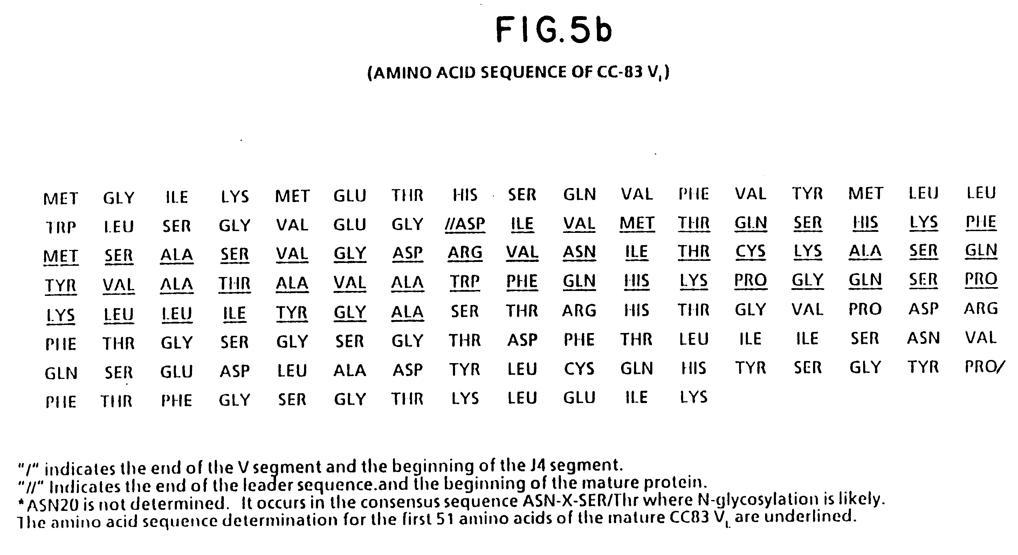

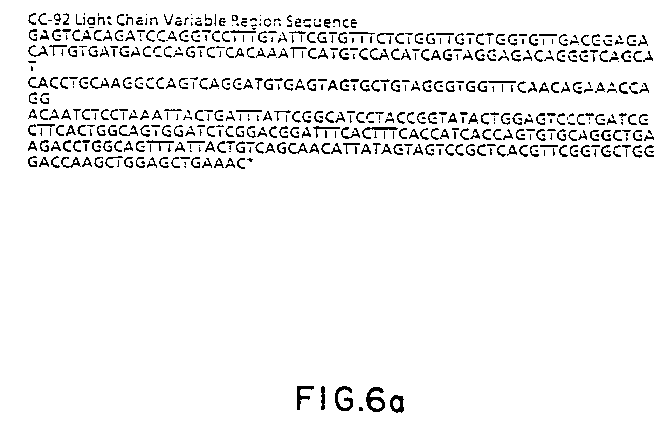

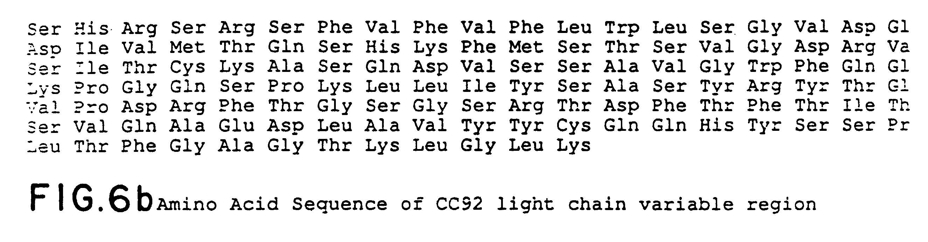

The nucleotide sequences and corresponding amino acid sequences of CC49 V_{L} are shown in Figures 4a and 4b, respectively. The nucleotide sequences and corresponding amino acid sequences of CC83 V_{L} are shown in Figures 5a and 5b, respectively. The nucleotide sequences and corresponding amino acid sequences of CC92 V_{L} are shown in Figures 6a and 6b, respectively.

### Probe Techniques

Other antibodies encoded by DNA derived from V_{H}αTAG may be derived by using V_{H}αTAG as a hybridization probe. Generally, a probe made from the DNA or RNA of the V_{H}αTAG or rearranged genes containing the recombined V_{H}αTAG could be used by those skilled in the art to find homologous genes in unknown hybridomas. Such homologous antibodies will have a DNA sequence whose mRNA hybridizes with the probe of all or a part of the V_{H}αTAG germline gene and its flanking regions. By "flanking regions" is meant to include those DNA sequences from the 5′ end of the V_{H}αTAG to the 3′ end of the upstream gene, and from 3′ end of the V_{H}αTAG to the 5′ end of the downstream gene.

### Rationally Synthesized Variable Regions

A yet further approach is the rational synthesis of altered variable regions of the antibodies disclosed herein, as well as antibodies discovered via probing. Such an approach has several potential advantages. Namely, a researcher would not have to screen immunized host animals attempting first to cull those antibodies which bind to TAG and next to cull those antibodies which specifically have V_{H} regions encoded by DNA derived from V_{H}αTAG.

### Mutagenic Techniques

The V_{H} and/or V_{L} gene segments may be "altered" by mutagenesis. Exemplary techniques include the addition, deletion, or nonconservative substitution of a limited number of various nucleotides or the conservative substitution of many nucleotides, provided that the proper reading frame is maintained.

Substitutions, deletions, insertions or any subcombination may be combined to arrive at a final construct. Since there are 64 possible codon sequences but only twenty known amino acids, the genetic code is degenerate in the sense that different codons may yield the same amino acid. However, the code is precise for each amino acid; thus there is at least one codon for each amino acid, i.e., each codon yields a single amino acid and no other. It will be apparent that during translation, the proper reading frame must be maintained in order to obtain the proper amino acid sequence in the polypeptide ultimately produced.

Techniques for additions at predetermined amino acid sites having a known sequence are well known. Exemplary techniques include oligonulceotide-mediated, site-directed mutagenesis and polymerase chain reaction.

Techniques for deletions at predetermined amino acid sites having a known Sequence are well known. Exemplary techniques include oligionulceotide-mediated site-directed mutagenesis and the polymerase chain reaction.

Techniques for substitutions at predetermined amino acid sites having a known sequence are well known. Exemplary techniques include site-directed mutagenesis, and the polymerase chain reaction technique.

Oligonucleotide site-directed mutagenesis in essence involves hybridizing an oligonucleotide coding for a desired mutation with a single strand of DNA containing the region to be mutated and using the single strand as a template for extension of the oligonucleotide to produce a strand containing the mutation. This technique, in various forms, is described by Zoller, M.J. and Smith, M., *Nuc. Acids Res.* 10, 6487-6500 (1982); Norris, K., Norris, F., Christiansen, L. and Fiii, N., *Nuc. Acids Res.* 11, 5103-5112 (1983); Zoller, M.J. and Smith, M., *DNA* 3, 479-488 (1984); Kramer, W., Schughart, K. and Fritz, W. J., *Nuc. Acids Res.* 10, 6475-6485 (1982).

Polymerase chain reaction (PCR) in essence involves exponentially amplifying DNA in vitro using sequence specified oligonucleotides. The oligonucleotides can incorporate sequence alterations if desired. The polymerase chain reaction technique is described in Mullis and Faloona, *Meth. Enz.* 155, 335-350 (1987). Examples of mutagenesis using PCR are described in Higuchi et al., *Nucl. Acids Res.* 16, 7351-7367 (1988), Ho et al., *Gene* 77, 51-59 (1989), and "Engineering Hybrid Restriction Genes Without the Use of Restriction Enzymes: Gene Splicing by Overlap Extension", Horton et al., *Gene* 77, 61 (1989).

Alteration of the antibody variable regions may be of particular use in the therapeutic use of monoclonal antibodies. At present, when a chimeric antibody comprising a complete mouse variable domain is injected into a human, the human body's immune system recognizes the mouse variable domain, albeit less than a complete murine antibody, as foreign and produces an immune response thereto. Thus, on subsequent injections of the mouse antibody or chimeric antibody into the human, its effectiveness is considerably reduced by the action of the body's immune system against the foreign antibody. Consequently, alterations of the murine V_{H} and V_{L} regions may reduce the human immune response to the altered antibody.

### Recombinant Techniques

The antibodies may be constructed by recombinant techniques. In other words, because the nucleotide sequences of various V_{H}- and V_{L}-encoding regions are now provided, a skilled artisan could *in vitro* produce a complete gene coding for the heavy and light chain variable regions.

The constructed gene may be engineered in which selected D_{H} and J_{H} gene segments are in functional combination with a selected V_{H} gene segment, i.e., the V_{H}αTAG segment, or the V_{H} gene segment of CC49 or CC83.

For example, the constructed heavy chain coding DNA will include D_{H} and J_{H} gene sequences which are contiguous with the 3′ end of the germline V_{H}αTAG gene segment, thereby completing the CDR3 and framework (FR) 4 of the V_{H} domain. A leader sequence may be present but may be subsequently removed.

Depending upon the light chain employed, it may also be necessary to provide a constructed light chain coding DNA. Such a DNA gene will comprise a V_{L} gene segment in functional combination, e.g., contiguous with a J_{L} gene segment, including the leader sequence which may be subsequently removed. The J_{L} gene segment will vary depending upon whether the light chain is of the lambda or kappa system. The J region sequence is contiguous with the end of the V_{L} exon to complete FR 4 of the V_{L} domain. Such a construction may be carried out by the techniques used to construct the V_{H} gene.

The constructed gene may be engineered by conventional recombinant techniques for example, to provide a gene insert in a plasmid capable of expression. Thereafter, the plasmids may be expressed in host cells. Exemplary recombinant biological techniques are set forth below.

In providing a fragment encoding either the light chain or heavy chain variable region, it will usually be desirable to include all or a portion of the intron downstream from the J region, particularly where the variable region is derived from the host in which the fused gene is to be expressed. Where the intron is retained, it will be necessary that there be functional splice acceptor and donor sequences at the intron termini. The intron between the J and the constant region of the fused gene may be primarily the intron sequence associated with (1) the constant region, (2) the J domain, or (3) portions of each. The last may be a matter of convenience where there is a convenient restriction site in the introns from the two sources. It may be necessary to provide adapters to join the intron to the constant region. In some instances, all or a portion of the intron may be modified by deletion, nucleotide substitution(s) or insertion, to enhance ease of manipulation, expression, or the like. Preferably, a sufficient amount of the intron should be present to contain an enhancer that is functionally active with the naturally-occurring promoter.

Alternatively, it may be desirable to have the fused gene free of the intron between the J gene and C gene. Thus, the 3′ terminus of the J gene will be adjacent to the 5′ terminus of the C gene. One can use an exonuclease and, by employing varying periods of digestion, one can provide for varying 3′-termini, which can then be used for linking to the constant region and selection made for a functional product in a variety of ways; or by splicing with overlap extension using polymerase chain reaction technology, see Horton et al., *supra*. In this case, an artificial promoter, which does not need to be functionally active with an enhancer, will generally be utilized

In one preferred embodiment, the genes encoding the V_{H} and V_{L} regions may be altered by replacing at least parts of the complementarity determining regions (CDRs) in the light or heavy chain variable domains of the antibody with analogous parts of CDRs from an antibody of different specificity. An exemplary technique replacing the CDRs is taught in European Published Patent Application 0 239 400, by Gregory Winter; and in PCT application Wo 88/09344, by Huston et al. In an altered antibody of the present invention, only the CDRs of the antibody will be foreign to a human body, and this should minimize side effects if used for human therapy. However, human and mouse framework regions have characteristic features which distinguish human from mouse framework regions. Thus, an antibody comprised of mouse CDRs in a human framework may well be no more foreign to the body than a genuine human antibody.

The nucleotide sequences corresponding to the V_{H} amino acid sequences of the V_{H}αTAG, CC46, CC49, CC83 and CC92, as well as of the CC49, CC83 and CC92 V_{L} gene segments are provided. Consequently, it is envisaged that the CDRs from the antibodies of the present invention could be grafted onto the framework regions of a human antibody.

Generally, the CDR regions from a human V_{H} or V_{L} domain may be replaced by CDRs from the V_{H} or V_{L} regions of antibodies of the present invention. Exemplary human antibodies from which the framework portions may be used include human plasmacytoma NEWM, [Jones et al., "Replacing the complementarity-determining regions in a human antibody with those from a mouse", *Nature* 321, 522-525 (1986)], publicly available from Dr. Greg Winter; and various other human V_{H} and V_{L} genes available from Dr. Terrence Rabbitts, both researchers being from the Medical Research Council, 20 Park Crescent, London, W1N 4AL.

The determination as to what constitutes a CDR and what constitutes a framework region may be made on the basis of the amino-acid sequences of a selected Ig as indicated in Kabat et al., Sequences of Proteins of Immunological Interest. Fourth Edition (1987), U.S. Dept. of Health and Human Services, NIH.

The four framework regions largely adopt a β-sheet conformation and the CDRs form loops connecting, and in some cases forming part of, the β-sheet structure.

Moreover, not all of the amino-acid residues in the loop regions are solvent accessible and in one case, amino-acid residues in the framework regions are involved in antigen binding. [Amit, A.G., Mariuzza, R.A., Phillips, S.E.V. and Poljak, R.J., *Science* 233, 747 -753, (1986)].

It is also known that the variable regions of the two parts of an antigen binding site are held in the correct orientation by inter-chain, non-covalent interactions. These may involve amino-acid residues within the CDRs.

Thus, in order to transfer the antigen binding capacity of one variable domain to another, it may not be necessary to replace all of the CDRs with the complete CDRs from the donor variable region. It may be necessary only to transfer those residues which are necessary for the antigen binding site, and this may involve transferring framework region residues as well as CDR residues.

It is thus clear that merely replacing one or more CDRs with complementary CDRs may not always result in a functional altered antibody. However, given the explanations set forth in European Published Patent Application 0 239 400, it will be well within the competence of those skilled in the art, either by carrying out routine experimentation or by trial and error testing, to obtain a functional altered antibody.

Preferably, the variable domains in both the heavy and light chains are altered by at least partial CDR replacement and, if necessary, by partial framework region replacement and sequence changing. Although the CDRs may be derived from an antibody of the same class or even subclass as the antibody from which the framework regions are derived, it is envisaged that the CDRs will be derived from an antibody of different class and preferably from an antibody from a different species.

### Composite Variable Regions

Generally, the V gene encoding the V_{L} is the same V gene which encodes the V_{L} naturally combined with the V_{H} of choice. For example, the V gene which encodes the V_{L} regions of CC49 and CC83 are beneficially used when employing the V gene which encodes the V_{H} of CC49 and CC83, respectively.

Surprisingly, because the V_{H} regions of the antibodies of the present invention are encoded by V_{H} genes derived from V_{H}αTAG, composite antibodies may be beneficially formed. In other words, the V_{H} region of one antibody of the present invention may suitably be combined with the V_{L} region of another antibody of the present invention. Although the amino acid sequences of the CC49 and CC83 heavy chains are superficially close, it would be expected that a change of a few or even one amino acid would drastically affect the binding function of the antibody, i.e., the resultant antibodies are generally presumed to be a non-specific immunoglobulin (NSI), i.e.--lacking in antibody character, (see European Published Patent Application 0 125 023).

Quite surprisingly, it has now been found that an antibody having the requisite V_{H} of this invention, need not be recombined only with a V_{L} from the same naturally occuring animal antibody. For instance, as set forth in the examples, it is possible to produce a chimeric antibody having a heavy chain with a V_{H} from CC83 and a light chain with a V_{L} from CC49, wherein the composite antibody thus formed has a binding specificity 25% greater than the binding affinity of B72.3 to TAG72.

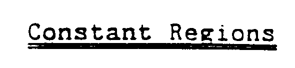

### Heavy Chain (C_{H}) Domain

The C_{H} domains may be of various human isotypes, i.e., IgG (e.g., IgG₁, IgG₂, IgG₃ and IgG₄), IgA, IgD, IgM, as well as the various subtypes of the individual groups.

For a discussion of the human γ1, see Ellison et al., "The nucleotide sequence of a human immunoglobulin C-gamma-1 gene", *Nucl. Acid Res* 10, 4071-4079 (1982); Takahashi et al., "Structure of human immunglobulin gamma genes: Implications for evolution of a gene family", *Cell* 29, 671-679 (1982). For a discussion of the human gamma 2 (γ2), see Krawinkel et al., "Comparison of the hinge-coding segments in human immunoglobulin gamma heavy genes and the linkage of the gamma 2 and gamma 4 subclass genes, *EMBO* J 1, 403-407 (1982); Ellison et al., "Linkage and sequence homology of two human immunoglobulin gamma heavy chain constant region genes, *Proc. Nat. Acad. Sci. (USA)* 79, 1984-1988 (182); Takahashi et al., *infra*. For a discussion of human gamma 3 (γ3), see Krawinkel et al. *infra*, and Takahashi et al., *infra*. For a discussion of human gamma 4 (γ4), see Ellison et al. "Nucleotide sequence of a human immunoglobulin C-gamma-4 gene, *DNA* 1, 11-18 (1981), Krawinkel et al. *infra*, and Takahashi et al., *infra*.

For a discussion of the human mu, see Rabbitts et al., Human Immunoglobulin Heavy Chain Genes: Evolutionary Comparisons of Cµ, Cδ, and Cγ genes and Associated Switch Sequences", *Nucl.. Acid Res.* 9, 4509-45024.

For a discussion of the human alpha, see Flanagan et al., "Mechanisms of Divergence and Convergence of the Human Immunoglobulin alpha 1 and alpha 2 Constant Region Gene Sequences", *Cell* 36, 681-688 (1984).

For a discussion of the human delta, see White et al., "Human Immunoglobulin D: Genomic Sequences of the Delta Heavy Chain", *Science* 228, 733-737 (1985).

For a discussion of the human epsilon, see Max et al., "Duplication and Deletion in the Human Immunoglobulin ε Genes", *Cell* 29, 691-699 (1982).

### Light Chain (C_{L}) Domain

The C_{L} domain may be human kappa (κ) or human lambda (λ).

For a discussion of the human κ, see "Cloned Human and Mouse Kappa Immunoglobulin Constant and J Region Genes Conserve Homology in Functional Segments", Heiter et al., *Cell* 22, 197-207, November (1980)

For a discussion of the human λ, see "Processed Genes: A Dispersed Human Immunoglobulin Gene Bearing Evidence of RNA-Type Processing", Hollis et al., *Nature* 296, 321-325 (1982).

The C_{H} and/or C_{L} gene segments may be "altered" by mutagenesis. Exemplary techniques include the addition, deletion, or nonconservative substitution of a limited number of various nucleotides or the conservative substitution of many nucleotides, provided that the proper reading frame is maintained. In addition, entire domains of the protein can be altered, for example, by substituting C_{H}2 for C_{H}3. This substitution is made at the DNA level by inserting, deleting or substituting entire exons of sequence.

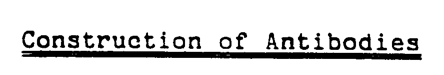

### Immunizations

The first technique for producing antibodies, whether monoclonal or polyclonal, having V_{H} regions encoded by DNA derived from V_{H}αTAG is to immunize a host animal with purified TAG72. Exemplary protocols for immunizing a host animal with TAG72 are set forth in U.S. Patents 4,522,918 and 4,612,282, using a human breast carcinoma extract as the immunogen; and United States Patent Application 7-073,685 (which is available to the public), using TAG72 purified with B72.3 as the immunogen.

Thereafter, monoclonal or polyclonal antibodies produced from the immunization protocol are screened to determine which of said antibodies selectively bind to TAG72. Such screening may be accomplished by any of a number of well-known procedures, such as solid-phase radioimmunoassay, enzyme-linked immunosorbent assays, rosetting assays and blocking assays. The above-described procedures are well-known in the art.

### Synthesis of Amino Acid Sequences

Immunoglobulins of the present invention can be synthesized from their constituent amino acids. Suitable techniques are the Merrifield solid phase method, as described in *J. Amer. Chem. Soc.* 85, 2149-2154 (1963). This solid phase method for synthesizing sequences of amino acids is also described on pages 1-4 of of a book by Stewart and Young, Solid Phase Peptide Synthesis (W. H. Freemen and Co., San Francisco, 1969).

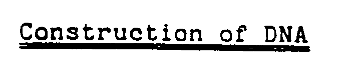

### DNA Encoding the V_{H} and V_{L}

The DNA encoding the antibody heavy and light chains may be obtained from a variety of sources known to those of ordinary skill in the art, for example, genomic DNA, cDNA, synthetic DNA, or a combination thereof.

Cells coding for the desired sequence may be isolated, and genomic DNA fragmented by one or more restriction enzymes. The genomic DNA may or may not include naturally-occurring introns. The resulting fragments may then be cloned and screened using a heavy chain J region (J_{H}) probe for the presence of the DNA sequence coding for the polypeptide sequence of interest. DNA fragments isolated by preparative agarose gel electrophoresis are ligated. Recombinant plaques of the libraries are screened with a mouse J_{H} probe.

The DNA may also be obtained from a cDNA library. Messenger RNA coding for heavy or light chain is isolated from a suitable source, either mature B cells or a hybridoma culture, employing standard techniques of RNA isolation, and the use of oligo-dT cellulose chromatography to segregate the poly-A mRNA. The poly-A mRNA may, further, be fractionated to obtain sequences of sufficient size to code for the amino acid sequences in the light or heavy chain of the desired antibody as necessary.

A cDNA library is then prepared from the mixture of mRNA using a suitable primer, preferably a nucleic acid sequence which is characteristic of the desired cDNA. Such a primer may be synthesized based on the amino acid sequence of the antibody. In the alternative cDNA from unfractionated poly-A mRNA from a cell line producing the desired antibody or poly-dT may also be used. The resulting cDNA is optionally size fractionated on polyacrylamide gel and then extended with, for example, dC residues for annealing with pBR322 or other suitable cloning vector which has been cleaved by a suitable restriction enzyme, such as Pst I, and extended with dG residues. Alternative means of forming cloning vectors containing the cDNA using other tails and other cloning vector remainder may, of course, also be used but the foregoing is a standard and preferable choice. A suitable host cell strain, typically *Escherichia coli (E. coli)*, is transformed with the annealed cloning vectors, and the successful transformants identified by means of, for example, ampicillin or tetracycline resistance or other phenotypic characteristics residing on the cloning vector plasmid.

Successful transformants are picked and transferred to microtiter dishes or other support for further growth and preservation. Nitrocellulose filter imprints of these growing cultures are then probed with suitable nucleotide sequences containing bases known to be complementary to desired sequences in the cDNA. Several types of probe may be used, preferably synthetic single stranded DNA sequences labeled by kinasing with γ-³²P ATP. The cells fixed to the nitrocellulose filter are lysed, the DNA denatured. and then fixed before reaction with kinased probe. Clones which successfully hybridize are detected by contact with a photoplate, then plasmids from the growing colonies isolated and sequenced by means known in the art to verify that the desired portions of the gene are present.

The desired gene fragments are excised and tailored to assure appropriate reading frame with the control segments when inserted into suitable expression vectors. Typically, nucleotides are added to the 5′ end to include a start signal and a suitably positioned restriction endonuclease site.

Because the inventors have provided the nucleotide sequences of the V_{H}αTAG, the DNA also may be synthetically synthesized, for example, using an Applied Biosystems™ Model 380A DNA Synthesizer, and constructed by standard techniques.

Finally, an exemplary technique for utilizing combination of the above techniques is by splicing with overlap extension using polymerase chain reaction technology, see Horton et al., *supra*. Generally, a synthetically synthesized primer, having a so-called "wagging tail", may be inserted with a selected sequence, for example genomic DNA. Thereafter, the sequences are amplified and spliced together.

### DNA Encoding the C_{H} and C_{L}

The DNA fragment encoding the amino acid sequence of the human constant region may be obtained by screening the chromosomal DNA of cells producing human immunoglobulin.

### Vectors

The desired DNA fragment may be positioned in a biologically functional expression vehicle which may contain appropriate control sequences not present in the selected DNA fragment. By "biologically functional" is meant that the expression vehicle provides for replication and/or expression in an appropriate host, either by maintenance as an extrachromosomal element or by integration into the host genome. A large number of vectors are available or can be readily prepared, and are well-known to skilled artisans.

A number of plasmids, such as those described in Eureopean Published Patent Applns. 0036776, 0048970 and 0051873, have been described which already contain a promoter in reading frame with the gene and compatible with the proposed host cell.

The vectors and methods disclosed herein are suitable for use over a wide range of microorganisms, either prokaryotic or eukaryotic, which are susceptible to transformation. The plasmid will be capable of replicating in the microorganism, particularly a bacterium.

In general, plasmid vectors containing the appropriate promoters, which can be used by the microbial organism for expression of its own protein, also contain control sequences, ribosome binding sites, and transcription termination sites. Generally, the replicon and control sequences which are derived from species compatible with the host cell are used in connection with these hosts.

Smaller or larger SV40 fragments may also be used, provided there is included the approximately 250 base pair (bp) sequence extending from the Hind III site toward the *Pvu* II site located in the viral origin of replication. Further, it is also possible, and often desirable, to utilize promoter or control sequences normally associated with the desired gene sequence, provided such control sequences are compatible with the host cell systems.

Finally, the plasmid should desirably have a gene, a marker gene, that is capable of providing a phenotypical property which allows for selection of host cells containing the expression vector. Particularly useful is a gene that provides for survival selection. Survival selection can be achieved by providing resistance to a growth inhibiting substance or providing a growth factor capability to a bacterium deficient in such capability.

In general, prokaryotes are preferred. For example, pBR322 a plasmid derived from an *E. coli* species [Bolivar, et al., *Gene* 2, 95 (1977)] is particularly useful. pBR322 contains genes for ampicillin and tetracycline resistance and thus provides an easy means for identifying transformed cells.

While these prokaryotes are the most commonly used, other microbial strains which may be used include *E. coli* strains such as *E. coli* B, *E. coli* K12 strain 294 (ATCC No. 31446) and *E. coli* X1776 (ATCC No. 31537), *E. coli* W3110 (F⁻, γ⁻, prototrophic, ATTC No. 27325), bacilli such as *Bacillus subtilus*, and other enterobacteriaceae such as *Salmonella typhimurium* or *Serratia macrcesans*, and various *Pseudomonas* species may be used. These examples are intended to be illustrative only.

In addition to prokaryates, eukaryotic microbes may also be used. *Saccharomyces cerevisiae*, or common baker's yeast, is the most commonly used among eukaryotic microorganisms although a number of other strains are commonly available.

For expression in *Saccharomyces*, the plasmid YRp7, for example, [Stinchcomb, et al., *Nature* 282, 39 (1979); Kingsman et al., *Gene* 7, 141 (1979); Tschemper, et al., *Gene* 10, 157 (1980)] is commonly used. This plasmid already contains the *trpl* gene which provides a selection marker for a mutant strain of yeast lacking the ability to grow in tryptophan, for example ATCC No. 44076 or PEP4-1 [Jones, *Genetics* 85, 12 (1977)]. The presence of the *trpl* lesion as a characteristic of the yeast host cell genome then provides an effective environment for detecting transformation by growth in the absence of tryptophan.

Any plasmid vector containing a yeast-compatible promoter, origin of replication and termination sequence is suitable for use in yeast. Suitable promoting sequences in yeast vectors include the promoters for 3-phosphoglycerate kinase [Hitzeman, et al., *J. Biol. Chem.* 255, 2073 (1980)] or other glycolytic enzymes [Hess, et al., *J. Adv. Enzyme Reg.* 7, 149 (1968); Holland et al., *Biochemistry* 17, 4900 1978)].

For use in mammalian cells, the control functions on the expression vectors are often provided by viral material. For example, commonly used promoters are derived from polyoma. Adenovirus 2, and most frequently Simian Virus 40 (SV40). The early and late promoters of SV40 virus are particularly useful because both are obtained easily from the virus as a fragment which also contains the SV40 viral origin of replication [Fiers, et al., *Nature* 273, 113 (1978)].

For example, pSV2neo contains a gene for ampicillin resistance neomycin resistance, which is under the control of an SV40 promoter. Thus, pSV2neo provides easy means for identifying cells transformed with genes for both the animal variable region and human constant region.

### Preparation of Chimeric DNA

The genes coding for the heavy chain or the light chain will be constructed by joining the 5′-end of a DNA fragment which encodes the constant region to the 3′ end of a DNA fragment which encodes the variable region. The DNA sequence coding for the antibody amino acid sequence may be obtained in association with the promoter and replication site from genomic DNA. To the extent that the host cells recognize the transcriptional regulatory and translational initiation signals associated with the heterologous genes, then the region 5′ and 3′ of the variable region coding sequence may be retained with the variable region coding sequence and employed for transcriptional and translational initiation regulation. The non-coding region 3′ to the constant region may be retained for its transcriptional termination regulatory sequences, such as termination and polyadenylation. In referring to 5′ or 3′ for a double strand, it is intended to mean the direction of transcription, with 5′ being upstream from 3′.

The intron sequence between the variable region for each respective chain may be joined to the corresponding human constant DNA fragment at any convenient restriction site. In providing a fragment encoding the variable region, it will usually be desirable to include a portion of the intron downstream from the J region. Where the intron is retained, it will be necessary that there be functional splice acceptor and donor sequences at the intron termini. The contiguous non-coding region 5′ to the variable region will normally include those sequences involved with initiation of transcription and translation, such as the TATA box, capping sequence and CAAT sequence. Usually, the 5′-non-coding sequence does not exceed about 1-2 kilo bases (kb).

An enhancer sequence should exist between the J region and the constant region. The enhancer employed may be the enhancer of either (1) the animal V region or the (2) the human constant region.

By retaining the 3′-region naturally contiguous to the DNA sequence coding for the constant region, the transcriptional termination signals may be provided for the gene. Where the transcriptional termination signals are not satisfactorily functional in the expression host cell, then a 3′ region functional in the host cell may be substituted. Conveniently, the non-coding 3′ region may be obtained from a non-coding contiguous 3′ region of a constant region from the expression host. The 3′-non-coding region may be joined to the constant region by any of the means described previously for manipulation and ligation of DNA fragments. This region could then be used as a building block in preparing the gene.

### Preparation of Expression Vehicles

Construction of suitable expression vehicles containing the desired coding and control sequences may be produced as follows. The termini of the vectors and DNA fragments may then be religated to form the desired expression vehicles. The methods employed are not dependent on the DNA source, or intended host.

DNA fragments coding for the light chain and heavy chain may be inserted into separate expression vehicle, or into the same vector. Preferably, the fused genes encoding the light and heavy chimeric chains are assembled in two different expression vectors which can be used to cotransform a recipient cell, either concurrently or sequentially.

The means for insertion of the DNA fragments containing the chimeric genes into expression vectors includes using restriction endonucleases. "Restriction endonucleases" (or "restriction enzymes") are hydrolytic enzymes capable of catalyzing site-specific cleavage of DNA molecules. The locus of restriction endonuclease action is determined by the existence of a specific nucleotide sequence. Such a sequence is termed the recognition site for the restriction endonuclease. Many restriction endonucleases from a variety of bacterial species have been isolated and characterized in terms of the nucleotide sequence of their recognition sites. Some restriction endonucleases hydrolyze the phosphodiester bonds on both strands at the same point, producing blunt ends. Others catalyze hydrolysis of bonds separated by a few nucleotides from each other, producing free single stranded regions at each end of the cleaved molecule. Such single stranded ends are self-complementary, hence cohesive, and may be used to rejoin the hydrolyzed DNA. Exemplary restriction enzymes include *Aat* II, *Bam* HI, *Eco* RI, *Hind* III, *Nde* I, *Spe* I, *Xba* I, *Sac* I, *Bgl* II, *Pst* I, *Sal* I and *Pvu* II.

Additionally, the expression vector may have a polylinker inserted therein which has a a plurality of unique restriction sites. By digestion of the expression vector with the appropriate restriction enzymes, the polylinker will be cleaved so that at least one DNA fragment containing the gene can be inserted. Where the polylinker allows for distinguishable termini, the DNA fragment can be inserted in a single orientation; were the termini are the same, insertion of the DNA fragment will result in plasmids having two different orientations.

Cleavage is performed by treating the plasmid with a restriction enzyme(s). In general, about 10 µg plasmid or DNA fragments is used with about 10 units of enzyme in about 100 µl of buffer solution. Endonuclease digestion will normally be carried out at temperatures ranging from 37° to 65°C, at a pH of from 7 to 9. (Appropriate buffers and substrate amounts for particular restriction enzymes are specified by the manufacturers.) Time for the reaction will be from 1 to 18 hours.

It may be useful to prevent religation of the cleaved vector by pretreatment with alkaline phosphatase. Specific conditions are prescribed by the manufacturer.

After the restriction enzyme digest is complete, protein is removed by extraction with phenol and chloroform. The nucleic acid is recovered from the aqueous fraction (containing about 0.3M sodium acetate) by precipitation with about 2.5 volumes of ethanol.

Descriptions of methods of cleavage with restriction enzymes may be found in the following articles: Greene et al., Methods in Molecular Biology, Vol. 9, ed. Wickner, R. B., Marcel Dekker, Inc., New York, "DNA Replication and Biosynthesis"; Mertz and Davis, *Proc. Nat. Acad. Sci., (USA)*, 69, 3370 (1972).

Size separation of the cleaved fragments by agarose gel electrophoresis is readily performed to follow the course of the reaction. Once the digestion has gone to the desired degree, the endonuclease may be inactivated by heating above 65°C for about 10 minutes or organic extraction.

The desired fragment is then purified from the digest. Suitable purification techniques include gel electrophoresis or sucrose gradient centrifugation.

The plasmid vehicle and foreign DNA fragments are then ligated with DNA ligase to recircularize. This process is referred to as annealing and DNA ligation.

An appropriately buffered medium containing the DNA fragments, DNA ligase, and appropriate cofactors is employed. The temperature employed will be between 25° to 4°C. When DNA segments hydrogen bond, the DNA ligase will be able to introduce a covalent bond between the two segments. The time employed for the annealing will vary with the temperature employed, the nature of the salt solution, as well as the nature of the sticky ends or cohesive termini. Generally, the time for ligation may be from 5 to 18 hours. See Maniatis T., *Molecular Cloning*, Cold Spring Harbor, *supra*.

### Host Cells

Thereafter, the expression vehicle constructs may be used to transform an appropriate host cell. Suitable host cells include cells derived from unicellular as well as multicellular organisms.

The chimeric immunoglobulin genes can be expressed in nonlymphoid cells such as bacteria or yeast.

Various unicellular microorganisms can be transformed, such as bacteria. That is, those unicellular organisms which are capable of being grown in cultures or fermentation. Since bacteria are generally the most convenient organisms to work with, bacteria will be hereinafter referred to as exemplary of the other unicellular organisms. Bacteria, which are susceptible to transformation, include members of the Enterobacteriaceae, such as strains of *Escherichia coli*; *Salmonella*; Bacillaceae, such as *Bacillus subtilis*; *Pneumococcus*; *Streptococcus*, and *Haemophilus influenzae.*

When expressed in bacteria, the immunoglobulin heavy chains and light chains become part of inclusion bodies. The chains then must be isolated, purified and then assembled into functional immunoglobulin molecules.

In addition to prokaryates, eukaryotic microbes, such as yeast cultures may also be used. *Saccharomyces cerevisae*, or common baker's yeast is the most commonly used among eukaryotic microorganisms, although a number of other strains are commonly available. The presence of the trpl lesion as a characteristic of the yeast host cell genome provides an effective environment for detecting transformation by growth in the absence of tryptophan.

In addition to microorganisms, cultures of cells derived from multicellular organisms may also be used as hosts. In principle, any such cell culture is workable, whether from vertebrate or invertebrate culture, provided that the cell line is one that at least originally produced antibodies. Propagation of vertebrate cells in culture has become a routine procedure in recent years [*Tissue Culture*, Academic Press, Kruse and Patterson, editors (1973)]. Examples of such useful host cell lines are Sp2/0, VERO and HeLa cells, Chinese hamster ovary (CHO) cell lines, and W138, BHK, COS-7 and MDCK cell lines.

The preferred recipient cell line is a plasmacytoma cell such as B lymphocytes or hybridoma cells. Plasmacytoma cells can synthesize, assemble and secrete immunoglobulins encoded by transformed immunoglobulin genes. Further, they possess the mechanism for glycosylation of the immunoglobulin. Sp2/0 is a preferred recipient cell because it is an immunoglobulin-nonproducing plasmacytoma cell. The cell produces only immunoglobulin encoded by the transformed immunoglobulin genes. Plasmacytoma cells can be grown in culture or in the peritoneum of mice where secreted immunoglobulin can be obtained from ascites fluid.

### Transformation of Host Cells

Transformation of host cells is accomplished as follows. The expression vehicle is linearized and the DNA is inserted into host cells for production of the antibody. Exemplary methods for inserting the DNA into host cells include electroportion, protoplast fusion, calcium phosphate-precipitation, or other conventional techniques, which use dextran sulfate, and PEG.

If cells without formidable cell wall barriers are used as host cells, transformation may be carried out by the calcium phosphate precipitation method as described by Graham and Van der Eb, *Virology*, 52, 546 (1978).

If prokaryotic cells or cells which contain substantial cell wall constructions are used, the preferred method of transformation is calcium treatment using calcium chloride as described by Cohen, F.N. et al, *Proc. Natl. Acad. Sci. (USA)* 69, 2110 (1972).

The host cells may be transformed via either co-transformation or targeted transformation.

For co-transformation, the genes coding for the light chain and heavy chain may be used to transform separate cell cultures, either of the same or of differing species; separate plasmids for light and heavy chain may be used to co-transform a single cell culture; or finally, a single expression plasmid containing both genes and capable of expressing the genes for both light and heavy chain may be transformed into a single cell culture.

In the targeted transformation technique, the host cells are transformed with genes encoding for the light chain, and the cells containing the light chain marker are selected. The light chain is found using cytostaining or possibly by detection of the light chain in the supernatant if it has been secreted. Cells selected to have the light chain are transformed with the heavy chain construct, and resultant cells additionally containing the heavy chain marker selected.

It is known that some immortalized lymphoid cell lines, such as plasmacytoma cell lines, in their normal state secrete isolated Ig light or heavy chains. Consequently, if such a cell line is transformed with the vector containing the chimeric heavy or light chain of the present invention, it will not be necessary to transform the cell line or another cell line with the other Ig chain, provided that the normally secreted chain is complementary to the variable domain of the Ig chain encoded by the vector initially used to transform the cell line.

### Selection and Expression of Transformed Host Cells

Generally, after transformation of the host cells, the cells may be grown for about 48 hours to allow for expression of marker genes. The cells are then placed in a selective medium, where untransformed cells are killed, leaving only cells transformed with the DNA constructions.

Heavy and light chains or portions thereof, may be produced in isolation from each other and antibodies and fragments thereof may be obtained. Such preparations require the use of techniques to reassemble isolated chains.

The ability of the method of the invention to produce heavy and light chains or portions thereof, in isolation from each other offers the opportunity to obtain unique assemblies of immunoglobulins, Fab regions, and univalent antibodies. It is possible to recombine the heavy and light chains *in vitro*, disrupted by cleavage of only the interchain disulfides, and to regain antibody activity even without restoration of the inter-chain disulfides [see Edelman, G.M., et al., *Proc. Natl. Acad. Sci. (USA)* 50, 753 (1963)].

The transformed cells are grown under conditions appropriate to the production of the light chains and/or heavy chains, and assayed for heavy and/or light chain protein synthesis. Exemplary assay techniques include enzyme-linked immunosorbent assay (ELISA), radioimmunoassay (RIA), or flourescence-activated cell sorter analysis (FACS), immunohistochemistry and the like.

The binding affinity of monoclonal antibodies for TAG72 is determined by means well known in the art (see Heyman, B. et al. *J. Immunol. Methods* 68, 193-204 (1984) and as described in detail in the Examples provided hereinafter).

Selected positive cultures are subcloned in order to isolate pure transformed colonies. A suitable technique for obtaining subclones is via the limited dilution method taught by McKeara in *Monoclonal Antibodies*, Plenum Press, N.Y. (1980).

Hybridomas that produce such chimeric antibodies may be grown using known procedures. The transformed cells can secrete large quantities of the light chains and/or heavy chains by culture *in vitro*, such as by hollow fiber systems, spinner culture, static culture; or *in vivo* such as ascites production.

The chimeric antibodies may be produced in large quantities by injecting a hybridoma into the peritoneal cavity of pristane-primed mice, and after an appropriate time (about 1-2 weeks), harvesting ascites fluid from the mice, which yields a very high titer of homogeneous monoclonal antibody, and isolating the monoclonal antibodies therefrom by methods well known in the art [see Stramignoni, P. et al., *Intl. J. Cancer* 31, 543-552 (1983)]. The hybridomas are grown up *in vivo*, as tumors in animals, the serum or ascites fluid of which can provide up to about 50 mg/mL of monoclonal antibodies. Usually, injection (preferably intraperitoneal) of about 10⁶ to 10⁷ histocompatible hybridoma cells into mice or rats will result in tumor formation after a few weeks. The antibodies can then be collected and processed by well known methods. (See generally, Immunological Methods, vols. I & II, eds. Lefkovits, I. and Pernis, B., (1979 & 1981) Academic Press, New York, N.Y.; and Handbook of Experimental Immunology, ed. Weir, D., (1978) Blackwell Scientific Publications, St. Louis, MO.)

The antibodies can then be stored in various buffer solutions such as phosphate buffered saline (PBS), which gives a generally stable antibody solution for further use.

The chimeric antibodies of the present invention may be fragmented using known protease enzymes, for example papain and pepsin, to obtain highly immunoreactive F(ab′)2. F(ab′) and Fab fragments. In addition, active fragments of Ig formed by proteolysis (approximately 50,000 MW) can be split into their fully reduced heavy chain and light chain components and fairly efficiently reconstructed to give an active antibody [Haber, E., *Proc. Natl. Acad. Sci. (USA)* 52, 1099 (1964); Whitney, P.L., et al., *Proc. Natl. Acad. Sci. (USA)* 53, 524 (1965)]. The reactivity of the resulting F(ab′)2, F(ab′) and Fab fragments are determined by methods as described above for the complete monoclonal antibody molecule.

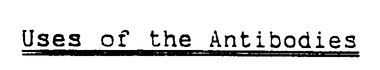

The antibodies of the present invention, as well as immunoreactive fragments or recombinants thereof, provide unique benefits for use in a variety of cancer treatments. In addition to the ability to bind specifically to malignant cells and to localize tumors, the antibodies have constant variable regions which do not bind detectably to normal cells such as fibroblasts, endothelial cells, or epithelial cells in the major organs.

Specifically, the antibodies, immunoreactive fragments or recombinants thereof are useful for, but not limited to, the following types of cancer treatment: (1) *in vivo* diagnostic assays conjugated to an imaging marker, for the *in situ* detection of carcinoma lesions, as further described below; (2) *in vivo* therapy, using the antibodies of the present invention alone or conjugated to a therapeutic agent such as a radionuclide, toxin, effector cells, other antibodies or via a complement mechanism, as described below; and (3) radioimmunoguided surgery, as described below.

Moreover, a pharmaceutical composition comprising the antibodies of the present invention in a pharmaceutically acceptable, non-toxic, sterile carrier such as physiological saline, non-toxic buffers and the like, is also now possible.

Injectable compositions of the present invention may be either in suspension or solution form. In solution form the complex (or when desired the separate components) is dissolved in a pharmaceutically acceptable carrier. Such carriers comprise a suitable solvent, preservatives such as benzyl alcohol, if needed, and buffers. Useful solvents include, for example, water, aqueous alcohols, glycols, and phosphonate or carbonate esters. Such aqueous solutions contain no more than 50% of the organic solvent by volume.

Injectable suspensions as compositions of the present invention require a liquid suspending medium, with or without adjuvants, as a carrier. The suspending medium can be, for example, aqueous polyvinylpyrrolidone, inert oils such as vegetable oils or highly refined mineral oils, or aqueous carboxymethlycellulose. Suitable physiologically acceptable adjuvants, if necessary to keep the complex in suspension, may be chosen from among thickeners such as carboxymethylcellulose, polyvinylpyrrolidone, gelatin, and the alginates. Many surfactants are also useful as suspending agents, for example, lecithin, alkylphenol, polyethylene oxide adducts, naphthalenesulfonates, alkylbenzenesulfonates, and the polyoxyethylene sorbitan esters. Many substances which effect the hydrophibicity, density, and surface tension of the liquid suspension medium can assist in making injectable suspensions in individual cases. For example, silicone antifoams, sorbitol, and sugars are all useful suspending agents.

Because cancer cells are heterogeneous and consequently, a single monospecific chimeric antibody may not be able to recognize all cells expressing different epitopes of a tumor.

Thus, it may be desirable to administer several different chimeric antibodies of the present invention. The sequential use of these various antibodies should substantially reduce the anti-idiotypic responses in human patients when compared to repeated use of a single antibody. For example, CH92, CH88, and CH44 could be sequentially administered to a patient. Since these antibodies have different light chains and, in fact different CDR3 regions anti-idiotypic responses should be minimized.

### In Vivo Diagnostic Assays

*In vivo* diagnostic assays of human tumors or metastasis thereof using the antibodies, immunoreactive fragments or recombinants thereof are conjugated to a marker, administered to a patient, and then the presence of the imaging marker in the patient is detected by exposing the patient to an appropriate detection means.

Administration and detection of the antibody-imaging marker conjugate as well as methods of conjugation of the antibody to the imaging marker are accomplished by methods readily known or readily determined, as described, for example, in Goldenberg, D.M. et al., *New England J. Med.*, 298, 1384-1388 (1978); Goldenberg, D.M. et al., *J. Amer. Med. Assoc.* 280, 630-635 (1983); Goldenberg, D.M. et al., *Gastroenterol.* 84, 524-532 (1983); Siccardi A.G. et al., *Cancer Res.* 46, 4817-4822 (1986); Epenetos, A.A. et al., *Cancer* 55, 984-987 (1985); Philben, V.J. et al., *Cancer* 57, 571-576 (1986); Chiou, R. et al., *Cancer Inst.* 76, 849-855 (1986); Colcher, E. et al., *Cancer Res.*, 43, 736-742 (1983); Colcher, E. et al., Laboratory Research Methods in Biology and Medicine Immunodiagnostics, New York, Alan R. Liss, pp. 215-258 (1983); Keenan, A.M. et al., *J. Nucl. Med.* 25, 1197-1203 (1984); Colcher D. et al., *Cancer Res.* 47, 1185-1189 (1987); Estaban, J.M. et al., *Intl. J. Cancer* 39, 50-59 (1987); Martin, D.T., et al., *Curr. Surg.* 41, 193-194 (1984); Martin, E.W. Jr. et al., *Hybridoma* 5, S97-S108 (1986); Martin, D.T. et al., *Am. J. Surg.* 150, 672-675 (1985); Meares et al., *Anal. Biochem.* 142, 68-78 (1984); and Krejcarek et al., *Biochem. and Biophys. Res. Comm.* 77, 581-585 (1977).

The dosage will vary depending upon the age and weight of the patient. Generally, the dosage should be effective to visualize or detect tumor sites, distinct from normal tissues. Preferably, a one-time dosage will be between 0.1 to 200 mg of an antibody-marker conjugate per patient.

Examples of imaging markers which can be conjugated to the antibody are well known to those skilled in the art and include substances which can be detected by diagnostic imaging using a gamma scanner or hand held gamma probe or Positron Emission Tomography or the like, as described in the references cited above, and substances which can be detected by nuclear magnetic resonance imaging using a nuclear magnetic resonance spectrometer or the like, as described in the references cited above.

Suitable examples of substances which can be detected using a gamma scanner or the like include, for example, radioisotopes such as ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re, and ^{99m}Tc. ¹²⁵I, ¹²³I, ¹⁵³Sm and ^{99m}Tc are preferred due to their low energy and suitability for long range detection.

An example of a substance which can be detected using a nuclear magnetic resonance spectrometer or the like is gadolinium (Gd).

### In Vivo Cancer Treatment

In this method, the antibody-therapeutic agent conjugate can be delivered to the carcinoma site thereby directly exposing the carcinoma tissue to the therapeutic agent.

The antibodies of the present invention, immunoreactive fragments or recombinants thereof may be administered in a pharmaceutically effective amount for the *in vivo* treatment of human carcinomas or metastasis thereof. A "pharmaceutically effective amount" of the antibody, immunoreactive fragment or recombinant thereof, conjugated or unconjugated to a therapeutic agent, means the amount of said antibodies in the pharmaceutical composition should be sufficient to achieve effective binding with the antigens against which said antibodies have specific affinity. The pharmaceutical composition may be administered in a single or multiple dosage.

Methods of preparing and administering conjugates of the antibody, immunoreactive fragments or recombinants thereof and a therapeutic agent are well known or readily determined by those skilled in the art. Moreover, suitable dosages will depend on the age and weight of the patient and the therapeutic agent employed and are well known or readily determined by those skilled in the art. Representative protocols are described in the references cited below.

Examples of the antibody-therapeutic agent conjugates which can be used in therapy include the following: (1) antibodies coupled to radionuclides, such as ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I and ¹¹¹In as described, for example, in Goldenberg, D.M. et al., *Cancer Res.* 41, 4354-4360 (1981); Carrasquillo, J.A. et al., *Cancer Treat. Rep.* 68, 317-328 (1984); Zalcberg, J.R. et al., *J. Natl. Cancer Inst.* 72, 697-704 (1984); Jones, D.H. et al., Int. *J. Cancer* 35, 715-720 (1985); Lange, P.H. et al., *Surgery* 98, 143-150 (1985); Kaltovich, F.A. et al., *J. Nucl. Med.* 27, 897 (1986), Order, S.E. et al., *Int. J. Radiother. Oncol. Biol. Phys.* 8, 259-261 (1982), Courtenay-Luck, N. et al., *Lancet* 1, 1441-1443 (1984) and Ettinger, D.S. et al., *Cancer Treat. Rep.* 66, 289-297 (1982); (2) antibodies coupled to drugs or biological response modifiers such as methotrexate, adriamycin, and lymphokines such as interferon as described, for example, in Chabner, B. et al., Cancer, Principles and Practice of Oncology, Philadelphia, PA, J.B. Lippincott Co. Vol. 1, pp. 290-328 (1985); Oldham, R.K. et al., Cancer, Principles and Practice of Oncology, Philadelphia, PA, J.B. Lippincott Co., Vol. 2, pp. 2223 -2245 (1985); Deguchi, T. et al., *Cancer Res.* 46, 3751 -3755 (1986); Deguchi, T. et al., *Fed. Proc.* 44, 1684 (1985); Embleton, M.J. et al., *Br. J. Cancer* 49, 559-565 (1984) and Pimm, M.V. et al., *Cancer Immunol. Immunother.* 12, 125-134 (1982); (3) antibodies coupled to toxins, as described, for example, in Uhr, J.W. et al., Monoclonal Antibodies and Cancer, Academic Press, Inc., pp. 85-98 (1983), Vitetta, E.S. et al., Biotechnology and Bio. Frontiers, Ed. P.H. Abelson, pp. 73-85 (1984) and Vitetta, E.S. et al., *Sci.*, 219, 644-650 (1983); (4) heterofunctional antibodies, for example, antibodies coupled or combined with another antibody so that the complex binds both to the carcinoma and effector cells, e.g., killer cells such as T cells, as described, for example, in Perez, P. et al., *J. Exper. Med.* 163, 166-178 (1986); and Lau, M.A. et al. *Proc. Natl. Acad. Sci. (USA)* 82, 8648-8652 (1985); and (5) native, i.e., non-conjugated or non-complexed, antibodies, as described in, for example, Herlyn, D. et al., *Proc. Natl. Acad. Sci., (USA)* 79, 4761-4765 (1982); Schulz, G. et al., *Proc. Natl. Acad. Sci., (USA)* 80, 5407-5411 (1983); Capone, P.M. et al., *Proc. Natl. Acad. Sci., (USA)* 80, 7328-7332 (1983); Sears, H.F. et al., *Cancer Res.* 45, 5910-5913 (1985); Nepom. G.T. et al., *Proc. Natl. Acad. Sci., (USA)* 81, 2864-2867 (1984); Koprowski, H. et al., *Proc., Natl. Acad. Sci., (USA)* 81, 216-219 (1984); and Houghton, A.N. et al., *Proc. Natl. Acad. Sci.,(USA)* 82, 1242-1246 (1985).

The methods for combining the antibody or antibody fragment to a desired therapeutic agent as described above are conventional and well known in the art. For example, the methods given in the references above.

### RadioImmunoguided Surgery

Antibodies, immunoreactive fragments or recombinants thereof, are important for radioimmunoguided surgery (RIGS). In RIGS, an intraoperative therapy, tumors are localized and excised. An antibody labeled with an imaging marker is injected into the patient, and bound antibody localized by a hand-held gamma detecting probe (GDP) and excised. An exemplary GDP is Neoprobe™, commercially available from Neoprobe™ Corporation, Tampa, FL. See Martin et al., "Radioimmunoguided surgery: a new approach to the intraoperative detection of tumor using antibody B72.3", *Amer. J. Surg.* 156, 386-392 (1988); Martin et al. "Radioimmunoguided surgery: intraoperative use of antibody 17-1A in colorectal cancer", *Hybridoma* 5, S97-S108 (1986).

Administration and detection of the antibody-imaging marker conjugate as well as methods of conjugation of the antibody to the imaging marker are accomplished by methods readily known or readily determined by one skilled in the art, as described, for example, above.

The dosage will vary depending upon the age and weight of the patient, but generally a one time dosage of 0.1 to 200 mg of antibody-marker conjugate per patient is sufficient.

The following nonlimiting examples are merely for illustration of the construction and expression of chimeric DNA sequences encoding the antibodies of this invention. All temperatures not otherwise indicated are in Centigrade. All percents not otherwise indicated are by weight.

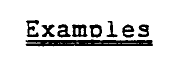

### Replacement of Mouse Constant Regions

CC antibodies were derived from mice, and are significantly less capable of carrying out the effector functions possessed by the human constant regions.

Consequently, in the following examples, selected antibodies are "humanized" by genetically removing the constant regions of the heavy and light chains and replacing them with their human equivalents.

The mouse light chain constant region genes were replaced with the human kappa (k) gene, and the mouse heavy chain genes were replaced with each of the four human gamma isotypes (γ1, γ2, γ3 and γ4). Each of these four gamma isotypes possess unique biological properties. For a general review, see "The Human IgG subclasses", Hamilton, R.G. (1989) Doc. No. CB0051-289, Calbiochem Corporation.

### Preparation of Heavy and Light Chain Variable Region

### Isolation of CC49 light chain

CC49 hybridoma cells secrete an antibody having an IgG₁ isotype heavy chain and a kappa light chain.

Total DNA from CC49 hybridoma cells, Balb/C mouse kidney cells and NSI plasmacytoma cells was isolated according to the procedures set forth in *Cell*; 24, 353-356 (1981).

Generally, about 10-20 µg of the extracted DNA from each cell line was digested to completion with 80 units of *Bam* HI, *Eco* RI, *Hind* III, *Spe* I, *Xba* I, *Sac* I, *Bgl* II, and *Pst* I in 50-100 microliters of a reaction mixture containing the appropriate reaction buffer at 37°C overnight.

Next, the total extracted DNA from each cell line was subjected to the Southern hybridization technique, developed by E. M. Southern, [*J. Mol. Biol.* 98, 503-517 (1975)]. The DNA fragments were fractionated on the basis of their size by means of electrophoresis on a 0.8% agarose gel. The double-stranded DNA fragments were modified into single-stranded DNA fragments in an alkali solution; and then a nitrocellulose filter was placed into close contact with the gel to transfer the modified DNA segments onto the filter in the presence of a high salt concentration solution.

Hybridization was carried out using, as the probe, a random primed 〈32P〉-labelled L chain.

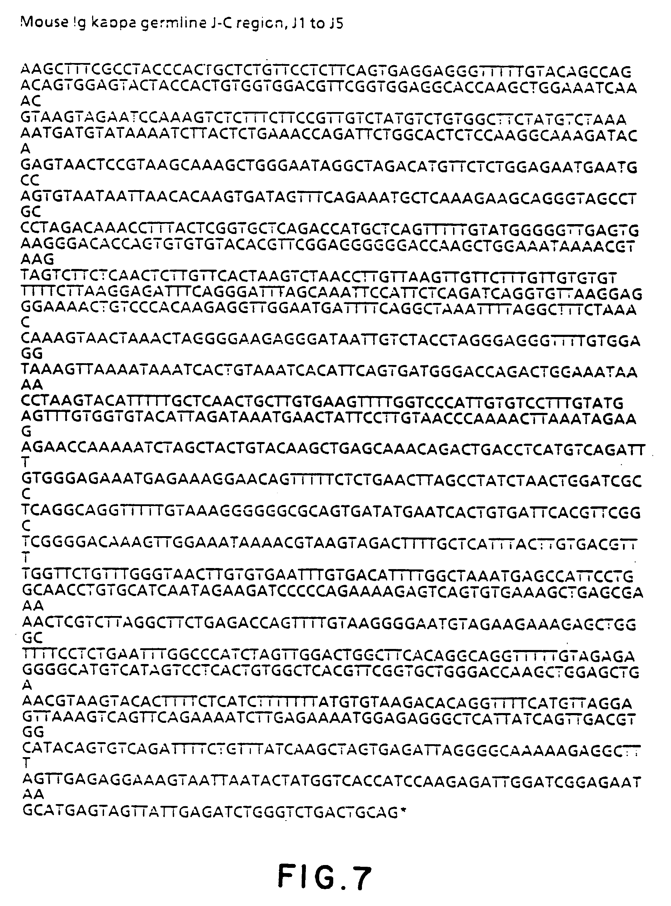

More specifically, the probe was a 1.71 kilo base pair (kbp) *Hind* III-*Pst* I fragment containing the coding exons for the murine J_{L} regions (J1-J5) and was isolated from the plasmid pGD1. A nucleotide sequence of the probe fragment is provided in Figure 7. This plasmid is described in "Site Directed Cleavage of Immunoglobulin Gene Segments by Lymphoid Cell Extracts", Agostaro et al., *Can. J. Biochem. Cell Biol.* 63, 969-976 (1985). The plasmid was provided by Nobumichi Hozumi and John Roder, Mt. Sinai Research Institute, Toronto, Ontario, Canada.

To radiolabel the probe, alpha〈32〉 dCTP was obtained from Amersham, Arlington Heights, IL, USA, and the random priming kit was obtained from Pharmacia, Piscataway, NJ, USA.

The signals in Southern transfers were visualized by autoradiography using Kodak X-OMAT™ AR film. No obviously rearranged band was observed. Thus, relative to the standards, no unique band was detected on the autoradiogram for the CC49 DNA digested with *Hind* III. It could not be ruled out from the Southern data, however, that the rearranged band for the L chain was masked by a band migrating in the CC49 *Hind* III digested DNA parallel to the band resulting from a *Hind* III digest of mouse kidney cell DNA (representing the germline DNA). This actually turned out to be the case.

### Preparation of Plasmid Containing Mouse V_{L} Genes

LAMBDA-ZAP™, a lambda-based insertion cloning vector capable of self excision, was purchased from Stratagene Co., La Jolla, CA, USA. LAMBDA-ZAP™ is described on pages 20-21 of the 1987 Stratagene catalog. The cohesive (cos) ends of LAMBDA-ZAP™ were ligated overnight by following the manufacturer's protocol.

Twenty micrograms of the ligated LAMBDA-ZAP™ were digested with 5 microliters (15 units) of *Spe* I, purchased from New England Biolabs, Inc. The total volume of the digest was 100 microliters. After 55 minutes of digestion, another 6 units of *Spe* I were added. After 70 minutes, the reaction was stopped by phenol extraction and ethanol precipitation carried out as per Stratagene's protocol.

Digestion with *Spe* I restriction enzyme results in production of "sticky ends" at both termini. These sticky ends were modified with T4 DNA polymerase to create half filled-in *Spe* I sticky ends, e.g., 5′ACT/3′TCATG. To accomplish the half fill-in reaction, the DNA pellet obtained in the ethanol precipitation above was dissolved in 8 microliters of water. To this was added 2 microliters of 10 millimolar dTTP, 2 microliters of 10 millimolar dCTP, 2 microliters of Stratagene's 10X ligase buffer, 4 microliters of reionized, distilled water, and 2 microliters of a Klenow fragment from Bethesda Research Laboratories (BRL). The reaction was carried out at ambient temperatures for 30 minutes. The reaction was stopped by inactivating the DNA polymerase at 65°C for 10 minutes.

One hundred sixty micrograms of total CC49 hybridoma DNA (containing the mouse light chain promoter and the L and VJ exons) were digested to completion with *Hind* III. Fragments between about 1 kb to about 20 kb were cut out of 0.8% agarose gels. The DNA was purified using GENECLEAN™, which is commercially available from BIO 101 (La Jolla, CA, USA).

The total CC49 hybridoma DNA *Hind* III digested fragments were half-filled similarly to the LAMBDA-ZAP™'s *Spe* I fragments with the exception that dATP and dGTP were employed. The half-filled *Hind* III digested fragments produced 5′AGCTT/3′GAA sticky ends, which are compatible with the *Spe* I half-filled λ LAMBDA-ZAP™ fragment above.

After phenol extraction and ethanol precipitation, according to the teachings of *Maniatis*, the total CC49 hybridoma *Hind* III modified and LAMBDA-ZAP™ *Spe* I modified DNA fragments were ligated by means of T4 DNA ligase. The ligation reaction was set using a 6.1 microliter ligation mixture containing the following: about 0.2 micrograms of the total CC49 hybridoma *Hind* III modified DNA in a 3 microliter solution, about 1 microgram of LAMBDA-ZAP™ *Spe* I modified DNA in a 1 microliter solution. 0.6 microliters of Stratagene's 10X ligase buffer, 0.5 microliters 10 millimolar ATP, and 1 microliter of Stratagene ligase. This was incubated overnight in a water bath and the temperature lowered incrementally from about 18°C to about 4°C. This ligation eliminated both the *Hind* III and the *Spe* I sites.

A genomic library of ligated mix was made according to Stratagene's protocol. Briefly, 2 microliters of the ligation mix produced above was used in Stratagene's Gigapack Gold packaging system, following the directions of the manufacturer. Fifteen 150 mm plates having a density of 50,000 plaques per plate were screened, am per manufacturer's directions, for positive clones by hybridization to nitrocellulose filters, obtained from Schleicher-Schuell, Keene, NH, USA. The 〈32P〉 random-labelled probe derived from pGD1, which was described above, was used for hybridization. Two positive clones were obtained.

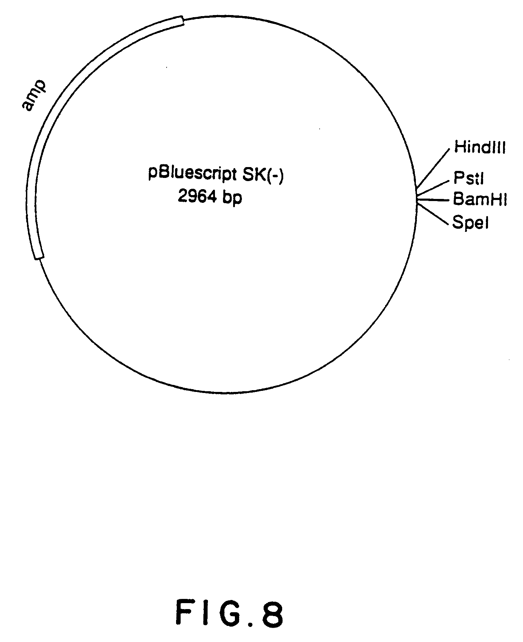

Each clone was plaque purified and recombinant plasmids (phagemids) of LAMBDA-ZAP™ containing the CC49 L chain variable region were obtained by using Stratagene's automatic excision protocol. The vector portion of the resulting recombined plasmid is called pBLUESCRIPT SK(-) and consists of 2964 bp as described in the 1987 Stratagene catalog. A plasmid map of pBLUESCRIPT SK(-) is shown in Fig. 8.

The DNA from the two positive clones was partially sequenced and both were identical. One of the clones, which was named pRL101, was used for further studies.

### Restriction Mapping of CC49 Light Chain

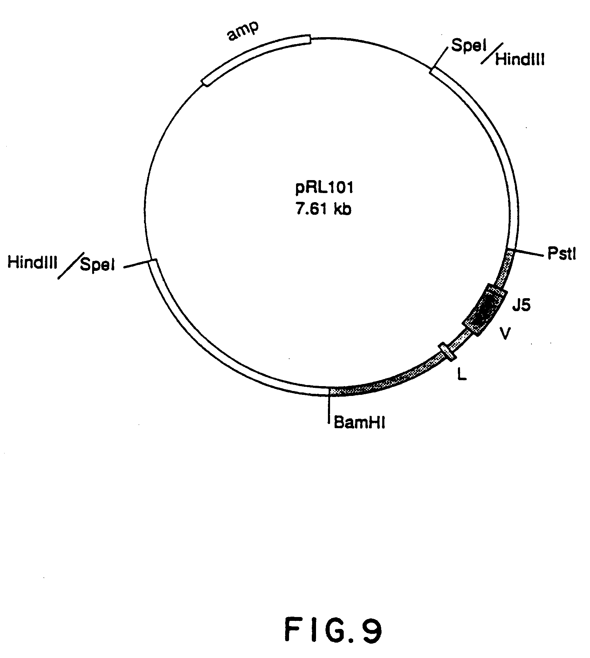

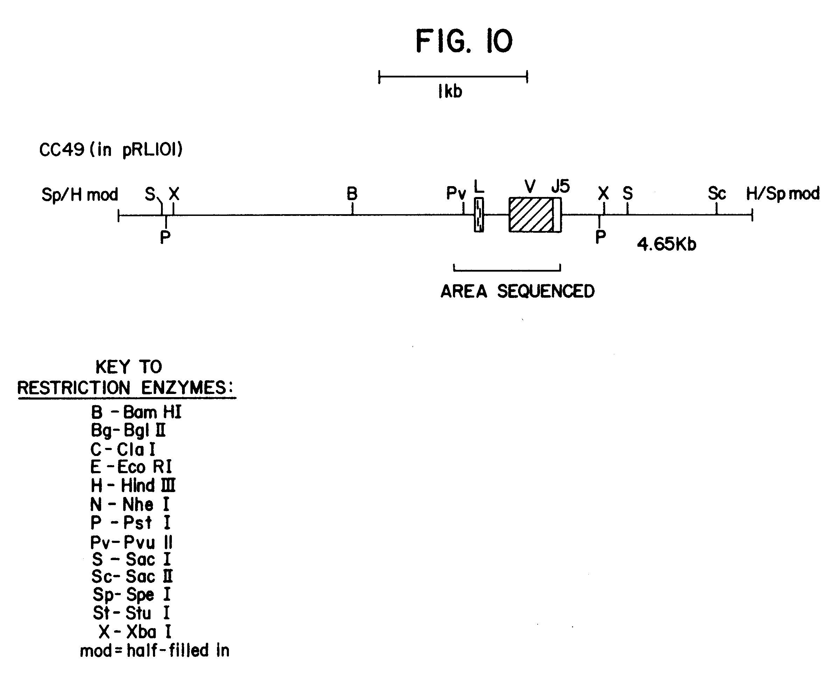

pRL101 was 7.61 kb, and the size of the DNA insert was determined by restriction enzyme mapping to be 4.65 kb. A plasmid map of pRL101 is shown in Figure 9. A restriction enzyme map of the CC49 L chain genomic DNA insert in pRL101 is shown in Fig. 10.

### Isolation of CC83 Light Chain Variable Region

The procedures used to isolate the CC83 light chain were essentially those used to isolate the CC49 light chain, with the following exception.

A genomic library containing 7 X 10⁵ plaques was screened using as the probe the 〈32P〉 random-labelled 1.71 *Hind* III-*Pst* I fragment derived from pGD1, as described above. One positive clone was obtained. The positive clone was named pRL200.

### Restriction Mapping of CC83 Light Chain

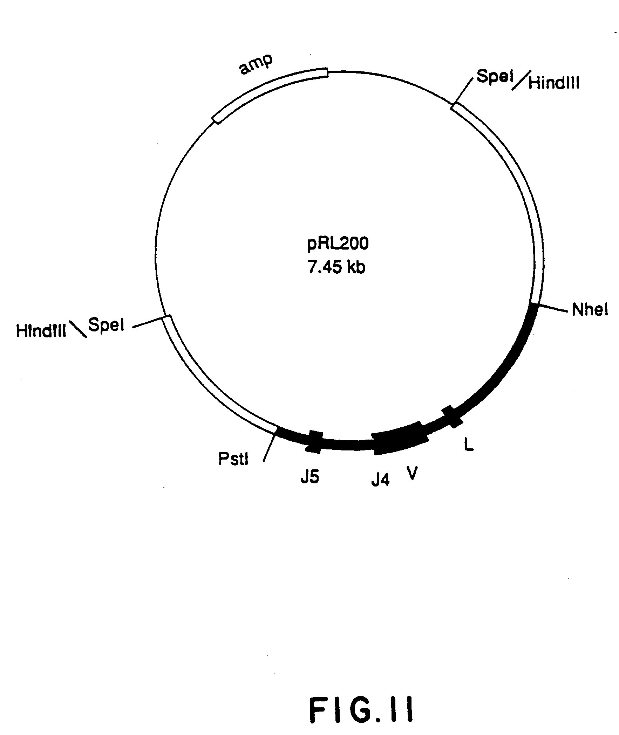

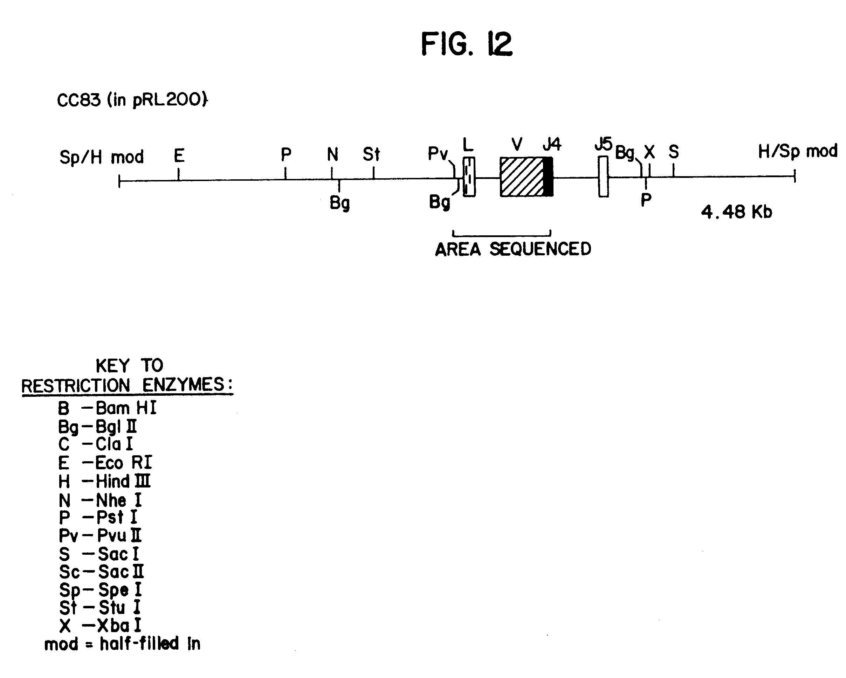

pRL200 was 7.44 kb, and the size of the DNA insert was determined by restriction enzyme mapping to be 4.48 kb. A plasmid map of pRL200 is shown in Figure 11. A restriction enzyme map of the CC83 L chain genomic DNA insert in pRL200 is shown in Fig. 12.

### Isolation of CC49 Heavy Chain Variable Region

The procedures used to isolate the CC49 heavy chain were essentially those used to isolate CC49 light chain, including the screening of the same CC49 *Hind* III modified DNA.

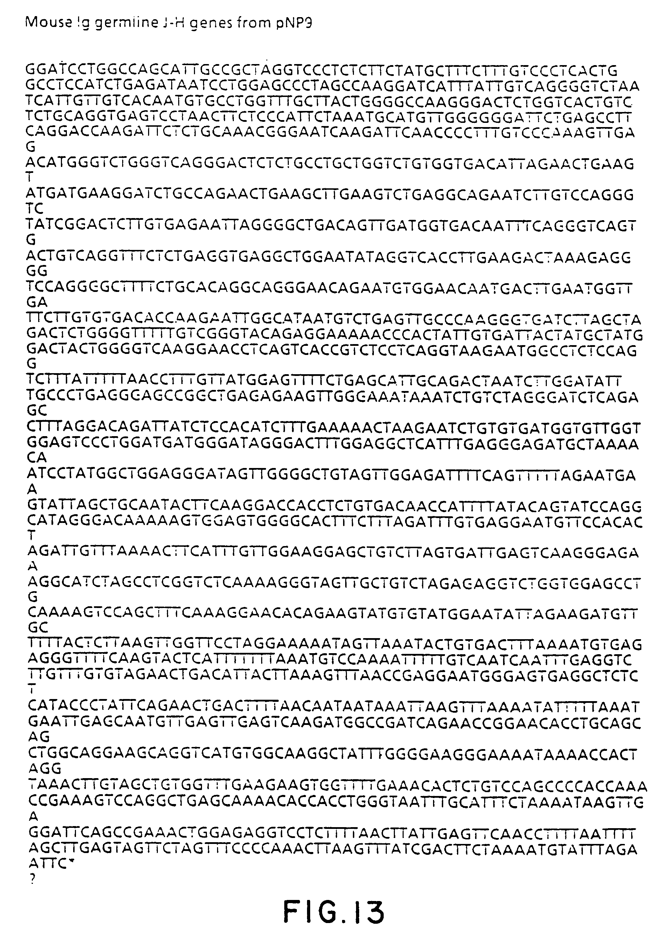

The hybridization probe used to screen the library was generated from pNP9, which contains a 1.98 kbp *Eco* RI-*Bam* HI fragment containing the coding exons for J_{H}3 and J_{H}4 of the CC49 immunoglobulin heavy chain. The nucleotide sequence of the probe fragment is provided in Figure 13. The plasmid was provided by Dr. Nobumichi Hozumi and Dr. John Roder, Mt. Sinai Research Institute, Toronto, Ontario, Canada.

A genomic library containing 9.5 X 10⁵ plaques was screened, from which one positive clone was obtained. The positive clone was named pHH49.

### Restriction Mapping of CC49 Heavy Chain

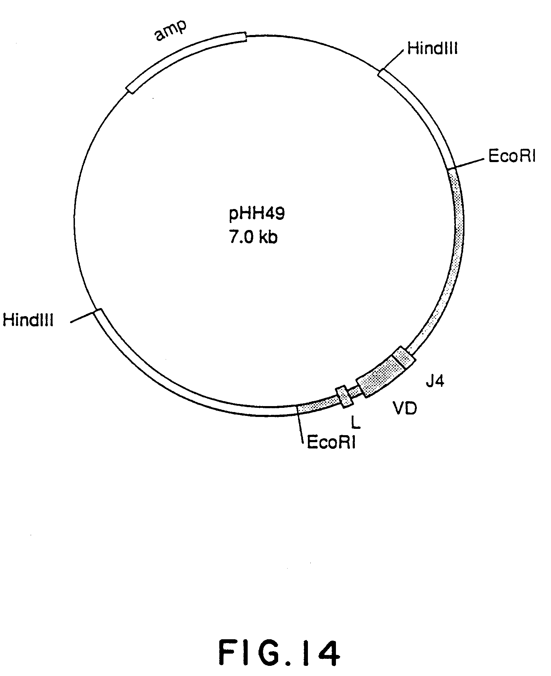

pHH49 was about 7.0 kb, and the size of the DNA insert was determined by restriction enzyme mapping to be about 4.0 kb. A plasmid map of pHH49 is shown in Figure 14.

### Isolation of CC83 Heavy Chain Variable Region

The procedures used to isolate the CC83 heavy chain were essentially those used to isolated CC49 heavy chain, with the following exceptions.

About thirteen micrograms of ligated LAMBDA-ZAP™ vector DNA were digested with 12 units of *Spe* I, purchased from New England Biolabs, Inc., in a total of 100 microliters of an appropriate buffer. The LAMBDA-ZAP™ was digested at 37°C for one hour. The reaction mixture was phenol extracted and ethanol precipitated as per Stratagene's protocol. The *Spe* I digested LAMBDA-ZAP™ was dephosphorylated according to procedures set forth in *Maniatis* except that 40 fold excess of calf intestinal alkaline phosphatase (Boehringer Mannheim, Indianapolis, IN, USA) was used.

DNA from CC83 was digested to completion with *Spe* I. Fragments between about 3 kb to about 40 kb were isolated from a 0.8 percent agarose gel slice by electroelution as described by Maniatis, and ligated with the dephosphorylated *Spe* I-cut LAMBDA-ZAP™ vector.

A genomic library containing 5 X 10⁵ plaques was screened using the probe generated from pNP9, the sequence of which is provided in Figure 13. One positive clone was obtained. The positive clone was named pHS83.

### Restriction Mapping of CC83 Heavy Chain

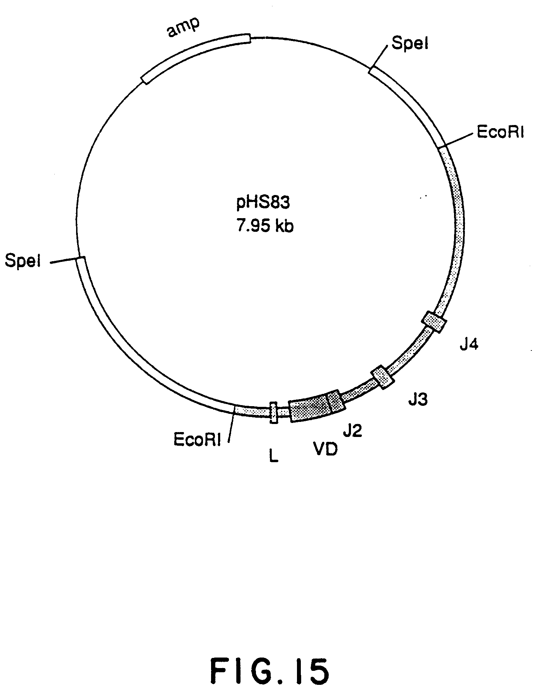

pHS83 was 7.95 kb, and the size of the DNA insert was determined by restriction enzyme mapping to be about 5 kb. A plasmid map of pHS83 is shown in Figure 15.

### Sequencing of CC46, CC49, CC83 and CC92 mRNA

Total RNA from about 1 X 10⁷ CC49 cells frozen at -70°C was extracted essentially as reported by *Maniatis*, with the following exceptions. Four molar guanidinium isothiocyanate and 2.5 molar sodium citrate, pH 7.0, and a SW40Ti rotor centrifuged at 31,000 rpm were used.

A total of 2.7 mg of CC49 RNA was isolated. After centrifugation, poly A+ mRNA was purified from about 1.68 mg of RNA by oligo(dT)-cellulose chromatography using Type 3 oligo(dT)-cellulose obtained from Collaborative Research, Inc., Bedford, MA, USA. The procedure was as described by Aviv and Leder, *Proc. Nat'l. Acad. Sci. (USA)* 69, 1408 (1972). A total of 50.24 µg of poly A+ mRNA was obtained from 1.68 milligrams of mRNA.

A total of 3.82 mg of CC83 RNA was isolated from approximately 1 X 10⁷ cells. A total of 54.6 µg of poly A+ mRNA was isolated from 1.91 milligrams of total RNA.

A total of 0.814 mg of CC92 RNA was isolated from approximately 2.6 x 10⁸ cells. A total of 41.88 micrograms of poly A+ RNA was isolated from 0.814 mg of total RNA.

A total of 1.7 mg of CC46 RNA was isolated from approximately 2.89 x 10⁸ cells. A total of 68.88 micrograms of poly A+ RNA was isolated from 1.7 mg of total RNA.

Synthetic oligonucleotide primers were synthesized using an Applied Biosystems' (Applied Biosystems (ABI), Foster City, CA, USA) Model 380A DNA synthesizer, by phosphoramadite-based chemistry as specified by ABI. The oligonucleotides were purified, as specified by the manufacturer, after electrophoresis on a 20% polyacrylamide gel containing 7M urea. Oligonucleotide concentrations were determined spectrophotometrically at an optical density of 260 nm, where 1 OD 260 nm unit is equal to 33 µg/mL of single-stranded DNA.

The following oligonucleotide primers were made for mRNA sequencing: (1) For the CC49, CC83 and CC92 light chains, K_{L}(-), a 22-mer:
5′-GGAAGATGGATACAGTTGGTGC-3′
complimentary to the coding sequence of the 5′ end of the constant region for mouse immunoglobulin kappa chains, is used to determine the 3′ most mRNA sequence of the light chain variable region.

Additionally, or CC49 light chain, 49FR1(-), a 17-mer:
5′-GGAAGATGGATACAGTTGGTGC-3′
was used to determine the remaining sequence.

Additionally, for CC83 light chain, J4(-), a 24-mer:
5′-CCAACTTTGTCCCCGAGCCGAACG-3′
and also 83L CDR2(-), a 17-mer:
5′-CAGGGACTCCAGTGTGC-3′
was used to determine the remaining sequence.

Additionally, for CC92 light chain, J5(-):
5′-CGTTTCAGCTCCAGCTTGGTCCC-3′
was used to determine the remaining sequence.

For the CC46, CC49, CC83, and CC92 γ1 heavy chains, CH1(-), a 24-mer:
5′-ATGGAGTTAGTTTGGGCAGCAGAT-3′
complimentary to the coding sequence of the 5′ end of the murine γ1 heavy chain constant region. The CH1 (-) 24-mer is used to determine the 3′-most mRNA sequence of heavy chain variable regions.

Additionally, for the CC49 heavy chain, JH4(-)-20mer:
5′-GGTGACTGAGGTTCCTTGAC-3′
was used to determine the remaining sequence.

Additionally, for the CC83 heavy chain, JH2(-)-16mer:
5′-CTGAGGAGACTGTGAG-3′
was used to determine the remaining sequence.

Additionally, for the CC92 heavy chain and the B72.3 heavy chain, B72.3/CC92 HC-20mer:
5′-CCTTGAACTTCTCATTGTAC-3′
was used to determine the remaining sequence.

The following procedures were carried out as outlined by Jan Gelliebter in BRL *FOCUS* 9, 1 (1987).

The oligonucleotide primers were end-labelled as follows: 100 ng of oligonucleotide were combined in 50mM Tris HCl (pH 8), 10mM MgCl₂, 5mM dithiothreitol, and 1mM spermidine, 100 µCi (γ-³²P) ATP (Amersham, 5000 Ci/mMole) and 7 units of T4 polynucleotide kinase in a volume of 13 µl. The reaction was allowed to proceed at 37°C for 30 minutes, then heated for 5 minutes at 65°C to inactivate the kinase, and then 7 µl of water was added to make the concentration 5 ng/µl. The labelled primers were stored at -20°C until needed.

Separate samples, each containing about 13 micrograms of poly(A)⁺ mRNA of CC49, CC83, CC92, or CC46, respectively, were resuspended in 10 µl of annealing buffer [10mM Tris HCl (pH 8.3), and 250mM KCl].

A 5 ng sample of end-labelled oligonucleotide primer was added to each mRNA sample, heated to 80°C for 3 minutes, and annealed for 45 minutes at 61°C, for the K_{L}(-) and 65°C for the CH1(-) oligonucleotides. AMV reverse transcriptase (Boehringer Mannheim) was used at a level of 6 units for each mRNA sequencing reaction. The remainder of the sequencing was carried out as set forth in BRL *FOCUS* 9, 1 (1987).

Initial sequence data showed that the heavy and light chains were rearranged as follows: CC49 kappa light chain used a J5; CC49 γ1 heavy chain used a J_{H}4. The CC83 light chain used a J4; the CC83 gamma 1 used a J_{H}2. The CC46 kappa light chain used a J2; the CC46 heavy chain used a J_{H}3. The CC92 light chain used a J5; the CC92 gamma 1 used a J_{H}2.

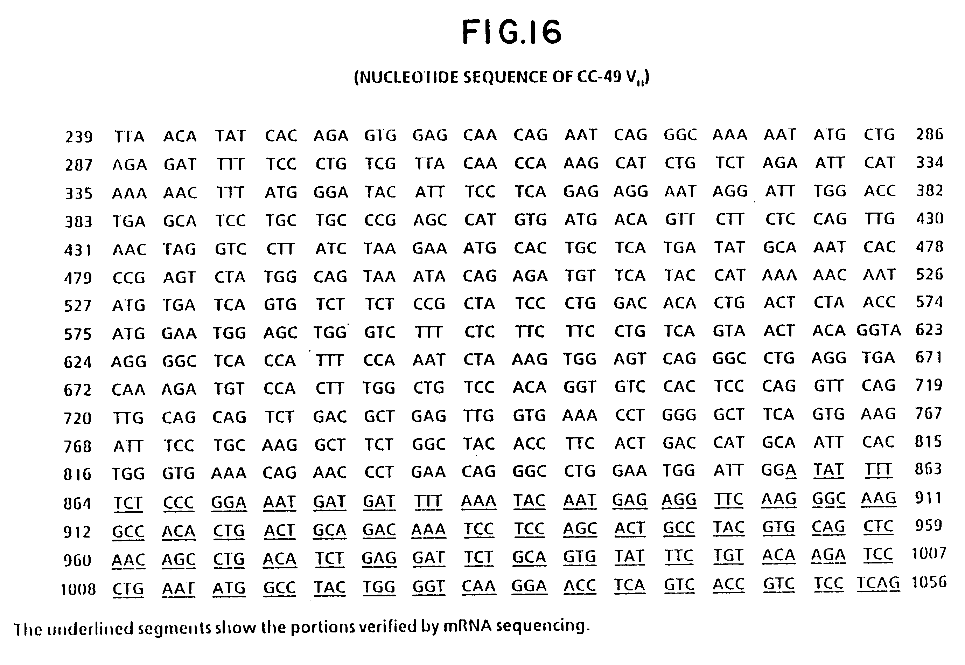

Figure 16 shows the nucleotide sequence of CC49 V_{H}, with the underlined segments showing the sequences derived using oligonucleotide primers on mRNA.

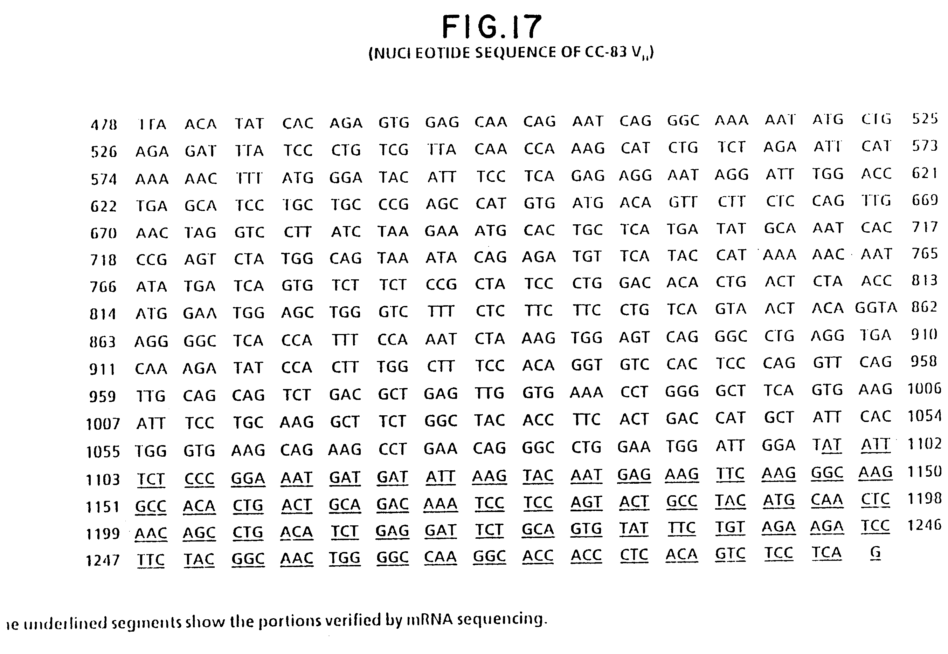

Figure 17 shows the nucleotide sequence of CC83 V_{H}, with the underlined segments show the sequences derived using oligonucleotide primers on mRNA.

The entire nucleotide sequences of CC46 V_{H} and CC92 V_{H}, shown in Figure 2, were derived using oligonucleotide primers on mRNA.

Figure 4a shows the nucleotide sequence of CC49 V_{L}, with the underlined segments show the sequences derived using oligonucleotide primers on mRNA.

Figure 5a shows the nucleotide sequence of CC83 V_{L,}, with the underlined segments show the sequences derived using oligonucleotide primers on mRNA.

The entire nucleotide sequence of CC92 V_{L}, shown in Figure 6, was derived using oligonucleotide primers on mRNA.

### Protein Sequence

Purified murine CC49 and CC83 immunoglobulin molecules were sent to Dr. George Tarr at the University of Michigan Protein Sequencing facility for NH₂-terminal amino acid sequence analysis. Dr. Tarr used the Edman degradation method, as modified by Tarr, G.E., in "Manual Edman Sequencing System", Microcharacterization of Polypeptides: A Practical Manual [John E. Shively, ed., Humana Press, Inc., Clifton, N.J., pp 155-194 (1986)]. Briefly, Dr. Tarr reduced and alkylated the immunoglobulin molecules. The light and heavy chains of the immunoglobulin molecules were separated by reverse phase HPLC.

Figure 4b shows the amino acid sequence for CC49 V_{L}, with the results of the amino acid sequence determination for the first 24 amino acids of the mature CC49 V_{L} being underlined. Figure 5b shows the amino acid sequence for CC83 V_{L}, with the results of the amino acid sequence determination for the first 51 amino acids of the mature CC83 V_{L} being underlined. ASN-20 could not be determined in the CC83 light chain, because of the presence of N-linked carbohydrate residues at this position, which is shown in the PNGase F experiment below. The sequence Asn-Ile-Thr corresponds to the consensus sequence Asn-X-Thr/Ser for carbohydrate attachment to Asn.

Since the heavy chains of immunoglobulins CC49 and CC83 are blocked at the N-terminus and unavailable for amino acid sequence determination, the native glycopeptide was treated with cyanogen bromide (CNBr) to cleave at the methionine residues. The cleavage resulted in fragments, which were purified by reverse phase HPLC. N-terminal amino acid sequencing was performed on the CNBr fragments.

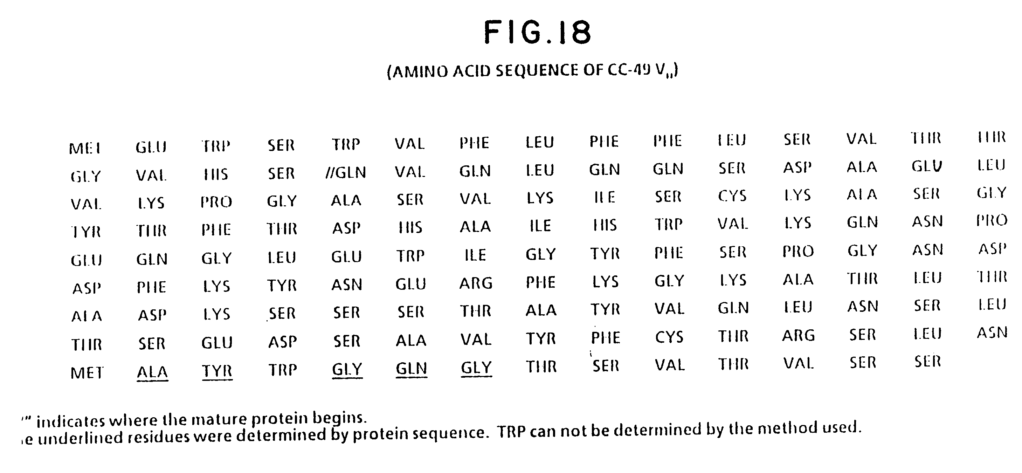

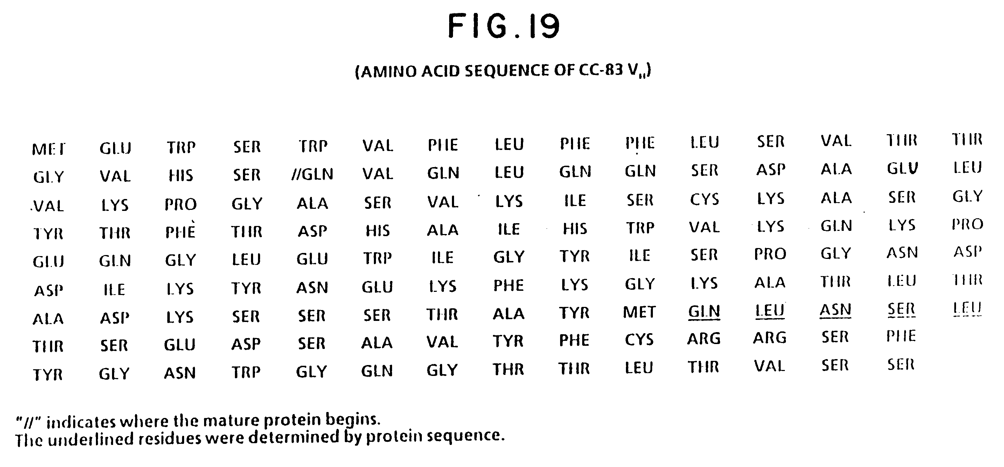

The results of the amino acid determination of one of the CC49 V_{H} CNBr peptide fragments are indicated as underlined residues in Figure 18. The results of the amino acid determination of one of the CC83 V_{H} CNBr peptide fragments are indicated as underlined residues in Figure 19. As with CC49, all other peptide sequences correspond to CNBr fragments derived from the constant region of mouse γ1.

### Determination of N-Linked Carbohydrate on CC83 L Chain

This experiment was done to verify that there is an N-linked carbohydrate attached to the CC83 light chain, presumably at ASN-20 (see Figure 5b). The enzyme glycopeptidase F (PNGase F), which is isolated from the culture filtrate of *Flavobacterium meningosepticum* [Tarentino, A. L et al., *Biochemistry* 24, 4665-4671 (1985)], will cleave high mannose and/or biantennary complex sugars N-linked to ASN to generate a free carbohydrate structure and an ASP residue from the ASN to which it was attached. The difference in molecular weight between the glycosylated and unglycosylated form of the same peptide can be determined by SDS-PAGE.

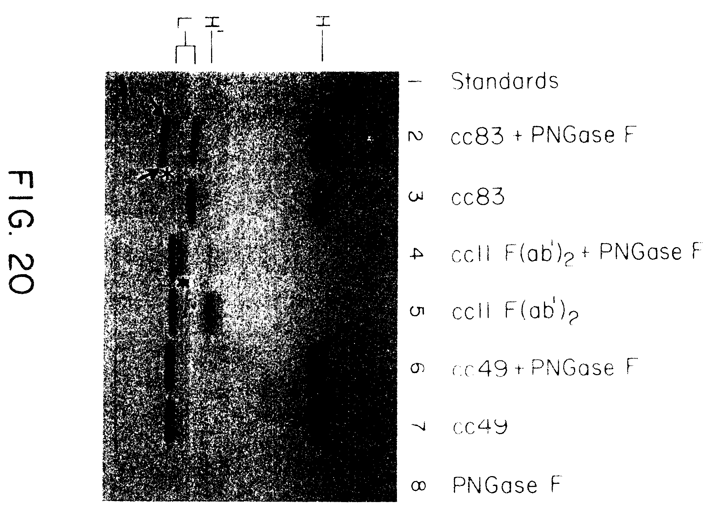

Twelve microgram reactions with and without PNGase F (Boehringer Mannheim, Indianapolis, IN, USA) for the purified murine antibodies CC49, CC83 and CC11 F(ab′)₂ (a positive control) were carried out in a final aqueous reaction volume of 40 microliters. Four microliters of 10 x buffer (1M potassium phosphate, 0.1M disodium EDTA pH 7.4) were added to each reaction mix. To those tubes designated "with PNGase F", 7.5 microliters of PNGase F were also added and all tubes were incubated at 37°C for 1 h. To the reaction tubes was added 40 microliters of Laemmli 2X sample dilution buffer containing β-mercaptoethanol. A 10 percent SDS polyacrylamide gel was electrophoresed, the gel stained with Coomassie Brilliant Blue R-250 and destained. Figure 20 shows the results. As shown in lane 2, a new band (*) appears in the PNGase F treated CC83 sample but not in the untreated CC83 sample (lane 3). The new band is approximately 2,000-3,000 molecular weight smaller than the native light chain band, which represents the removal of an N-linked carbohydrate moiety. The only consensus glycosylation site for the CC83 light chain is at ASN 20, so by inference it is assumed that this is the actual site of glycosylation and why it did not show up on the N-terminal sequence analysis of the CC83 light chains as ASN. The CC49 light chain does not change mobility when treated with PNGase F (lane 6), but a new band is observed for the heavy chain fragment of CC11 F(ab′)₂ (lane 4*) which serves as a positive control. mRNA sequence data of CC11 heavy chain indicates a consensus glycosylation site in the V domain (data not shown). The standards (lane 1) are bovine serum albumin (BSA), MW 68,000 and soybean trypsin inhibitor (STI), MW 21,500.

### DNA Sequence

Plasmid DNA was sequenced directly using the Sequenase DNA sequencing kit, obtained from United States Biochemical (USB), Cleveland, OH, USA. USB's protocol was followed to sequence double stranded DNA. The DNA of each variable region was sequenced using the J_{H} or J_{L} oligo determined from the mRNA sequence information to be specific for each productively rearranged heavy chain or light chain gene, respectively.

After the initial sequences were determined, the sequence was extended further by using additional primers. The additional primers were synthesized using information gathered from the sequences previously generated.

Using the above technique, the DNA sequences of the entire heavy chain variable region exons and light chain variable region exons of CC49 and CC83 were obtained. The DNA sequence was compiled and analyzed using Hitachi's DNA sequence analysis software program DNASIS™.

The following oligonucleotide primers were made for DNA sequencing:
(1) For both light chains, C_{K} intron(-):
   5′-GAAAACCTGTGTCTTACAC 3′.
(2) For the CC49 light chain, CC49 FRI(+):
   5′-GTACCTGTGGGGACATTG 3′,
   and JK5(-)-23mer
   5′CGTTTCAGCTCCAGCTTGGTCCC-3′.
(3) For the CC83 light chain, CC83 CDR2(-):
   5′-CAGGGACTCCAGTGTGC 3′,
   CC83 L intron (-):
   5′GACTTCAAGATACAAATGTTAG-3′,
   and JK4(-)-20mer:
   5′-CCAACTTTGTCCCCGAGCCGAACG.

The complete nucleotide sequences for CC49 V_{L} and CC83 V_{L} are shown in Figures 4a and 5a, respectively.

For the CC49 heavy chain, J_{H}4 (-)-20mer:
5′GGTGACTGAGGTTCCTTGAC-3′ a
nd J_{H}4 Intron (-):
5′-GCAATGCTCAGAAAACTCC.

For the CC83 heavy chain, JH2(-)-16mer:
5′CTGAGGAGACTGTGAG-3′
and J_{H}2 Intron(-):
5′-GCAGTAAAATCTATCTAAGCTG.

Thereafter, the sequencing of each heavy chain was extended with the following sequences: CC49/83 HC/5′(+)
5′-GCACTGCTCATGATATGCAAATC-3′;
CC49/83 HC/5′(-)
5′-GATTTGCATATCATGAGCAGTGC-3′;
and CC49/83 H chain FRI(-)
5′-CTCAGCGTCAGACTGCTG-3′.

The complete nucleotide sequences for CC49 V_{H} and CC83 V_{H} are shown in Figure 2.

Comparisons were made between the characterized mRNA sequence and the characterized DNA sequence, and between the characterized amino acid sequence with the amino acid sequence predicted from the DNA sequence. Based on these comparisons, the plasmid clones were identified to contain the correct DNA sequence to code for the CC49 and CC83 heavy and light chain variable regions.

The predicted amino acid sequences from the nucleotide sequences of the heavy chain variable regions of CC49 and CC83, as shown in Figure 2, show extensive sequence similarity throughout the framework regions and hypervariable regions 1 and 2. Hypervariable region 3 is quite different between the two due to the recombination of the V_{H} region with different D and J_{H} sequences, namely that the CC49 γ1 heavy chain used a J_{H}4, and the CC83 gamma 1 used a J_{H}2.

The extensive DNA sequence homology 5′ to the coding regions in the CC49 and CC83 heavy chain variable region genes shows the two heavy chain variable region genes were derived from the same germline exons.

### Isolation of V_{H}αTAG, Germline Precursor Gene to the Heavy Chain of CC46, CC49, CC83, and CC92

The procedures used to isolate the germline precursor gene to the heavy chain variable regions of CC46, CC49, CC83, and CC92 were essentially those used to isolate the CC49 heavy chain variable region except that the DNA used to generate the LAMBDA-ZAP™ library came from an irrelevant hybridoma cell line (i.e., a cell line which produces antibodies that do not appreciably bind to TAG72). A genomic library containing approximately 900,000 plaques was screened from which one positive clone was isolated. The positive clone was named pV_{H}αTAG. pV_{H}αTAG was about 5.2 kb, and the size of the DNA insert was determined by restriction enzyme mapping to be about 2.2 kb.

### DNA sequence of V_{H}αTAG

The following oligonucleotide primers were used for determining the DNA sequence of V_{H}αTAG:
B72.3/CC92 HC-20mer: 5′-CCTTGAACTTCTCATTGTAC-3′;
CC49/CC83 HC 5′(+): 5′-GCACTGCTCATGATATGCAAATC-3′;
CC49/CC83 HC 5′(-): 5′-GATTTGCATATCATGAGCAGTGC-3′;
V_{H}αTAG IVS (+): 5′-CTAAAGTGGAGTCAGGGCCTG-3′;
V_{H}αTAG IVS (-): 5′-CAGGCCCTGACTCCACTTTAG-3′;
V_{H}αTAG CDR2 (+): 5′-GAATGGATTGGATATATTTCTC-3′.

The complete nucleotide sequence of V_{H}αTAG is shown in Figure 2.

### Isolation of Human Heavy Constant Genes

Plasmid constructs containing the various heavy chain human constant regions (pγ1, pγ2, pγ3, and pγ4) were provided by Dr. Ilan R. Kirsch of the National Cancer Institute, Bethesda, Maryland.

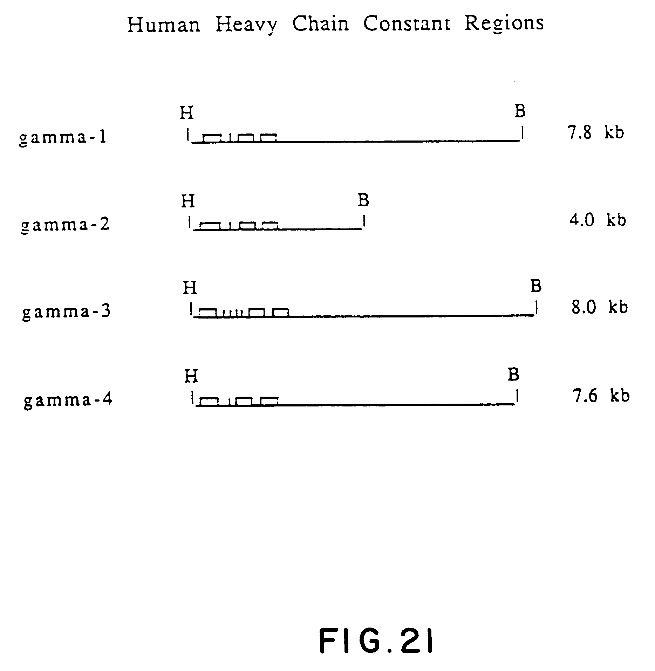

Restriction enzyme mapping was performed on these genes to confirm their identity. Restriction maps for the human constant regions are enclosed in Figure 21.

### Chimeric Light Chain

### Murine CC49 V Region

The *Hind* III site of the CC49 light chain genomic DNA located in the murine intron region between J5 and Cₖ (see Max, Edward E. et al., *J. Biol. Chem.* 256, 5116 (1981) was lost in the cloning procedure where half-filled in *Hind* III sites were ligated to half-filled in *Spe* I sites in the LAMBDA-ZAP vector. The plasmid pRL101 (Figure 9) carried this modification.

The intron *Hind* III site was regenerated as outlined in the steps below in order to enable a *Hind* III-*Bam* HI human germline kappa light chain DNA fragment (see Hieter, P. et al., *J. Biol. Chem*. 257, 1516 (1982) to be ligated to the murine variable region directly. All steps were performed using standard molecular biology techniques familiar to artisans and can be found in a manual such as *Manatis*.

A 1.69 kb *Bam* HI-*Pst* I fragment is isolated from pRL101, described supra. A 2.96 kb *Bam* HI-*Pst* I fragment is isolated from pBluescript SK(-) (purchased from Stratagene), described supra. The two fragments are then ligated and pRL103, below, is isolated. 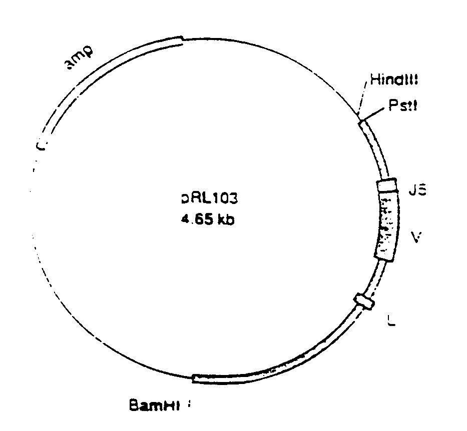
Plasmid pGD1, (described supra), was digested with *Pst* I and *Hind* III restriction enzymes to yield the necessary 1.03 kb intron-containing fragment, and pRL103 was also digested with *Pst* I and *Hind* III restriction enzymes to remove the small fragment of DNA in the polylinker.

The resulting fragments were ligated with T4 DNA ligase to produce a 5.68 kb plasmid, called pRL104. A partial restriction map of pGD1 and pRL104 are shown below.

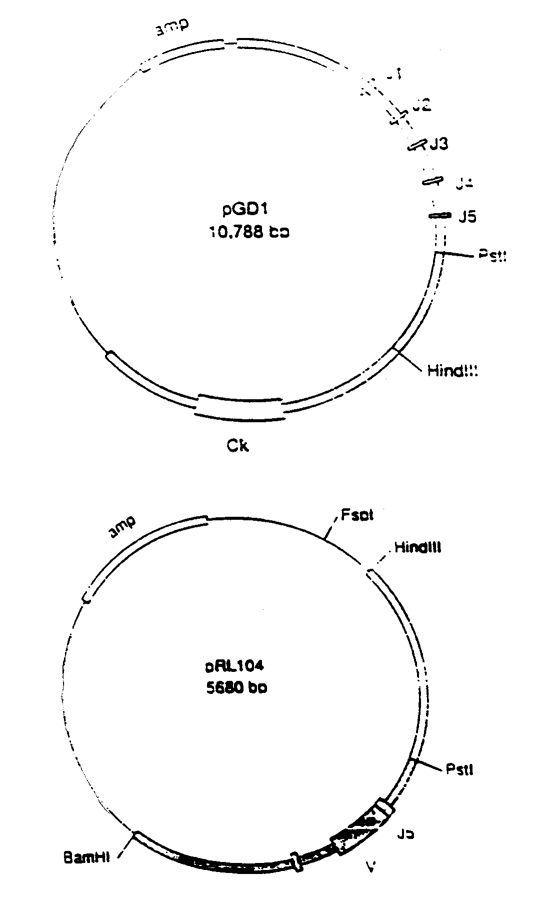

amp
J1
J2
J3
J4
pGD1
10,788 bp
J5
PstI
HindIII
Ck


amp
FspI
HindIII
pRL104
5680 bp
PstI
J5
BamHI
V

### Human C_{K} Region

Plasmid phum C_{K} was obtained from Dr. John Roder, Mt Sinai Research Institute, Toronto, Ontario, Canada. The plasmid is derived from pBR322, with a 12 kb *Bam* HI fragment containing the human C_{K} exon inserted therein. pBR322 is described on page 171 of the 1987 Stratagene catalog. The 12 kb *Bam* HI fragment restriction map is shown below [from Heiter. P. et al., *J. Biol. Chem* 257, 1516 (1982)]. 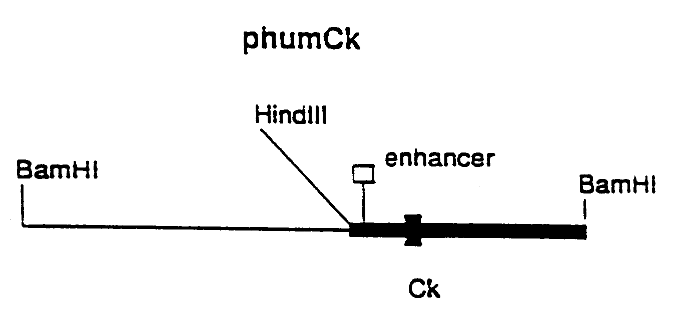
phumCk
HindIII
enhancer
BamHI
BamHI
Ck
The plasmid phum Cₖ was digested with *Hind* III and *Bam* HI restriction enzymes to yield a 5.0 kb fragment, containing the human Cₖ exon. pRL104 was digested with *Fsp* I and *Hind* III restriction enzymes to yield a 4.2 kb fragment, containing the mouse light chain variable exons of CC49.

The two resulting fragments were joined with T4 DNA ligase to produce a 9.2 kb fragment among the mixture of resulting fragments. This mixture was digested with *Bam* HI to yield an 7.7 kb *Bam* HI CC49 L chain chimeric construct with *Bam* HI sticky ends, which contains both the mouse variable region exons and the human constant region (kappa) exon. These constructions utilize the human enhancer sequences and the murine promoter sequences.

The chimeric *Bam* HI fragment containing both the murine light chain variable region exons (L and VJ) and the human constant region kappa (k) exon ws ligated into the BamHI site of with the plasmid pSV2neo (5.6 kb), a pBR322-derived plasmid containing the selectable marker gene *neo* (obtained from ATCC). The presence of the active *neo* gene renders a cell resistant to growth inhibition by Geneticin, a neomycin-like drug also called G418.

The chimeric *Bam* HI fragment was inserted into pSV2neo in both orientations as shown below. Both transcriptional orientations of the chimeric light chain gene, relative to the neo gene, were constructed. Plasmid pSV2neo was linearized at the Bam HI site, dephosphorylated (according to procedures set forth in *Maniatis*) using calf intestinal alkaline phosphatase (to prevent self-ligation) and ligated with chimeric CC49 L chain *Bam* HI fragments from above. 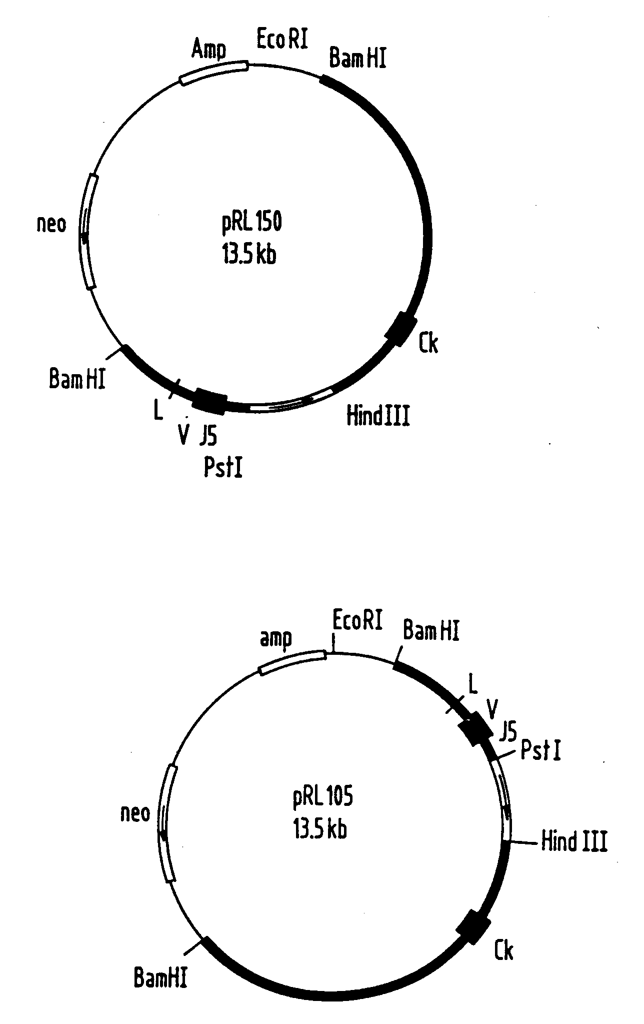
Amp
EcoRI
BamHI
pRL150
13.5 kb
neo
Ck
BamHI
L
V
J5
PstI
HindIII

amp
EcoRI
BamHI
L
V
J5
PstI
pRL105
13.5 kb
neo
HindIII
Ck
BamHI
The transcriptional orientations of the *neo* gene and the CC49 chimeric light chain are indicated by arrows in pRL150 and pRL105. The portions derived from pSV2neo are indicated. These plasmids were purified on a large scale from preparative scale (1.0L) fermentation of *E.coli* clones replicating each of the plasmids. The purified plasmids were used to introduce the chimeric CC49 light chain into SP2/0 plasmacytoma cells as discussed below.

### Murine CC83 V_{L} Region and Human C_{K} Region

The *Hind* III site in pRL200 which was lost in the cloning process of the CC83 light chain was regenerated for the same reason as for the CC49 light chain chimeric construction. The regeneration was accomplished as follows. The plasmid pRL200 was linearized at a unique *Nhe* I site, and both of its sticky ends were converted to blunt ends by filling in with dNTPs and DNA polymerase I. A *Bam* HI phosphorylated linker (purchased from New England Biolabs) was ligated to the filled-in site. The new plasmid is called pRL201 and is shown below. 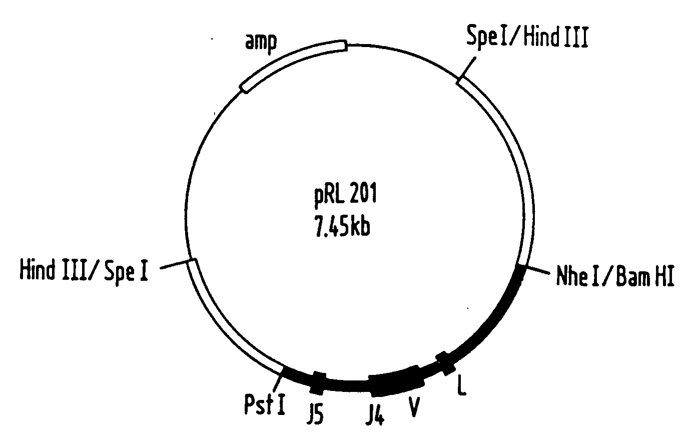
amp
Spe I/ Hind III
pRL 201
7.45kb
Hind III/ Spe I
Nhe I/ Bam HI
L
V
J4
J5
Pst I
The 2.5 kb *Bam* HI-*Pst* I fragment from pRL201 containing the CC83 light chain variable region genomic DNA was conveniently ligated to the 4kb *Bam* HI-*Pst* I vector fragment from pRL104 which was described earlier in the CC49 light chain constructions and which already had the Hind III-bearing intron fragment. The new plasmid is called pRL202 and is shown below. 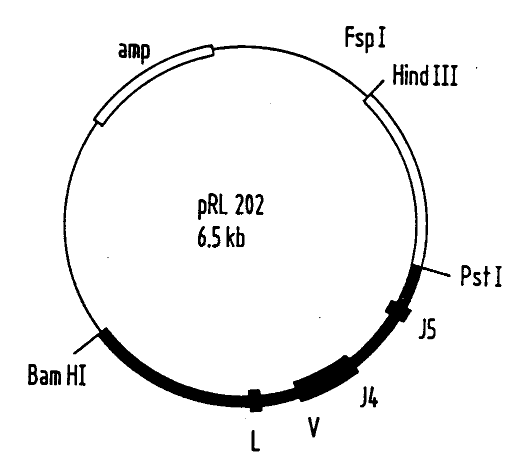
amp
Fsp I
Hind III
pRL 202
6.5 kb
Pst I
J5
Bam HI
J4
L
V
The approximately 5.05 kb *Fsp* I-*Hind* III fragment from pRL202 was isolated and ligated with the human Cₖ-containing 5.0 kb *Hind* III-*Bam* HI fragment already described for the CC49 light chain chimeric construction. The generation of the CC83 light chain vector was accomplished from this point in an identical fashion as carried out for the CC49 light chain. The resulting 8.5 kb *Bam* HI CC83 light chain chimeric construct was also ligated to pSV2neo-*Bam* HI (phosphatased) and plasmids with both possible orientations of the insert were obtained as diagramed below. 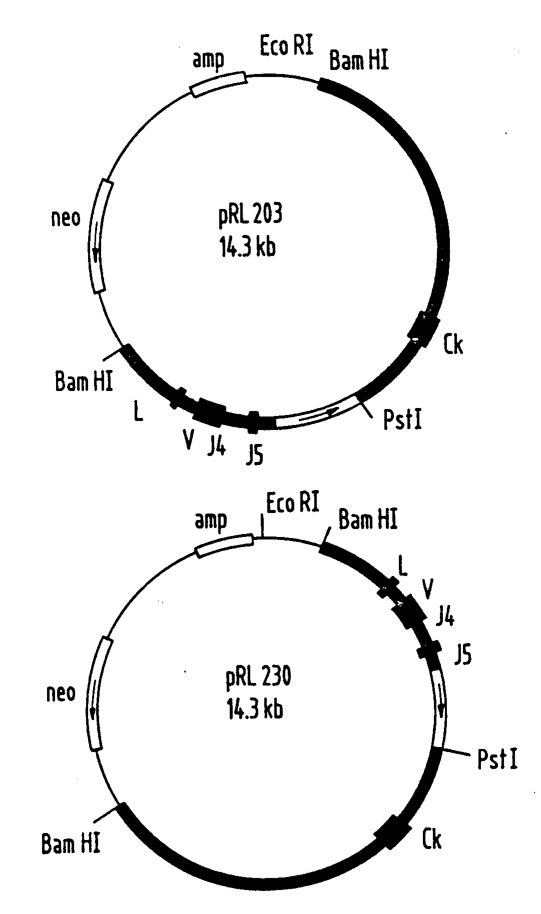
amp
Eco RI
Bam HI
neo
pRL 203
14.3 kb
Ck
Bam HI
L
Pst I
V
J4
J5
amp
Eco RI
Bam HI
L
V
J4
J5
neo
pRL 230
14.3 kb
Pst I
Bam HI
Ck
The transcriptional orientations of the *neo* gene and the CC83 chimeric light chain are indicated by arrows in pRL203 and pRL230. These plasmids were purified on a large scale from preparative scale (1.0L) fermentation in a commercial incubator of *E. coli* clones replicating each of the plasmids. The purified plasmids were used to introduce the chimeric CC83 light chain into Sp2/0 plasmacytoma cells, as discussed later.

All four of the chimeric light chain plasmid constructs (pRL105, pRL150, pRL203 and pRL230) can be linearized by digesting with the restriction enzyme Aat II. The *Aat* II site in the plasmids is in a region that is not essential for the expression of the chimeric light chain gene or the selectable marker gene, *neo*.

### Chimeric Heavy Chains

### Human Gamma Constant Gene Exons

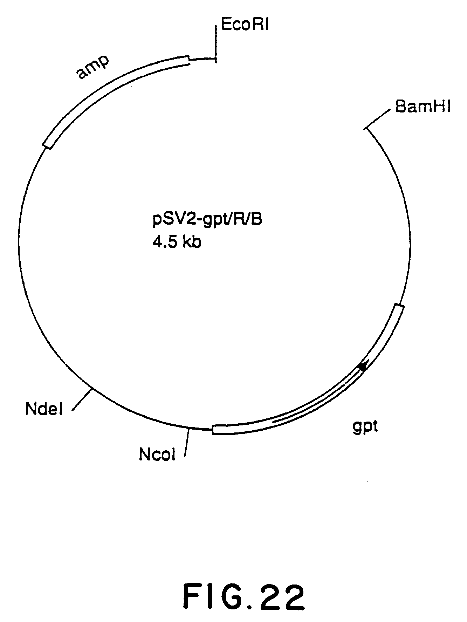

The plasmid vector used to carry the chimeric heavy chain constructs is designated pSV2gpt, set forth in Mulligan and Berg, "Selection of animal cells that express the *E. coli* gene coding for xanthine-guanine phosphoribosyltransferase", *Proc. Natl. Acad. Sci (USA)* 78(4), 2072-2076 (1982). pSV2gpt is a pBR322 derived plasmid containing the selectable marker gene, guanine phosphoribosyl transferase (gpt), which can be used for selective growth in media containing mycophenolic acid. To prepare pSVgpt as a recipient for the human Cγ1, Cγ2, Cγ3, Cγ4 exons, it was digested with *Eco* RI and *Bam* HI. The digested DNA was fractionated on a 4 percent polyacrylamide gel and the 4.5 kb vector fragment was recovered from the gel by electroelution as described in *Maniatis*. This linearized plasmid was designated pSV2gpt/R/B, a plasmid map is shown in Figure 22. It is able to accept *Eco* RI-*Bam* HI ended fragments.

The 5′ *Hind* III sites, present on the human IgG₁ constant region fragments, were converted to *Eco* RI sites for directed cloning into the *Eco* RI site of pSV2gpt. For γ1, γ2, γ3, and γ4, the *Eco* RI site in vector, pBR322 was employed.

### Cγ1

The fragment containing the human Cγ1 exons was obtained by digesting and linearizing pγ1 with *Hind* III followed by filling in the *Hind* III sticky ends using all four dNTP's and the Klenow fragment of DNA Polymerase to make the *Hind* III ends blunt. An *Eco* RI linker was ligated to the blunt ends to replace the *Hind* III site with an *Eco* RI site. This construct was then digested with *Eco* RI and *Bam* HI to release a 7.8 kb fragment containing the Cγ1 exons. This fragment was called Cγ1-7.8 kb.

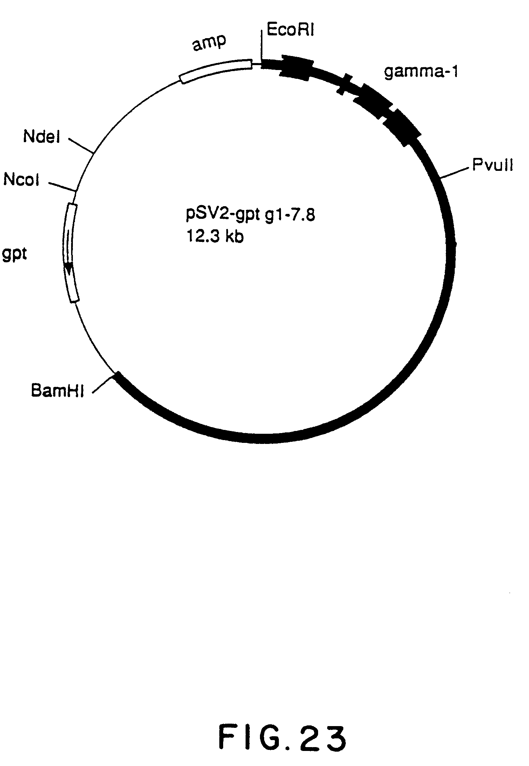

The fragment were each ligated into the *Eco* RI-*Bam* HI sites of pSV2-gpt/R/B. This vector (pSV2-gpt-γ1-7.2) design allows us to insert any murine heavy chain variable region gene (with *Eco* RI ends) into the *Eco* RI site of the human IgG heavy chain vectors. More specifically, 125 ng of the human Cγ1-7.8 kb fragment was ligated to 100 ng of the linearized pSV2gpt/R/B vector in a volume of 10 µl using 400 units of T4 DNA ligase (obtained from New England Biolabs). Frozen competent *E. coli* DH1 cells from Invitrogen (San Diego, CA) were transformed with a ligation reaction according to the Invitrogen's protocol. The resulting plasmid was designated pSV2gptγ1-7.8. A plasmid map of pSV2gptγ1-7.8 is shown in Figure 23.

In addition, another shorter fragment containing the Cγ1 exons was generated. Concerns about the total size of the chimeric heavy chain vector, with a 7.8kb Cγ1 fragment, a 4.5kb pSV2-gpt/R/B vector, and a CC49 variable region of 1.9kb (total=14.2kb) prompted the need to reduce the large size of the 7.8kb Cγ1 *Eco* RI-*Bam* HI fragment. The coding region of 7.8kb Cγ1 occupies only the first 1/3 of the 5′ end of the fragment.

Size reduction was accomplished by converting a downstream *Pvu* II site to a *Bam* HI site by blunt-end addition of a *Bam* HI linker. The *Hind* III site of pγ-1 was converted to an *Eco* RI site by digestion of pγ-1 with *Hind* III, filling in the 3′ end to create a blunt end, and addition of *Eco* RI linkers as above. The *Pvu* II site 2.3 kb downstream was converted to a *Bam* HI site by subsequent digestion with *Pvu* II and ligation of *Bam* HI linkers directly to the blunt *Pvu* II ends. This construct was then digested with *Eco* RI and *Bam* HI to release a 2.3 kb fragment containing the Cγ1 exons. The shortened EcoRI-BamHI fragment (2.3 kb) still contains the γ1 exons and the 3′ polyadenylation sequence. This reduces the total vector size by 5.5kb, making the overall construct more manageable (total-8.7 kb).

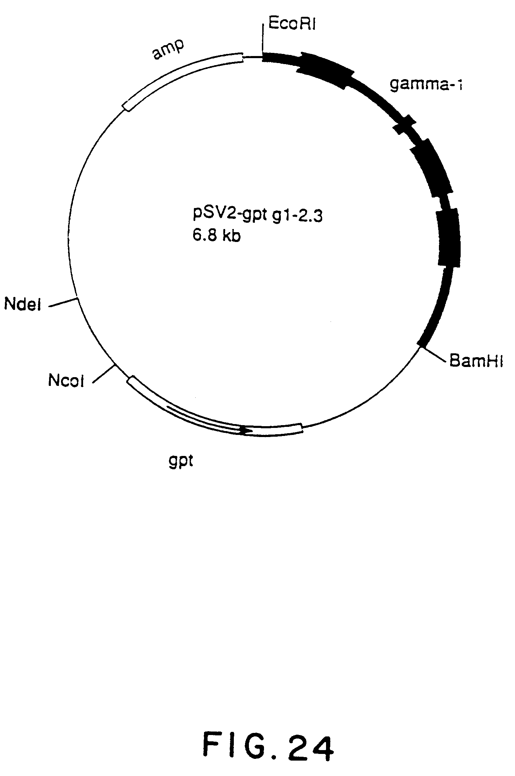

Approximately 200 ng of the human Cγ1 2.3 kb fragment was ligated to 100 ng of the linearized plasmid pSV2gpt/R/B vector in a volume of 10 µl using 400 units of T4 DNA ligase (New England Biolabs). Frozen competent *E. coli* cells, obtained from Invitrogen, were transformed with the ligation reaction according to Invitrogen's protocol. The resulting plasmid was designated pSV2gptγ1-2.3. A plasmid map of pSV2gptγ1-2.3 is shown in Figure 24.

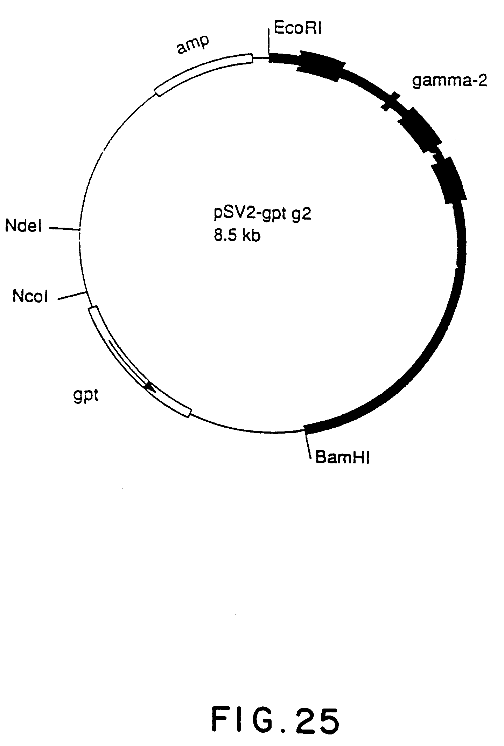

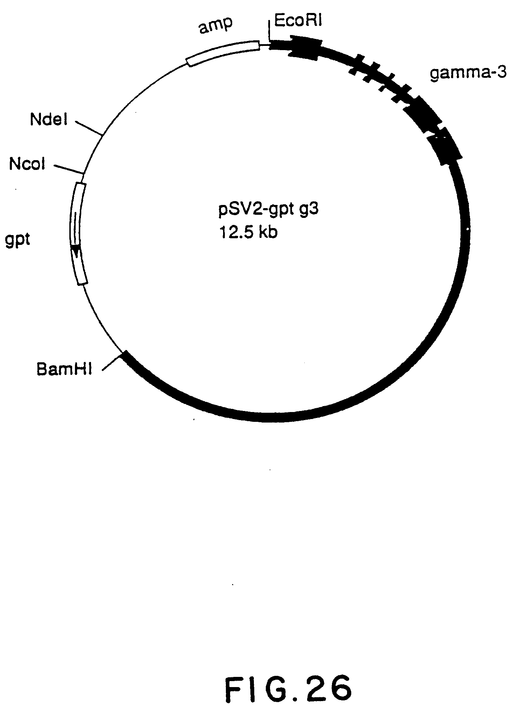

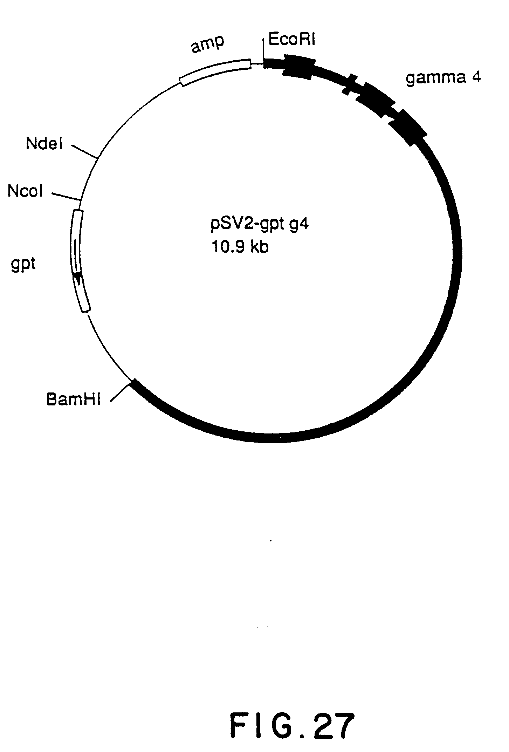

DNA fragments containing the other three human IgG constant region exons were also isolated. The Cγ2 exons were recovered from the plasmid pγ2 as a 4.0 kb *Eco* RI-*Bam* HI fragment. The Cγ3 exons were recovered from the plasmid pγ3 as an 8.0 kb *Eco* RI-*Bam* HI fragment. The Cγ4 exons were recovered from the plasmid pγ4 as a 7.6 kb *Eco* RI-*Bam* HI fragment. The fragments were separately ligated into a pSV2gpt/R/B as described for Cγ1-7.8 and Cγ1-2.3. Plasmid maps of the resultant plasmids are shown in Figure 25, pSV2gpt-γ2; Figure 26, pSV2gpt-γ3; and Figure 27, pSV2gpt-γ4.

### Heavy Chain Chimeric Constructs:

The complete heavy chain variable region human γ1 constant region chimeric constructs were generated by inserting a fragment containing the murine heavy chain variable region exons into the plasmids containing the human γ1 constant region exons described as follows.

*Eco* RI fragments containing the murine heavy chain variable region genes from CC49 and CC83 hybridoma cells were then ligated into each of the γ1-γ4-containing pSV2-gpt vectors (pSV2gpt-γ1; pSV2gpt-γ2; pSV2gpt-γ3; pSV2gpt-γ4;) as follows.

### CC49

A fragment containing the heavy chain variable region exons coding for the CC49 heavy chain variable region was prepared by digesting 14 µg of pHH49 with 50 units of *Eco* RI (obtained from BRL) at 37°C for 2 hours. The digest was fractionated on a 4 percent polyacrylamide gel and the 1.9 kb *Eco* RI fragment containing the heavy chain variable region exons of CC49 was recovered by electroelution as described by *Maniatis*. This fragment was designated f49R.

A fragment containing the 7.8 kb sequence encoding for γ1 was prepared as follows.

Approximately 50 µg of the vector pSV2gpt γ1-7.8 was digested with *Eco* RI. The resultant fragment was dephosphorylated (to prevent self ligation) using calf intestinal alkaline phosphatase as described by *Maniatis*. The fragment was purified from the 0.8 percent agarose gel by electroelution. This vector was designated pSV2gptγ1-7.8/R.

The EcoRI site is located 245 bp upstream of the transcription initiation sites, and contains the promoter and the necessary tissue-specific sequences for efficient expression. The intron regions 3′ of the variable region genes contain the murine heavy chain enhancer sequences which are absent on the human IgG heavy chain vectors. Therefore, the heavy chain chimeric vectors use both murine promoter and enhancer sequences.

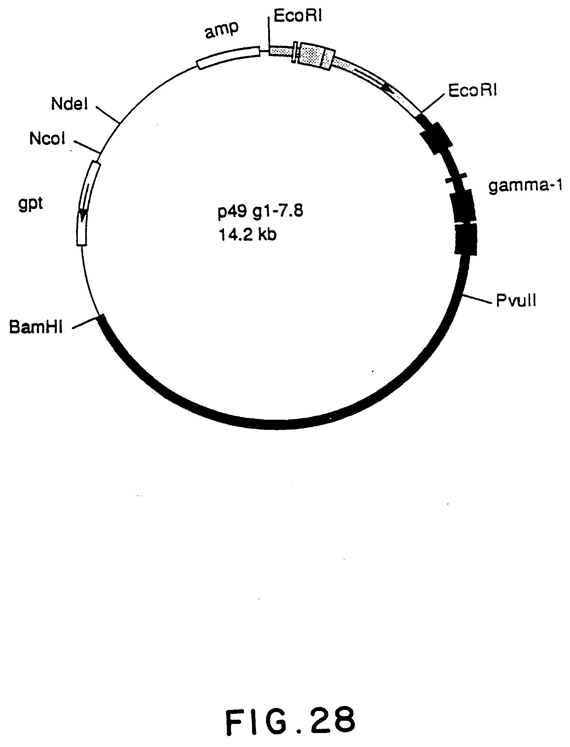

Approximately 325 ng of linearized pSV2gptγ1-7.8/R was ligated with 188 ng of f49R in a volume of 10 µL with 1 unit of T4 DNA ligase (BRL). Frozen competent *E. coli* AG-1 cells from Stratagene were transformed with the ligation reaction according to their protocol. The resulting plasmid was designated p49γ1-7.8. Figure 28 illustrates a plasmid map for p49γ1-7.8.

Approximately 50 µg of the vector pSV2gptγ1-2.3 was digested as for SV2gptγ1-7.8 with *Eco* RI. The resultant fragment was dephosphorylated using calf intestinal alkaline phosphatase as described by *Maniatis*. The fragment was purified from an 0.8 percent agarose gel by electroelution. This linearized plasmid was designated pSV2gptγ1-2.3/R.

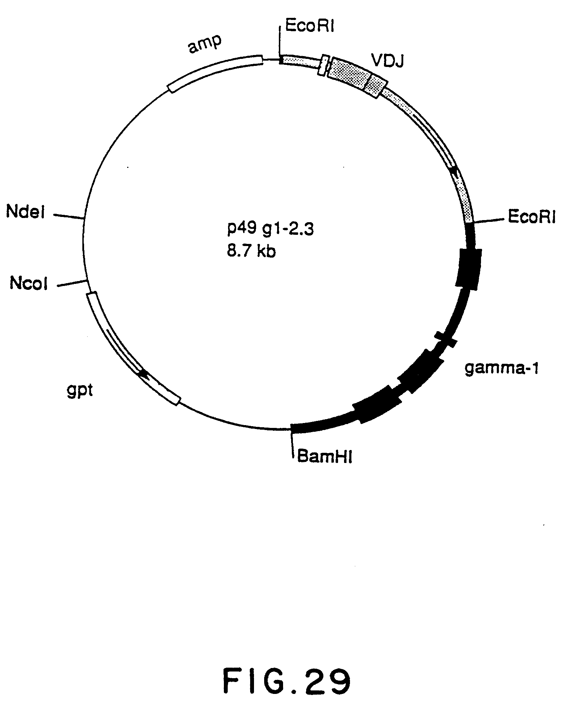

Approximately 300 ng of the linearized plasmid pSV2gpt γ1-2.3/R was ligated with 188 ng of f49R in a volume of 10 µl with 1 unit of T4 DNA ligase (BRL). Frozen competent *E. coli* AG-1 cells from Stratagene (La Jolla, CA) were transformed with the ligation reaction according to their protocol. The resulting plasmid was designated p49γ1-2.3. Figure 29 illustrates a plasmid map for p49γ1-2.3.

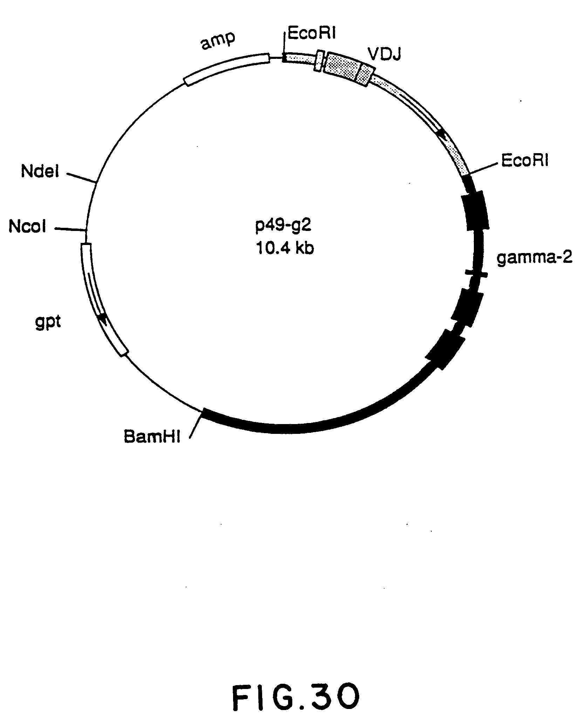

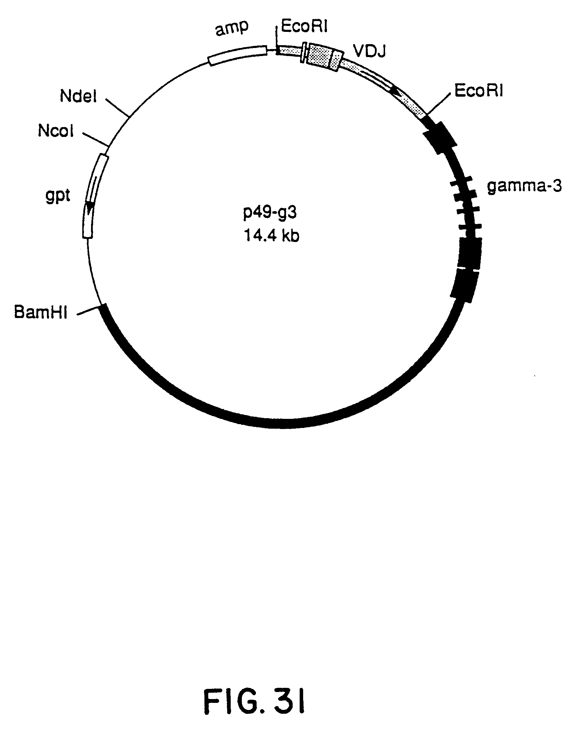

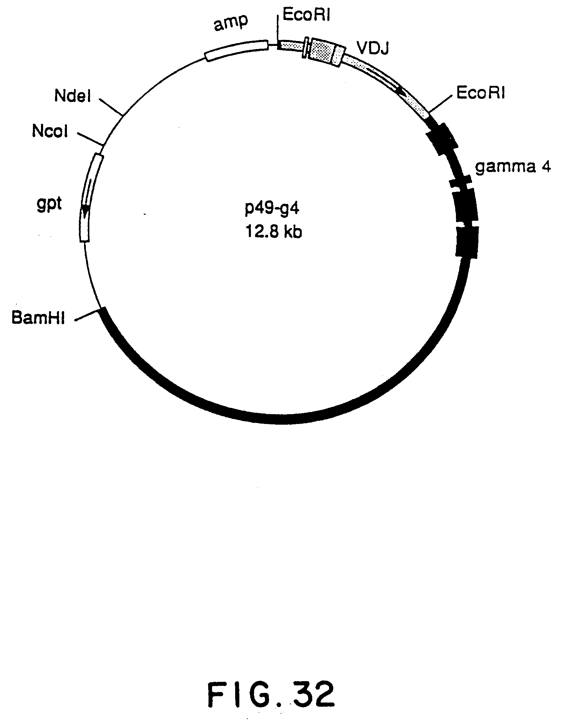

Plasmids pSV2gpt-γ2, pSV2gpt-γ3 and pSV2gpt-γ4 were separately digested with *Eco* RI to produce the linear plasmid vectors pSV2gpt-γ2/R, pSV2gpt-γ3/R and pSV2gpt-γ4/R respectively. Each of these 3 linear plasmid vectors were separately ligated with f49R. Plasmid maps of the resulting plasmids are shown in Figure 30, p49-γ2; Figure 31, p49-γ3; and Figure 32, p49-γ4.

### CC83

Chimeric constructs containing the heavy chain variable region of CC83 were generated in a similar manner as the chimeric constructs of CC49. A fragment containing the heavy chain variable region exons coding for the CC83 heavy chain region was prepared by digesting 19 µg of pHS83 with 50 units of *Eco* RI (obtained from BRL) at 37°C for 2 hours. The digest was fractionated on a 4 percent polyacrylamide gel and the 2.9 kb *Eco* RI fragment containing the heavy chain variable region exons of CC83 were recovered by electroelution as described in *Maniatis*. This fragment was designated f83R.

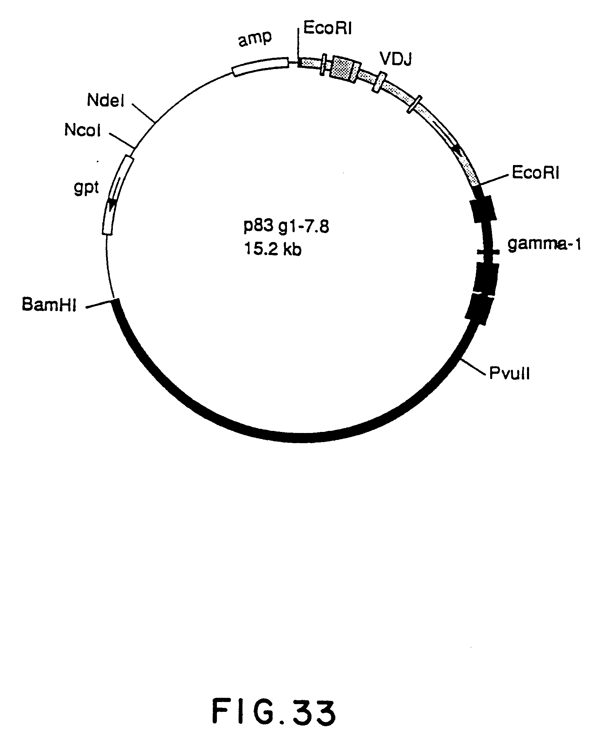

Approximately 300 ng of the linearized plasmid pSV2gptγ1-7.8/R, obtained as above, was ligated with 270 ng of f83R in a volume of 10 µl with 1 unit of T4 DNA ligase (obtained from BRL). Frozen competent *E. coli* AG-1 cells, obtained from Stratagene, were transformed with the ligation reaction according to Stratagene's protocol. The resulting plasmid was designated p83γ1-7.8. Figure 33 illustrates the plasmid map of p83γ1-7.8.

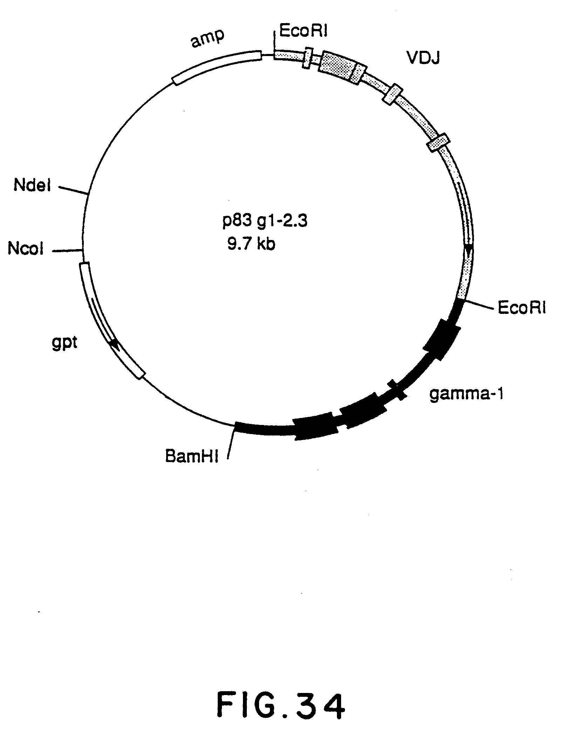

Approximately 90 ng of linearized plasmid pSV2gpt γ1-2.3/R, obtained as above, was ligated with 270 ng of f83R in a volume of 10 µl with 1 unit of T4 DNA ligase (BRL). Frozen competent *E. coli* AG-1 cells from Stratagene were transformed with the ligation reaction according to their protocol. The resulting plasmid was designated p83γ1-2.3. Figure 34 illustrates the plasmid map of p83γ1-2.3.

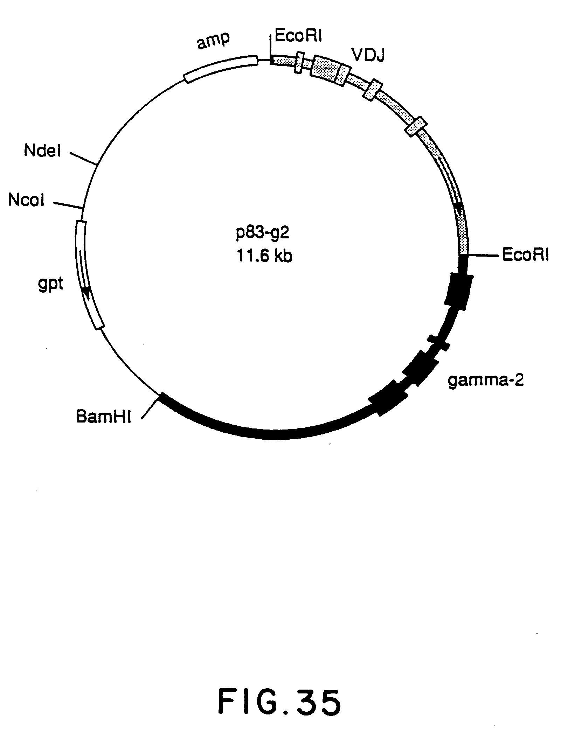

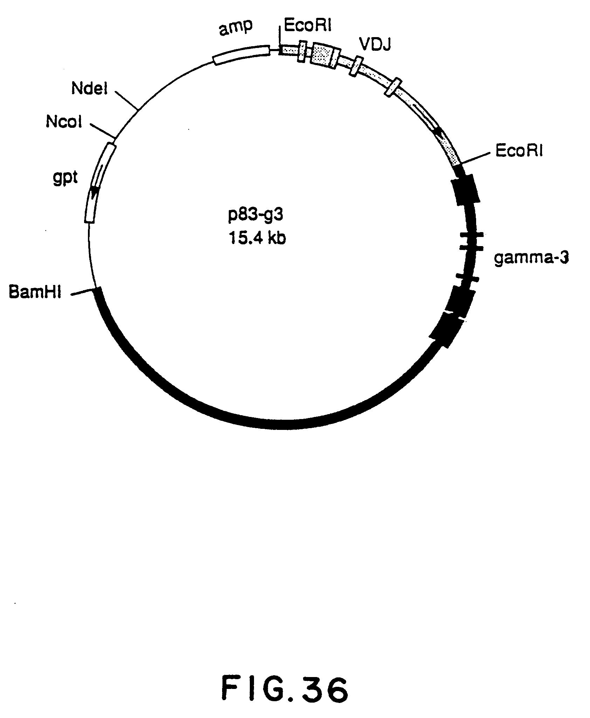

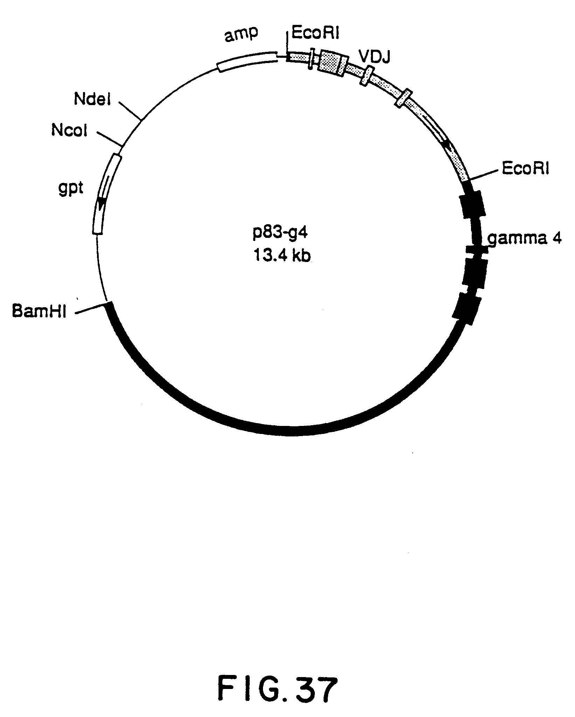

Plasmids pSV2gpt-γ2, pSV2gpt-γ3 and pSV2gpt-γ4 were separately digested as above for pSV2gpt-γ2/R, pSV2gpt-γ3/R and pSV2gpt-γ4/R, respectively, with *Eco* RI to produce the linear plasmid vectors pSV2gpt-γ2/R, pSV2gpt-γ3/R and pSV2gpt-γ4/R respectively. Each of these 3 linear plasmid vectors were separately ligated with f83R. Plasmid maps for the resulting plasmids are shown in Figure 35, p83-γ2; Figure 36, p83-γ3; and Figure 37, p83-γ4.

All ten of the circular plasmid constructs (p49γ1-7.8; p49γ1-2.3; p83γ1-7.8; p83γ1-2.3, p49-γ2; p83-γ2; p49-γ3; p83-γ3; p49-γ4; and p83-γ4) were then linearized for transformation by digesting with the restriction enzyme *Nde* I. The *Nde* I site in the plasmids is in a region that is not essential for the expression of the chimeric immunoglobulin gene or the selectable marker gene, gpt. The plasmids need to be in a linear form prior to transformation into a recipient cell to enhance selected integration of the DNA into the host cell genomic DNA.

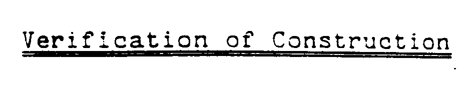

Since the EcoRI fragments can be ligated in either orientation, the correct orientation was determined by digestion with *Nco* I. In the constructions set forth above, correct ligations for plasmid construction are confirmed by performing restriction enzyme site mapping on the plasmid. The restriction enzyme map generated from restriction enzyme digestion and gel electrophoresis is compared to that which can be theoretically generated from the individual starting fragments. Because of the experience with the transcriptional orientation in the light chain vectors, the heavy chain vectors were constructed only in the opposite transcriptional orientation to the gpt gene.

### Transformation of Plasmids into Mouse Plasmacytoma Cells

When both light chain and heavy chain chimeric genes were transformed into the same cell, tetrameric (H₂L₂) immunoglobulins are obtained. Synthesis and secretion of these "chimeric" antibody proteins was accomplished by introducing the chimeric (mouse V:human C region) genes into mouse plasmacytoma cells(Sp2/). Transformation was achieved by electroporation [Sahagan, B.C. et al., *J. Immunology* 137, 1066 (1986)].

Expression of chimeric (mouse V:human C region) genes in transformed mouse plasmacytoma cells (Sp2/0) is achieved using two different techniques. In one mode, different ratios of light chain genes to heavy chain genes can be introduced together. This is referred to as cotransformation. Alternatively, stable clones carrying the chimeric light chain gene can be obtained and subsequently used in a second mode referred to as targeted transformation. In each method, the goal is to obtain clones containing genes for both the H chain and L chain which produce intact H₂L₂ immunoglobulin mentioned above.

### A. Cotransformations

Co-transformation involves the transformation of cells with both drug resistance markers at the same time and subsequent selection with one or both drugs. Co-transformation of heavy chain and light chain vectors (at ratios of 1:1 and 1:10, respectively) was originally performed using only neo selection. Neo-resistant cell lines were obtained which expressed the first chimeric IgG1 antibodies with demonstrable TAG72 binding activity. Cotransformation was conducted pursuant to the protocols set forth in Cornelia Gorman, "High Efficiency Gene Transfer into Mammalian Cells", DNA Cloning, Vol II, D. M. Glover ed, IRL Press, Oxford, England (1985).

### B. Targeted Transformations

Constructs containing light and heavy chimeric immunoglobulin genes were sequentially transformed using into Sp2/0 mouse plasmacytoma cells. Targeted transformation involves transformation and selection with a vector containing a first drug-resistance gene (i.e., Geneticin for the chimeric light chain gene vector), followed by transformation and selection with a vector containing a second drug resistance gene (i.e., mycophenolic acid for the chimeric heavy chain gene vector).

### Neo Selection

Prior to transformation with pSV2-neo vectors which contain chimeric light chain constructions, drug selection conditions for inhibition of growth of untransformed Sp2/0 plasmacytoma cells [obtained from the American Type Culture Collection (ATCC)] were established by titration of the neomycin-like drug, Geneticin (GIBCO). Published values for concentrations of Geneticin used for drug selection ranged from 100-1000 µg/mL. Concentrations above 400 µg/mL were found to prevent growth of Sp2/0 cells in our tissue culture environment.

### Construction of Light Chain Containing Cells

Sp2/0 mouse plasmacytoma cells were initially transformed with light chain-containing pSV2-neo vectors as follows. Cells were grown in RPMI 1640 medium with 5 percent fetal calf serum. Cells were washed in PBS and suspended to a concentration of 1 X 10⁷ viable cells/mL PBS. 0.8 mL of cells were transferred to an electroporation curvette (on ice) containing 20 µg of light chain-containing pSV2-neo vector (pRL105 and pRL 150 for the CC49 chimeric L chain and pRL203 and pRL230 for the CC83 chimeric L chain) linearized with Aat II restriction endonuclease. Aat II was inactivated by heating the samples to 65 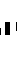 C for 10 minutes. The linearized DNA was ethanol precipitated and subsequently dissolved in 10-20 microliters of PBS. After 15 minutes on ice, electroporation was performed using a Gene Pulser electroporation apparatus with added capacitance extender (BioRad) at 0.2 kvolts and 960 µF. The time constant (τ) was generally about 26 msec.

After transformation, cells were allowed to recover on ice for 15 minutes to allow relaxation of perturbed membranes. Afterwards, the cells were suspended in 24 mL of RPMI 1640 medium containing 5% fetal calf serum (RPMI+) and transferred to a 96 or 24 well plate. To decrease the probability of more than one drug resistant cell per well, the cells were also diluted 10-fold in medium (RPMI+) and plated into another 96-well (or 24 well) plate. The cell suspension was incubated at 37°C and 5 percent CO₂ atmosphere.

After 48 hours (to allow for expression of drug resistance), the medium was removed and replaced with medium containing 1 mg/mL Geneticin.

After 7-10 days, Geneticin-resistant clones were subcultured and the cells screened for chimeric light chains by cytostaining.

### Cytostaining

Aliquots of cells were pelleted onto a glass slide using a cytospin-2 centrifuge (Shandon, Inc.). After air drying, the cells were fixed in acetic acid/ethanol (5 parts acetic acid/95 parts ethanol). After rinsing 3 times with PBS (without CA⁺² and Mg⁺²), the slides were placed in a humid chamber (100% RH), and stained for 20 minutes with 20 µl of goat anti-human Kappa-FITC, a fluorescent dye-conjugated antibody which is specific for human kappa light chains. The conjugated antibody was diluted 1:3 with 1% BSA in PBS. After washing overnight with PBS, the slides were mounted with fluoromount-G, histologic mounting medium (obtained from Southern Biotech) under a coverslip. The slides were observed with an Olympus model BH-2 microscope equipped with an epi-illumination U.V. attachment.

Based on the intensity of fluorescence, the constructions with the orientation of the light chain in opposite transcriptional orientation relative to the direction of transcription of the neo^{r} gene in the vector, was found to give the highest L chain expression. Therefore, pRL105 was the preferred CC49 L chain construction and pRL230 was the favored CC83 L chain construction. As a result of these experiments the following chimeric light chain-containing cell lines (derived from Sp2/0) were used for the targeted transformations:
For the CC49 chimeric L chain one cell line (49K-13-13) was obtained which expressed the chimeric light chain derived from CC49. This cell line was used for all subsequent targeted transformations with chimeric heavy chain vectors for constructs using the chimeric CC49 light chain.

For the CC83 chimeric L chain three cell lines (83K-26-5, 33K-34-10, and 83K-42-2) were obtained which expressed the chimeric light chain derived from CC83. One cell line (83K-26-5) stained more intensely than the others and had localized regions of cytoplasmic immunofluorescence. All three cell lines were compared for their relative ability to produce high levels of chimeric antibody after transformation with the chimeric CC83 g1 heavy chain vector. More clones expressing chimeric antibodies were derived from electroporation of the 83K-34-10 target than either of the other two chimeric light chain target cell lines. Therefore, the 83K-34-10 light chain cell line was used as a target for subsequent electroporations with chimeric heavy chain vectors for constructs containing the CC83 light chain variable region.

### Generation of gpt Resistant clones carrying CC49 and CC83 chimeric H chain constructions

Prior to transformation with PSV2-gpt vectors, which contain chimeric heavy chain constructions, drug selection for inhibition of growth of untransformed Sp2/0 plasmacytoma cells [obtained from the American Type Culture Collection (ATCC)] were established. Conditions for drug selection of cells transformed with pSV2-gpt vectors were more difficult to establish. The *E. coli gpt* gene, which codes for the enzyme guanosine phosphoribosyl transferase, confers the ability to utilize xanthine and hypoxanthine as substrates for the biosynthesis of guanine when the mammalian guanine metabolic pathway is inhibited by mycophenolic acid (MPA).

Published values for the concentrations of MPA which allow for the growth of other lymphoid cell lines transformed with pSV2-gpt vectors were found to be almost two orders of magnitude too high to allow for the growth of Sp2/0 cells transformed with pSV2-gpt vectors in our tissue culture environment. Subsequently, a concentration of 0.1ug/mL of MPA was found to be optimal for selection of gpt resistance. In addition, the use of aminopterin and thymidine (to further shut down the guanine pathway) was found to be unnecessary.

### Generation of Clones Producing of Chimeric 44 Antibody

### CH44-1

49K-13-13 cells were used as a target for chimeric heavy chain constructs. The cells were transformed with 20 µg chimeric heavy chain DNA vector (p49γ1-7.8 or p49γ1-2.3) linearized by *Nde* I digestion. Transformation by electroporation was performed as above for chimeric light chains.

Selection after 48 hours, however, was performed by replacing the geneticin-containing medium with medium containing geneticin and 0.3 µg/mL mycophenolic acid, 250 µg/mL xanthine, and 10 µg/mL hypoxanthine.

Transformed cells grew to macroscopically visible colonies in 14 days. At that time, 50ul of supernatant was removed and assayed by ELISA methods for binding to TAG and expression of human IgG constant region. Wells containing cells with positive TAG binding were expanded to 24-well plates with fresh drug selection medium and allowed to grow for 3-7 days.

Subcloning was performed as follows. Viable cell counts were determined and the cells were replated into two 96-well plates. One plate received 50 viable cells and the other received 250 viable cells. The unsubcloned cells were expanded to 6-well plates until the cell density was sufficient to allow for storage in liquid nitrogen in the event that re-subcloning would be necessary.

After subcloning, those clones exhibiting the highest chimeric antibody production were selected for chimeric antibody production in bioreactors.

### CH44-2

The procedures used to sequentially transform the Sp2/0 plasmacytoma cells in the construction of CH44-1 were repeated with the exception that 20 µg of p49-γ2, was used as the chimeric heavy chain vector.

### CH44-3

The procedures used to sequentially transform the Sp2/0 plasmacytoma cells in the construction of CH44-1 were repeated with the exception that 20 µg of p49-γ3, was used as the heavy chain vector.

### CH44-4

The procedures used to sequentially transform the Sp2/0 plasmacytoma cells in the construction of CH44-1 were repeated with the exception that 20 µg of p49-γ4, was used as the heavy chain vector.

### Generation of Clones Producing of Chimeric 88 Antibody

### CH88-1

The procedures used to sequentially transform the Sp2/0 plasmacytoma cells in the construction of CH44-1 were repeated with the following exceptions:
83K-26-5, 83K-34-10, and 83K-42-2 cells demonstrating production of chimeric CC83 light chain were transformed as described in the transformation of CH44-1, with the exception that 20 µg of p83γ1-7.8 or p83γ1-2.3, the pSV2gpt vector which contains the chimeric CC83 heavy chain gene was used as the heavy chain vector.

### CH88-2

The procedures used to sequentially transform the Sp2/0 plasmacytoma cells in the construction of CH88-1 were repeated with the exception that 20 µg of p83-γ2, was used as the heavy chain vector.

### CH88-3

The procedures used to sequentially transform the Sp2/0 plasmacytoma cells in the construction of CH88-1 were repeated with the exception that 20 µg of p83-γ3, was used as the heavy chain vector.

### CH88-4

The procedures used to sequentially transform the Sp2/0 plasmacytoma cells in the construction of CH88-1 were repeated with the exception that 20 µg of p83-γ4, was used as the heavy chain vector.

### Generation of Clones Producing of Chimeric 84 Antibody

Because of the high degree of sequence similarity between the heavy chain variable regions of CC49 and CC83, chimeric antibodies were generated whose light and heavy chains were derived from different parents by mixed targeted transformations. To generate both "mixed" combinations, the chimeric heavy chain γ1 isotype vectors of CC49 and CC83 were electroporated into the chimeric light chain targets 83K34-10 and 49K-13-13 respectively. The resulting cell lines were designated CH48-1 and CH84-1, where the first numerical designation represents the heavy chain and light chain parents, respectively. For example, CH48-1 represents the γ1 isotype with the heavy chain derived from CC49 and the light chain derived from CC83.

The CH48-1 composite antibody did not bind to TAG72. This was not due to the inability to make bona fide chimeric antibody, Since most drug-resistant cell lines produced chimeric IgG (as determined by ELISA analysis using Goat Anti-Human Ig trap with Goat Anti-Human IgG-Alkaline Phosphatase as a probe). If any binding affinity were present, it was significantly less than that observed for the first generation antibody B72.3, which was approximately an order of magnitude less affinity for TAG72 than either CC49 or CC83.

Surprisingly, CH84-1 bound to TAG72 with affinity similar to both parents. This new antibody was generated de novo in our laboratory and has not yet been detected in nature.

Competition studies were undertaken to determine the specificity of this new mixed-antibody, CH84-1. It should be noted that both CC49 and CC83 exhibit some competitive recognition for the TAG72 antigen. It was found that CH84-1 competed more with CC49 for binding to TAG72 than it did with CC83. This would indicate that the specificity for binding to TAG72 lies in the light chain.

Human γ2, -3, and -4 isotypes were also generated with this mixed-antibody, producing CH84-2, CH84-3, CH84-4 clones.

### CH84-1

The procedure used to sequentially transform the Sp2/0 plasmacytoma cells in the construction of CH44-1 were repeated with the following exception:
49K-13-13 cells demonstrating production of CH44 light chain by cytostaining were then transformed as described in the transformed of CH44-1, with the exception that 20 µg of p83γ1-2.3, the pSV2gpt vector which contains the CH83 heavy chain gene was substituted for p49γ1-2.3, the pSV2gpt vector which contains the CH44 heavy chain gene.

### CH84-2

The procedures used to sequentially transform the Sp2/0 plasmacytoma cells in the construction of CH84-1 were repeated with the exception that 20 µg of p83-γ2, was substituted for p83γ1-2.3.

### CH84-3

The procedures used to sequentially transform the Sp2/0 plasmacytoma cells in the construction of CH84-1 were repeated with the exception that 20 µg of p83-γ3, was substituted for p83γ1-2.3.

### CH84-4

The procedures used to sequentially transform the Sp2/0 plasmacytoma cells in the construction of CH84-1 were repeated with the exception that 20 µg of p83-γ4, was substituted for p83γ1-2.3.

### Purification of Recombinant Antibodies

Cells expressing the chimeric antibodies were removed by centrifugation from the culture medium and the medium was filtered through a 0.2µm filter. Chimeric antibodies were purified in two steps from culture supernatants. In the first step of the purification, a protein A affinity cartridge (Nygene Corporation Yonkers, NY) was utilized according to the manufacturer's specifications. Up to 1.0 L of culture supernatant was passed through a 1 mg capacity cartridge, at 5 mL/min. The cartridge was washed with phosphate buffered saline (PBS) to remove traces of albumin. The chimeric antibody was recovered by elution with 0.1M sodium nitrate buffer, pH 3.0. The pH of the fractions containing the chimeric antibody were immediately adjusted to neutrality with a 1M solution of Trizma base. Final purification was achieved from this solution, after concentration on an Amicon centricon 30 unit, by gel filtration using a Pharmacia Superose 12 HR 16/50 column as specified by the manufacturer (Pharmacia, Piscataway, NJ).

### EXAMPLE: Generation of an Immunoglobulin Containing the Murine V_{H}αTAG germline Variable Region

The following examples are set forth to provide a skilled artisan with a reproducible technique for preparing an antibody having a V_{H} region encoded by a DNA sequence derived from V_{H}αTAG.

### Components for an Expressible V_{H}αTAG Heavy Chain Gene

A mouse-human chimeric antibody molecule can be generated which contains the murine V_{H}αTAG germline heavy chain variable region, a light chain variable region that is complementary to the V_{H}αTAG V_{H}, such as either the CC49 or CC83 murine light chain variable region, and human constant regions.

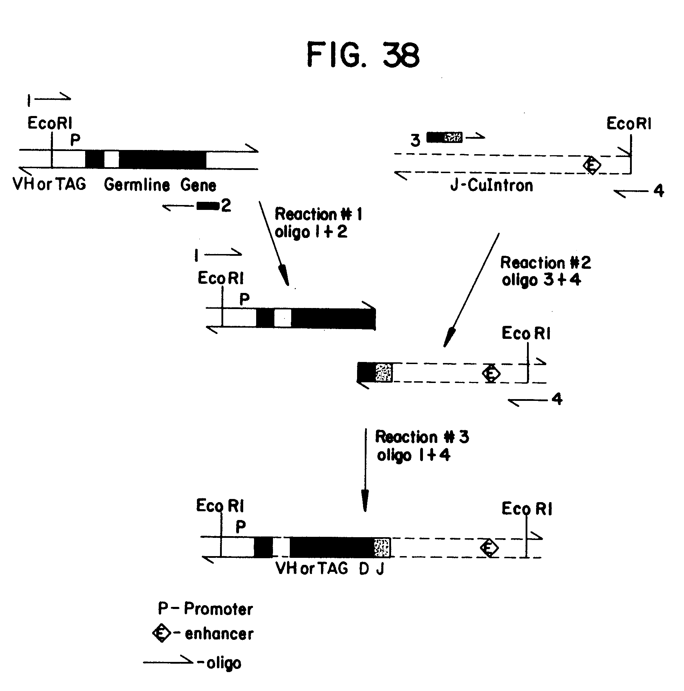

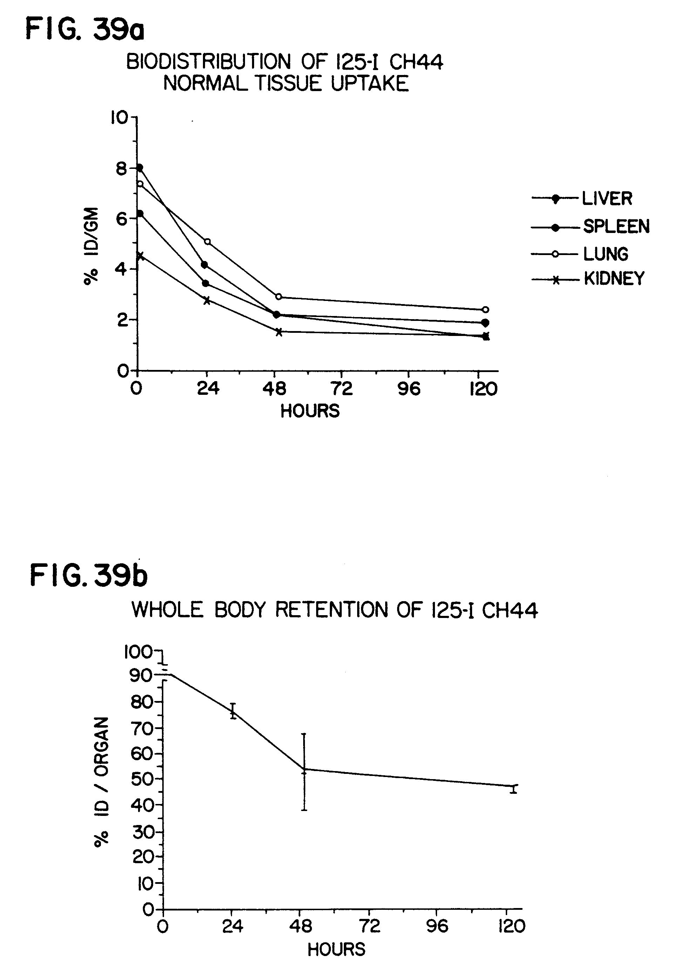

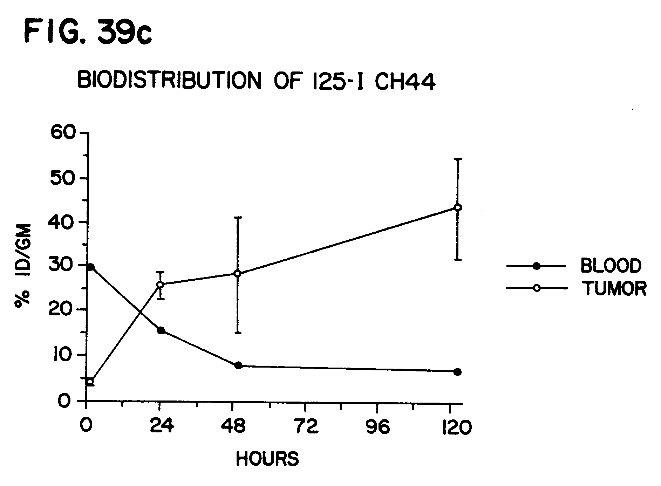

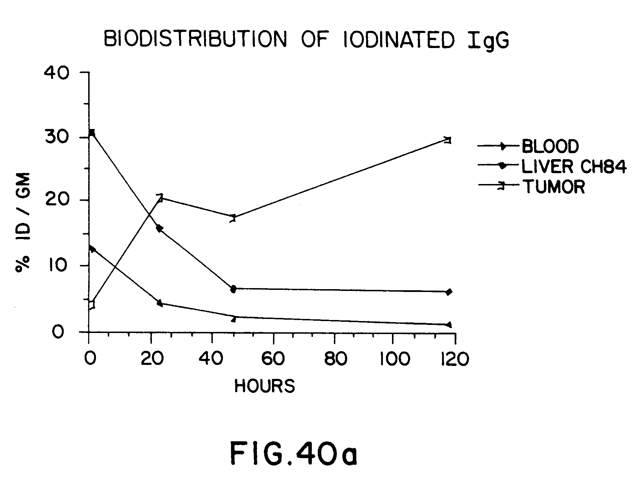

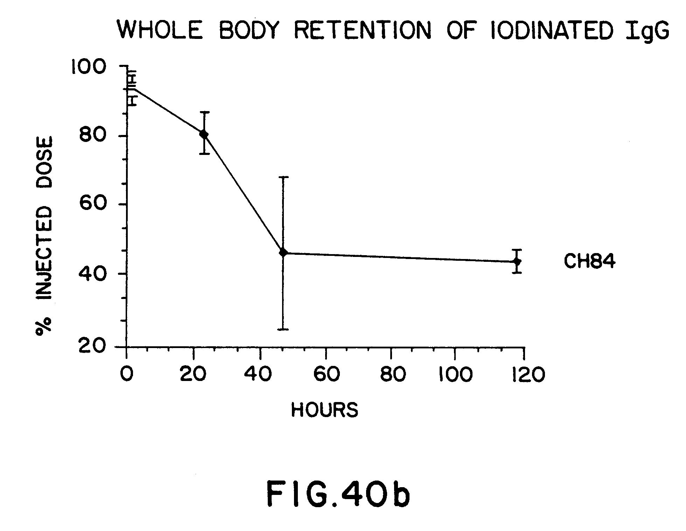

The 2.2 kb HindIII germline DNA fragment containing the V_{H}αTAG V_{H} exon sequence is used as a template to obtain a functionally rearranged V_{H}αTAG variable region. The murine genomic J-Cµ intron region is used as a source for the murine heavy chain enhancer sequences. This latter region is obtained from the plasmid pNP9 (see example above on "Isolation of CC49 Heavy Chain Variable Region"). Figure 38 shows the overall reaction for the engineering of hybrid genes based on the method of Horton et al., (1989), supra. Four oligonucleotides (oligos) are designed to be used in enzymatic amplification and modification of the target DNA. Oligo 1 anneals to the 5′ end of V_{H}αTAG spanning the *Eco*RI site which is 249 bp 5′ to the ATG initiation codon. Oligo 2 anneals to sequences complementary to the 3' end of the V_{H}αTAG exon and also contains sequences coding for a D segment. The D segment sequences in oligo 2 do not anneal with any V_{H}αTAG sequences. Oligo 3 contains sequences complementary to the 5' end of the murine genomic J-Cµ region and incorporates sequences encoding the D segment (same as in oligo 2) and the J segment. Oligo 4 anneals to the 3' end of the J-Cµ region and contains sequences complementary to the *Eco*RI site located 1219 bp 3' to J_{H}4 The sequence of these oligos follow:
Oligo 1 5'GTCTA*GAATTCAT*AAAAACTTTATG (25 mer)
Oligo 2 CAGTGTATTTCTGTAAAAGATCTACTATGGTTACG (35 mer) 
Oligo 3 5'TCTACTATGGTTACGTGGGGTCAAGGAACCTCAGTCACC
GTCTCCTCAGGTAAGAATGGCCTCTCCAGGTCT 3' (72 mer)
Oligo 4 5' ACTTCTAAAATGTATTTA*GAATTC*ATTTTC 3'
In this example, the D sequence is SP2.3 taken from the published sequence of Kurosawa and Tonegawa *J*. *Exp.Med*., 155:201 (1982). The D sequence is shown in bold face type in oligos 2 and 3. Any other characterized murine or human D segment can be used by substituting their sequence in these positions of oligo 2 and 3.

The J segment in oligo 3 is underlined. It is the murine J_{H}4 taken from the published sequence of Gough and Bernard *Proc. Natl. Acad. Sci. (USA)*, 78:509 (1981). The inclusion of any other murine of human J segment can be made by substituting their sequences for the sequence of J_{H}4 in oligo 3.
In oligo 1 and 4 the *Eco*RI sites (*GAATTC*) are shown in italics.

### Assembly of Intact V_{H}αTAG Genes

Two separate DNA amplification reactions are performed using the components described above. DNA amplification reaction #1 copies the V_{H}αTAG sequence and adds a D segment to its 3′ end. DNA amplification reaction #2 copies the murine intron sequences containing the heavy chain enhancer sequences and adds the D and J segments encoded within oligo 3. The amplified products from reaction 1 and 2 are gel purified, combined and oligos 1 and 4 are added to initiate reaction #3. In reaction 3, the products of reactions 1 and 2 anneal across their common D sequences. Subsequent DNA amplification from oligos 1 and 4 yields the product shown at the bottom of Figure 38. This fragment is digested with *Eco*RI and gel purified. The modified V_{H}αTAG fragment is ligated into the *Eco*RI site of pSV2gptγ1(2.3) as described in the above example "Heavy Chain Chimeric Constructs". The entire V_{H}αTAG-D-J-enhancer containing fragment is sequenced completely to ensure that no mutations have been introduced during the DNA amplification reactions. The other three heavy chain γ isotypes can be generated by ligating the same modified V_{H}αTAG fragment into the other three γ containing pSV2gpt vectors (pSV2gpt-γ2; pSV2gpt-γ3; pSV2gpt-γ4).

### Expression of the Modified V_{H}αTAG Gene

The modified V_{H}αTAG gene containing plasmids can be linearized with *Nde*I and introduced via electroporation into the chimeric CC49 or CC83 light chain expressing cell lines (see example above, "C. Targeted Transformations". The transformed cells are selected for growth in the presence of Geneticin and mycophenolic acid as outlined above in "C. Targeted Transformations". The presence of expressed antibody is monitored by TAG72 ELISA (see section in RESULTS, Enzyme-Linked Immunoassays (ELISA). The expressed antibody from these cells will contain human Ig γ1,κ constant regions with the CC49 or CC83 light chain variable region and a heavy chain variable region from the modified V_{H}αTAG germline V_{H} exons.

Four examples of modified V_{H}αTAG heavy chain variable region constructs having a variety of D and J segments are shown below;

| V_{H} Segment | D Segment | J Segment |
|---|---|---|
| V_{H}αTAG #i | mouse D (SP2.3) | mouse J |
| V_{H}αTAG #ii | human D (D1) | mouse J |
| V_{H}αTAG #iii | mouse D (SP2.3) | human J |
| V_{H}αTAG #iv | human D (D1) | human J |

The sequence of the human D sequence D1 is obtained from Siebenlist et al., *Nature*, 294:631 (1981). The sequence of the human J_{H}1 obtained from Ravetch et al., *Cell* 27, 583 (1981).

The generation of V_{H}αTAG #i is described with the above diagramed oligos 1 through 4. To generate V_{H}αTAG #ii through -iv the corresponding D and J segments need to be changed in oligos 2 and 3. The following oligos delineate these changes. Substitution of these oligos in reaction #1 and reaction #2 will result in the generation of the V_{H}αTAG #ii through -iv.

VHαTAG #ii

Oligo 2	5' CAGTGTATTTCTGTAAAAGAGTACTGGTGGT (34 mer)
GTAT

Oligo 3	5' GTACTGGTGGTGTATTGGGGTCAAGGAACC (72 mer)
TCAGTCACCGTCTCCTCAGGTAAGAATGGCCT
CTCCAGGTCT 3'

VHαTAG #iii

Oligo 2	5' CAGTGTATTTCTGTAAAAGATCTACTATGG (35 mer)
TTACG

Oligo 3	5' TCTACTATGGTTACGTGGGGCCAGGGCAC (72 mer)
CCTGGTCACCGTCTCCTCAGGTAAGAATGGCCTCTCCAGGTCT
3'

VHαTAG #iv

Oligo 2	5' CAGTGTATTTCTGTAAAAGAGTACTGGTG (35 mer)
GTGTAT

Oligo 3	5' GTACTGGTGGTGTATTGGGGCCAGGGCAC (72 mer)
CCTGGTCACCGTCTCCTCAGGTAAGAATGGC
CTCTCCAGGTCT 3'

### Results

### A. Chimeric Antibody - Producing Cell Lines

Simultaneous detection of heavy and light chains was accomplished using two probe antibodies:
1) Goat anti-human kappa labeled with the fluorescing dye FITC and;
2) Goat anti-human IgG labeled with the fluorescing dye TRITC.

Cell lines having positive responses for both heavy and light chains were tested further for associated chimeric immunoglobulin production and biological activity viz. binding to TAG72.

### Enzyme-Linked Immunoassays (ELISA)

In order to select a transformed cell producing a chimeric monoclonal antibody, the ELISA technique was employed. Clones containing the heavy chain and light chain drug selection constructs were selected by their growth in selective culture medium. The following cell lines were tested (1) CH44-1: a cell line having CC49 V_{H}, CC49 V_{L}, and constant region of IgG₁; (2) CH44-2: a cell line having CC49 V_{H}, CC49 V_{L}, and constant region of IgG₂; (3) CH44-4: a cell line having CC49 V_{H}, CC49 V_{L}, and constant region of IgG₄; (4) CH88-1: a cell line having V_{H}, CC83 V_{L}, and constant region of IgG₁; (5) CH88-2: a cell line having CC83 V_{H}, CC83 V_{L}, and constant region of IgG₂; (6) CH88-3: a cell line having CC83 V_{H}, CC83 V_{L}, and constant region of IgG₃; (7) CH88-4: a cell line having CC83 V_{H}, CC83 V_{L}, and constant region of IgG₄; (8) CH84-1: a cell line having CC83 V_{H}, CC49 V_{L}, and constant region of IgG₁; (9) CH84-2: a cell line having CC83 V_{H}, CC49 V_{L}, and constant region of IgG₂; (10) CH84-3: a cell line having CC83 V_{H}, CC49 V_{L}, and constant region of IgG₃; and (II) CH84-4: a cell line having CC83 V_{H}, CC49 V_{L}, and constant region of IgG₄.

Supernatants of these cultures were subjected to ELISA. The presence of chimeric anti-TAG72 antibody was measured directly by reaction of an excess of goat anti-human IgG antibody labeled with an enzyme such as alkaline phosphatase, after allowing the chimeric anti-TAG72 antibody to bind to microtiter wells coated with antigen (TAG72). Anti-TAG72 activity was determined as a criterion for successful recombination.

After growth for 14 days, 50 µl of supernatant was removed from the wells of the subcloned cells and re-assayed for TAG binding by ELISA. Samples of supernatants (50 µl) from drug resistant cell lines were applied to wells of Nunc Immulon 96-well plates which had previously been coated with TAG antigen (1/50 dilution). After washing to remove unbound material, the wells were incubated with Goat Anti-Human IgG antibodies conjugated with Alkaline Phosphatase (GAHIgG-AP) as a probe to detect the human constant regions of the chimeric antibodies which had bound to the TAG antigen immobilized on the plate. Another washing to remove unbound probe (GAHIgG-AP), followed by addition of a chromogenic alkaline phosphatase substrate, allowed color to develop in those wells which possessed TAG binding associated with human constant regions (i.e., chimeric anti-TAG72 antibodies). Absorbance readings at 405 nm indicate the relative amount of chimeric antibody produced by the drug-resistant cell lines.

### CH44-1

Anti-TAG72 activity was used as a criterion for successful recombination. Wells of microtiter plates were coated with TAG by incubating 50 µl of a 1:75 dilution of purified TAG72 [Muraro, R., et al., *Cancer Research* 48, 4588-4596 (1988)] for 18 hours at room temperature. The wells were then washed 4 times with phosphate buffered saline (PBS), and then blocked with BSA, by incubating 50 µl of 0.5 percent BSA in PBS for 2 hours at 37°C, followed by washing 4 times with PBS. These plates are stable if kept moist at 4°C. 50 microliters of sample are then applied to each well. A blank containing fresh medium is used as a control. All of the samples were incubated either in the plate for 90 minutes at 37°C or overnight at 4°C in a closed container.

The plates were then washed 4 times with PBS, and goat anti-human IgG-alkaline phosphate (Southern Biotech Assoc.) was applied to each well by adding 50 µl of a 1:250 dilution. The solution was incubated at 37°C for 90 minutes. Color development was monitored after washing the plates 4 times with PBS to remove the probe.

The substrate was incubated in 200 µl solution of substrate p-nitrophenyl phosphate (Kirkegaard & Perry) in ethanolamine buffered saline for 6 minutes at room temperature for color development. The optical density at 450 nm of each well was read by a Dynatech microplate reader (Dynatech Inc.).

The Sp2/0 colonies in wells with supernatants having TAG72-binding chimeric antibody activity were subcloned by limited dilution. Individual subclones were chosen on the basis of relatively high production of chimeric antibody.

### CH44-2

The TAG-ELISA procedure used with CH44-1 was repeated with the exception that the antibody was CH44-2.

### CH44-3

The TAG-ELISA procedure used with CH44-1 was repeated with the exception that the antibody was CH44-3.

### CH44-4

The TAG-ELISA procedure used with CH44-1 was repeated with the exception that the antibody was CH44-4.

### CH88-1

The TAG-ELISA procedure used with CH44-1 was repeated with the exception that the antibody was CH88-1.

### CH88-2

The TAG-ELISA procedure used with CH44-1 was repeated with the exception that the antibody was CH88-2.

### CH88-3

The TAG-ELISA Procedure used with CH44-1 was repeated with the exception that the antibody was CH88-3.

### CH88-4

The TAG-ELISA Procedure used with CH44-1 was repeated with the exception that the antibody was CH88-4.

### CH84-1

The TAG-ELISA procedure used with CH44-1 was repeated with the exception that the antibody was CH84-1.

### CH84-2

The TAG-ELISA procedure used with CH44-1 was repeated with the exception that the antibody was CH84-2.

### CH84-3

The TAG-ELISA procedure used with CH44-1 was repeated with the exception that the antibody was CH84-3.

### CH84-4

The TAG-ELISA procedure used with CH44-1 was repeated with the exception that the antibody was CH84-4.

### CH48-1

The TAG-ELISA procedure used with CH44-1 was repeated with the exception that the antibody was CH84-4.

### B. In Vivo Carcinoma Targeting

The chimeric monoclonal antibodies used in animal studies and shown in Tables 1- 4 below were labeled with Na¹²⁵I using Iodogen (Pierce Chemical, Rockford, IL). More specifically, from about 0.5-2 mg of purified chimeric monoclonal antibodies were adjusted to about 0.5 mL 0.1M sodium phosphate buffer (pH 7.2) and then added to a 12 cm x 75 cm glass tube coated with 50 µg of Iodogen followed by addition of from 0.1 -0.5 mCi of Na¹²⁵I (New England Nuclear, Boston, MA). After a 2 min incubation at room temperature, the protein was removed from the insoluble Iodogen, and the unincorporated ¹²⁵I was separated from the antibody by gel filtration through a 10 mL column Sephadex™ G-25 using PBS as the buffer. The iodination protocol yielded labeled IgG chimeric antibody with a specific activity of 0.05 to 0.2 µCi/µg.

Female athymic mice (nu/nu) on a CD1 background were obtained from Charles River at approximately 4 weeks of age. Nine days later, mice were inoculated subcutaneously (0.1 mL/mouse) with LS174T cells (1 x 10⁶ cells/animal).

Athymic mice bearing carcinomas 70 to 400 mg in weight, approximately 12 to 13 days after inoculation of the cells were given injections intravenously of from 0.5 to 2.0 µCi (10-50 µg protein) in PBS of the chimeric monoclonal antibodies, which had been iodinated as described above. Groups of five mice were sacrificed at varying times by exsanguination, the carcinoma and normal tissues were excised and weighed, and the cpm were measured in a gamma counter. The cpm/mg of each tissue was then determined and compared to that found in the carcinoma.

The results for CH44-1 are shown in Tables 1-2, and Figures 39A, 39B, and 39C. The results for CH84-1 are shown in Tables 3-4, and Figures 40A and 40B.

### Percent Injected Dose Per Gram of ¹²⁵I-Labeled Antibody

**Table 1**

| Tissue | CH44-1 | | | |
|---|---|---|---|---|
| | 0.75 Hour | 23.5 Hours | 49.5 Hours | 122 Hours |
| blood, total | 29.70 | 15.84 | 8.09 | 7.31 |
| Liver | 8.13 | 4.13 | 2.19 | 1.96 |
| Spleen | 6.19 | 3.39 | 2.12 | 1.36 |
| Kidney | 4.35 | 2.80 | 1.52 | 1.33 |
| tumor | 3.31 | 25.95 | 28.83 | 44.16 |
| lung | 7.34 | 5.39 | 2.90 | 2.36 |
| tumor,wt | 0.18 | 0.12 | 0.09 | 0.11 |

As shown in Table 1, at approximately 122 hours post-injection, the percent injected dose to tumor for CH44-1 was 44.16 percent. CH44-1 was, therefore, efficient in targeting the human tumor in-situ. This demonstrates that the chimeric monoclonal antibodies of the present invention were efficient for *in vivo* carcinoma targeting and thus are useful for *in vivo* treatment of cancer.

### Percent Injected Dose Per Organ of ¹²⁵I-Labeled Antibody

**Table 2**

| Tissue | CH44-1 | | | |
|---|---|---|---|---|
| | 0.75 Hour | 23.5 Hours | 49.5 Hours | 122 Hours |
| blood, total | 47.72 | 23.03 | 13.29 | 12.01 |
| Liver | 10.97 | 5.20 | 3.20 | 2.69 |
| Spleen | 1.09 | 0.48 | 0.25 | 0.22 |
| Kidney | 1.25 | 0.72 | 0.42 | 0.40 |
| tumor | 0.57 | 3.08 | 2.82 | 4.55 |
| lung | 1.20 | 0.87 | 0.57 | 0.37 |
| Gl tract | 6.64 | 4.78 | 3.96 | 2.83 |
| carcass | 43.17 | 49.68 | 35.35 | 29.95 |
| whole body retention | 91.30 | 76.34 | 53.28 | 46.20 |

As shown in Table 2, at 122 hours post-injection, the percent of injected dose tumor for CH44-1 was 4.55 percent. CH84-1 was, therefore efficient in targeting the human tumor in-situ. This demonstrates that the chimeric monoclonal antibodies of the present invention were efficient for *in vivo* carcinoma targeting and thus were useful in *in vivo* treatment of cancer.

### Percent Injected Dose Per Gram of ¹²⁵I-Labeled Antibody

**Table 3**

| Tissue | CH84-1 | | | |
|---|---|---|---|---|
| | 1 Hour | 23 Hours | 47 Hours | 118-119 Hours |
| blood | 30.68 | 15.65 | 6.74 | 6.49 |
| Liver | 12.55 | 4.26 | 2.35 | 1.57 |
| Spleen | 10.93 | 3.35 | 2.56 | 1.70 |
| Kidney | 5.59 | 2.51 | 1.53 | 1.55 |
| tumor | 4.06 | 20.52 | 17.58 | 30.27 |
| lung | 10.77 | 4.80 | 2.58 | 2.24 |
| tumor,wt. | 0.15 | 0.22 | 0.20 | 0.24 |

As shown in Table 3, at approximately 118 hours post-injection, the percent of injected dose to tumor for CH84-1 was 30.27 percent. CH84-1 was, therefore, efficient in targeting the human tumor in-situ. This demonstrates that the chimeric monoclonal monoclonal antibodies of the present invention were efficient for *in vivo* carcinoma targeting and thus were useful in *in vivo* treatment of cancer.

### Percent Injected Dose Per Organ of ¹²⁵I-Labeled Antibody

**Table 4**

| Tissue | CH84-1 | | | |
|---|---|---|---|---|
| | 1 Hour | 23 Hours | 47 Hours | 118-119 Hours |
| blood, total | 45.98 | 22.11 | 10.08 | 9.37 |
| Liver | 13.64 | 5.34 | 3.13 | 1.94 |
| Spleen | 1.35 | 0.49 | 0.32 | 0.16 |
| Kidney | 1.39 | 0.62 | 0.38 | 0.38 |
| tumor | 0.59 | 4.33 | 3.63 | 7.02 |
| lung | 1.77 | 0.69 | 0.42 | 0.31 |
| Gl tract | 7.38 | 4.92 | 3.41 | 2.32 |
| carcass | 44.83 | 52.19 | 30.32 | 24.06 |
| whole body retention | 93.58 | 81.00 | 47.14 | 45.48 |

As shown in Table 4, at approximately 118 post-injection, the percent of injected dose to tumor for CH84-1 was 7.02 percent. CH84-1 was, therefore, efficient in targeting the human tumor in-situ. This demonstrates that the chimeric monoclonal antibodies of the present invention were efficient for *in vivo* carcinoma targeting and thus were useful in *in vivo* treatment of cancer.

Deposit of Cell lines Producing Chimeric Antibodies

Eleven illustrative cell lines secreting chimeric antibodies, all having a kappa light chains, made by the above examples were deposited at the American Type Culture Collection (ATCC) on October 19, 1988. Specifically, the following cell lines have been deposited: (1) CH44-1: a cell line having CC49 V_{H}, CC49 V_{L}, and constant region of IgG₁ (ATCC No. HB 9884); (2) CH44-2: a cell line having CC49 V_{H}, CC49 V_{L}, and constant region of IgG2 (ATCC No. HB 9880); (3) CH44-4: a cell line having CC49 V_{H}, CC49 V_{L}, and constant region of IgG₄ (ATCC No. 9877); (4) CH88-1: a cell line having V_{H}, CC83 V_{L}, and constant region of IgG₁ (ATCC No. 9882); (5) CH88-2: a cell line having CC83 V_{H}, CC83 V_{L}, and constant region of IgG₂ (ATCC No. 9881); (6) CH88-3: a cell line having CC83 V_{H}, CC83 V_{L}, and constant region of IgG₃ (ATCC No. 9876); (7) CH88-4: a cell line having CC83 V_{H}, CC83 V_{L}, and constant region of IgG₄ (ATCC No. 9874); (8) CH84-1: a cell line having CC83 V_{H} CC49 V_{L}, and constant region of IgG₁ (ATCC No. 9883); (9) CH84-2: a cell line having CC83 V_{H}, CC49 V_{L}, and constant region of IgG₂ (ATCC No. 9879); (10) CH84-3: a cell line having CC83 V_{H}, CC49 V_{L}, and constant region of IgG₃ (ATCC No. 9878); and (11) CH84-4: a cell line having CC83 V_{H}, CC49 V_{L}, and constant region of IgG₄ (ATCC No. 9875).

The present invention is not to be limited in scope by the cell lines deposited since the deposited embodiments are intended as a single illustration of one aspect of the invention and all cell lines which are functionally equivalent are within the scope of the invention. Indeed, while this invention has been described in detail and with reference to specific embodiments thereof, it will be apparent to one skilled in the art that various changes and modifications could be made therein without departing from the spirit and scope of the appended claims.

## Claims (Claims for the following Contracting State(s): AT, BE, CH, DE, FR, GB, IT, LI, LU, NL, SE)

1. An antibody produced by one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875) or a fragment thereof, which is capable of selectively reacting with a particular antigen or antigen family.

2. An antibody or antibody fragment conjugate comprising an antibody or antibody fragment of claim 1 conjugated to an imaging marker.

3. The antibody or antibody fragment conjugate of claim 2, wherein the imaging marker is ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re or ^{99m}Tc.

4. An antibody or antibody fragment conjugate comprising the antibody or antibody fragment of claim 1 conjugated to a therapeutic agent.

5. The antibody or antibody fragment conjugate of claim 4, wherein the therapeutic agent is a radionuclide, drug or biological response modifier, toxin or another antibody.

6. The antibody or antibody fragment conjugate of claim 5, wherein the radionuclide is ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I or ¹¹¹In.

7. The antibody or antibody fragment conjugate of claim 5, wherein the drug or biological response modifier is methotrexate, adriamycin or lymphokine.

8. A DNA sequence encoding at least a portion of an antibody, which is capable of selectively reacting with a particular antigen or antigen family, and being contained in one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875).

9. The DNA sequence of claim 8, wherein the sequence encodes for at least a portion of a C_{H} gene encoding IgG₁₋₄, IgM, IgA, IgD or IgE.

10. A biologically functional expression vehicle containing the DNA sequence of any one of claims 8 or 9.

11. A cell transformed with the biologically functional expression vehicle of claim 10.

12. A cell which produces an antibody as produced by one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875).

13. A composition comprising the antibody or antibody fragment of claim 1 in a pharmaceutically acceptable, non-toxic, sterile carrier.

14. A composition comprising the antibody or antibody fragment conjugate of any one of claims 2 through 7 in a pharmaceutically acceptable, non-toxic, sterile carrier.

15. A process for preparing an antibody or antibody fragment conjugate which comprises contacting an antibody or antibody fragment, as defined in claim 1, with an imaging marker or therapeutic agent.

16. A process for preparing a recombinant expression vehicle which comprises inserting a DNA sequence, as defined in any one of claims 8 or 9, into an expression vehicle.

17. A process for preparing a transformed host which comprises inserting the expression vehicle, as defined in claim 10, into a suitable host.

## Claims (Claims for the following Contracting State(s): ES)

1. A process for preparing an antibody or a fragment thereof, which is capable of selectively reacting with a particular antigen or antigen family, characterized by
culturing one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875) and collecting the produced antibody.

2. A process for preparing an antibody or antibody fragment conjugate comprising conjugating an antibody or antibody fragment of claim 1 to an imaging marker.

3. The process of claim 2, wherein the imaging marker is ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re or ^{99m}Tc.

4. A process for preparing an antibody or antibody fragment conjugate comprising conjugating the antibody or antibody fragment of claim 1 to a therapeutic agent.

5. The process of claim 4, wherein the therapeutic agent is a radionuclide, drug or biological response modifier, toxin or another antibody.

6. The process of claim 5, wherein the radionuclide is ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I or ¹¹¹In.

7. The process of claim 5, wherein the drug or biological response modifier is methotrexate, adriamycin or lymphokine.

8. A process for preparing a recombinant expression vehicle which comprises inserting a DNA sequence encoding at least a portion of an antibody, which is capable of selectively reacting with a particular antigen or antigen family, and being contained in one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875) into an expression vehicle.

9. The process of claim 8, wherein the sequence encodes for at least a portion of a C_{H} gene encoding IgG₁₋₄, IgM, IgA, IgD or IgE.

10. A process for preparing a therapeutic composition comprising the antibody or antibody fragment of claim 1 in accordance with any one of claims 4 through 7 in a pharmaceutically acceptable, non-toxic, sterile carrier.

11. A process for preparing a transformed host which comprises inserting an expression vehicle containing the DNA sequence of any one of claims 8 or 9 into a suitable host.

## Claims (Claims for the following Contracting State(s): GR)

1. An antibody produced by one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875) or a fragment thereof, which is capable of selectively reacting with a particular antigen or antigen family.

2. An antibody or antibody fragment conjugate comprising an antibody or antibody fragment of claim 1 conjugated to an imaging marker.

3. The antibody or antibody fragment conjugate of claim 2, wherein the imaging marker is ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re or ^{99m}Tc.

4. Use of an antibody or antibody fragment of claim 1 for the preparation of a therapeutic agent, characterized by conjugating the antibody or antibody fragment to a therapeutic agent.

5. Use of an antibody or antibody fragment according to claim 4, wherein the therapeutic agent is a radionuclide, drug or biological response modifier, toxin or another antibody.

6. Use of an antibody or antibody fragment according to claim 5, wherein the radionuclide is ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I or ¹¹¹In.

7. Use of an antibody or antibody fragment according to claim 5, wherein the drug or biological response modifier is methotrexate, adriamycin or lymphokine.

8. A DNA sequence encoding at least a portion of an antibody, which is capable of selectively reacting with a particular antigen or antigen family, and being contained in one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875).

9. The DNA sequence of claim 8, wherein the sequence encodes for at least a portion of a C_{H} gene encoding IgG₁₋₄, IgM, IgA, IgD or IgE.

10. A biologically functional expression vehicle containing the DNA sequence of any one of claims 8 or 9.

11. A cell transformed with the biologically functional expression vehicle of claim 10.

12. A cell which produces an antibody as produced by one of the cell lines CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) or CH 84-4 (ATCC HB 9875).

13. Use of the antibody or antibody fragment of claim 1 in accordance with any one of claims 4 through 7 in a pharmaceutically acceptable, non-toxic, sterile carrier.

14. A process for preparing a recombinant expression vehicle which comprises inserting a DNA sequence, as defined in any one of claims 8 or 9, into an expression vehicle.

15. A process for preparing a transformed host which comprises inserting the expression vehicle, as defined in claim 10, into a suitable host.

## Patentansprüche (Patentansprüche für folgende(n) Vertragsstaat(en): AT, BE, CH, DE, FR, GB, IT, LI, LU, NL, SE)

1. Antikörper, hergestellt von einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) oder ein Fragment davon, der (das) zum selektiven Reagieren mit einem bestimmten Antigen oder einer bestimmten Antigenfamilie fähig ist.

2. Antikörper- oder Antikörperfragmentkonjugat, umfassend einen Antikörper oder ein Antikörperfragment nach Anspruch 1, der (das) an einen bilderzeugenden Marker konjugiert ist.

3. Antikörper- oder Antikörperfragmentkonjugat nach Anspruch 2, wobei der bilderzeugende Marker ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re oder ^{99m}Tc ist.

4. Antikörper- oder Antikörperfragmentkonjugat, umfassend den Antikörper oder das Antikörperfragment von Anspruch 1, der (das) an ein therapeutisches Mittel konjugiert ist.

5. Antikörper- oder Antikörperfragmentkonjugat nach Anspruch 4, wobei das therapeutische Mittel ein Radionuklid, ein Arzneimittel oder ein Modifikator einer biologischen Antwort, ein Toxin oder ein anderer Antikörper ist.

6. Antikörper oder Antikörperfragmentkonjugat nach Anspruch 5, wobei das Radionuklid ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I oder ¹¹¹In ist.

7. Antikörper- oder Antikörperfragmentkonjugat nach Anspruch 5, wobei das Arzneimittel oder der Modifikator einer biologischen Antwort Methotrexat, Adriamcyin oder Lymphokin ist.

8. DNA-Sequenz, die mindestens einen Teil eines Antikörpers, welcher fähig ist mit einem bestimmten Antigen oder einer bestimmten Antigenfamilie selektiv zu reagieren, codiert und in einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) enthalten ist.

9. DNA-Sequenz nach Anspruch 8, wobei die Sequenz mindestens einen Teil eines C_{H}-Gens codiert, welches IgG₁₋₄, IgM, IgA, IgD oder IgE codiert.

10. Biologisch funktionelles Expressionsvehikel, welches die DNA-Sequenz nach einem der Ansprüche 8 oder 9 enthält.

11. Zelle, die mit dem biologisch funktionellen Expressionsvehikel nach Anspruch 10 transformiert ist.

12. Zelle, die einen Antikörper produziert, wie von einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) produziert.

13. Zusammensetzung, umfassend den Antikörper oder das Antikörperfragment nach Anspruch 1 in einem pharmazeutisch akzeptablen, nicht-toxischen, sterilen Träger.

14. Zusammensetzung umfassend das Antikörper- oder Antikörperfragmentkonjugat nach einem der Ansprüche 2 bis 7 in einem pharmazeutisch akzeptablen, nicht-toxischen, sterilen Träger.

15. Verfahren zum Herstellen eines Antikörper- oder Antikörperfragmentkonjugats, das das in Kontakt Bringen eines Antikörpers oder Antikörperfragments, wie in Anspruch 1 definiert, mit einem bilderzeugenden Marker oder therapeutischen Mittel umfaßt.

16. Verfahren zum Herstellen eines rekombinanten Expressionsvehikels, das das Einfügen einer DNA-Sequenz, wie in einem der Ansprüche 8 oder 9 definiert, in ein Expressionsvehikel umfaßt.

17. Verfahren zum Herstellen eines transformierten Wirts, das das Einbringen des Expressionsvehikels, wie in Anspruch 10 definiert, in einen geeigneten Wirt umfaßt.

## Patentansprüche (Patentansprüche für folgende(n) Vertragsstaat(en): ES)

1. Verfahren zum Herstellen eines Antikörpers oder eines Fragments davon, der (das) fähig zum selektiven Reagieren mit einem bestimmten Antigen oder einer bestimmten Antigenfamilie ist, gekennzeichnet durch
Kultivieren einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875), und Sammeln des produzierten Antikörpers.

2. Verfahren zum Herstellen eines Antikörper- oder Antikörperfragmentkonjugats, umfassend das Konjugieren eines Antikörpers oder eines Antikörperfragments nach Anspruch 1 an einen bilderzeugenden Marker.

3. Verfahren nach Anspruch 2, wobei der bilderzeugende Marker ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re oder ^{99m}Tc ist.

4. Verfahren zum Herstellen eines Antikörper- oder Antikörperfragmentkonjugats, umfassend das Konjugieren des Antikörpers oder Antikörperfragments nach Anspruch 1 an ein therapeutisches Mittel.

5. Verfahren nach Anspruch 4, wobei das therapeutische Mittel ein Radionuklid, ein Arzneimittel oder ein Modifikator einer biologischen Antwort, ein Toxin oder ein anderer Antikörper ist.

6. Verfahren nach Anspruch 5, wobei das Radionuklid ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I oder ¹¹¹In ist.

7. Verfahren nach Anspruch 5, wobei das Arzneimittel oder der Modifikator einer biologischen Antwort Methotrexat, Adriamcyin oder Lymphokin ist.

8. Verfahren zum Herstellen eines rekombinanten Expressionsvehikels, welches umfaßt, Einfügen einer DNA-Sequenz, die mindestens einen Teil eines Antikörpers, welcher fähig ist mit einem bestimmten Antigen oder einer bestimmten Antigenfamilie selektiv zu reagieren, codiert und in einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) enthalten ist, in ein Expressionsvehikel.

9. Verfahren nach Anspruch 8, wobei die Sequenz mindestens einen Teil eines C_{H}-Gens codiert, welches IgG₁₋₄, IgM, IgA, IgD oder IgE codiert.

10. Verfahren zum Herstellen einer therapeutischen Zusammensetzung, umfassend den Antikörper oder das Antikörperfragment nach Anspruch 1 in Übereinstimmung mit einem der Ansprüche 4 bis 7, in einem pharmazeutisch akzeptablen, nicht-toxischen, sterilen Träger.

11. Verfahren zum Herstellen eines transformierten Wirts, das das Einbringen eines die DNA-Sequenz nach einem der Ansprüche 8 oder 9 enthaltenden Expressionsvehikels in einen geeigneten Wirt umfaßt.

## Patentansprüche (Patentansprüche für folgende(n) Vertragsstaat(en): GR)

1. Antikörper, hergestellt von einer der Zellinie CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) oder ein Fragment davon, der (das) zum selektiven Reagieren mit einem bestimmten Antigen oder einer bestimmten Antigenfamilie fähig ist.

2. Antikörper- oder Antikörperfragmentkonjugat, umfassend einen Antikörper oder ein Antikörperfragment von Anspruch 1, der (das) an einen bilderzeugenden Marker konjugiert ist.

3. Antikörper- oder Antikörperfragmentkonjugat nach Anspruch 2, wobei der bilderzeugende Marker ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re oder ^{99m}Tc ist.

4. Verwendung eines Antikörpers oder Antikörperfragments nach Anspruch 1 zur Herstellung eines therapeutischen Mittels, gekennzeichnet durch Konjugieren des Antikörpers oder Antikörperfragments an ein therapeutisches Mittel.

5. Verwendung eines Antikörpers oder Antikörperfragments nach Anspruch 4, wobei das therapeutische Mittel ein Radionuklid, ein Arzneimittel oder ein Modifikator einer biologischen Antwort, ein Toxin oder ein anderer Antikörper ist.

6. Verwendung eines Antikörpers oder Antikörperfragments nach Anspruch 5, wobei das Radionuklid ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I oder ¹¹¹In ist.

7. Verwendung eines Antikörpers oder Antikörperfragments nach Anspruch 5, wobei das Arzneimittel oder der Modifikator einer biologischen Antwort Methotrexat, Adriamcyin oder Lymphokin ist.

8. DNA-Sequenz, die mindestens einen Teil eines Antikörpers, welcher fähig ist mit einem bestimmten Antigen oder einer bestimmten Antigenfamilie selektiv zu reagieren, codiert und die in einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) enthalten ist.

9. DNA-Sequenz nach Anspruch 8, wobei die Sequenz mindestens einen Teil eines C_{H}-Gens codiert, welches IgG₁₋₄, IgM, IgA, IgD oder IgE codiert.

10. Biologisch funktionelles Expressionsvehikel, das die DNA-Sequenz nach einem der Ansprüche 8 oder 9 enthält.

11. Zelle, die mit dem biologisch funktionellen Expressionsvehikel nach Anspruch 10 transformiert ist.

12. Zelle, die einen Antikörper produziert, wie von einer der Zellinien CH44-1 (ATCC HB 9884), CH44-2 (ATCC HB 9880), CH44-4 (ATCC HB 9877), CH88-1 (ATCC HB 9882), CH88-2 (ATCC HB 9881), CH88-3 (ATCC HB 9876), CH88-4 (ATCC HB 9874), CH84-1 (ATCC HB 9883), CH84-2 (ATCC HB 9879), CH84-3 (ATCC HB 9878) oder CH84-4 (ATCC HB 9875) produziert.

13. Verwendung eines Antikörpers oder Antikörperfragments nach Anspruch 1 in Übereinstimmung mit einem der Ansprüche 4 bis 7 in einem pharmazeutisch akzeptablen, nicht-toxischen, sterilen Träger.

14. Verfahren zum Herstellen eines rekombinanten Expressionsvehikels, das das Einfügen einer DNA-Sequenz, wie in einem der Ansprüche 8 oder 9 definiert, in ein Expressionsvehikel umfaßt.

15. Verfahren zum Herstellen eines transformierten Wirts, das das Einbringen des Expressionsvehikels, wie in Anspruch 10 definiert, in einen geeigneten Wirt umfaßt.

## Revendications (Revendications pour l'(les) Etat(s) contractant(s) suivant(s): AT, BE, CH, DE, FR, GB, IT, LI, LU, NL, SE)

1. Anticorps produit par l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875) ou un fragment de celui-ci, qui est capable de réagir sélectivement avec un antigène particulier ou une famille d'antigènes.

2. Anticorps ou fragment d'anticorps conjugué comprenant un anticorps ou fragment d'anticorps selon la revendication 1 conjugué à un marqueur d'imagerie.

3. Anticorps ou fragment d'anticorps conjugué selon la revendication 2, dans lequel le marqueur d'imagerie est ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re ou ^{99m}Tc.

4. Anticorps ou fragment d'anticorps conjugué comprenant l'anticorps ou fragment d'anticorps selon la revendication 1 conjugué à un agent thérapeutique.

5. Anticorps ou fragment d'anticorps conjugué selon la revendication 4, dans lequel l'agent thérapeutique est un radionuclide, un médicament ou un modificateur de réponse biologique, une toxine ou un autre anticorps.

6. Anticorps ou fragment d'anticorps conjugué selon la revendication 5, dans lequel le radionuclide est ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I ou ¹¹¹In.

7. Anticorps ou fragment d'anticorps conjugué selon la revendication 5, dans lequel le médicament ou le modificateur de réponse biologique est le méthotrexate, l'adriamycine ou une lymphokine.

8. Séquence d'ADN codant au moins pour une portion d'un anticorps, qui est capable de réagir sélectivement avec un antigène particulier ou une famille d'antigènes, et qui est contenue dans l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875).

9. Séquence d'ADN selon la revendication 8, dans laquelle la séquence code pour au moins une portion C_{H} d'un gène codant pour IgG₁₋₄, IgM, IgA, IgD ou IgE.

10. Vecteur d'expression biologique fonctionnelle contenant la séquence d'ADN selon l'une des revendications 8 ou 9.

11. Cellule transformée avec le vecteur d'expression biologique fonctionnelle selon la revendication 10.

12. Cellule qui produit un anticorps, comme produit par l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875).

13. Composition comprenant l'anticorps ou fragment d'anticorps selon la revendication 1 dans un support stérile, non toxique, pharmaceutiquement acceptable.

14. Composition comprenant l'anticorps ou fragment d'anticorps conjugué selon l'une des revendications 2 à 7 dans un support stérile, non toxique, pharmaceutiquement acceptable.

15. Procédé pour la préparation d'un anticorps ou fragment d'anticorps conjugué qui comprend le couplage d'un anticorps ou fragment d'anticorps, comme défini selon la revendication 1, avec un marqueur d'imagerie ou un agent thérapeutique.

16. Procédé pour la préparation d'un vecteur d'expression recombinant qui comprend l'insertion d'une séquence d'ADN, comme définie selon l'une des revendications 8 ou 9, dans un vecteur d'expression.

17. Procédé pour la préparation d'un hôte transformé qui comprend l'insertion du vecteur d'expression, comme défini selon la revendication 10, dans un hôte convenable.

## Revendications (Revendications pour l'(les) Etat(s) contractant(s) suivant(s): ES)

1. Procédé pour la préparation d'un anticorps ou d'un fragment de celui-ci, qui est capable de réagir sélectivement avec un antigène particulier ou une famille d'antigènes caractérisé par le fait que l'on cultive l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875) et que l'on recueille l'anticorps produit.

2. Procédé pour la préparation d'un anticorps ou fragment d'anticorps conjugué dans lequel on conjugue un anticorps ou fragment d'anticorps selon la revendication 1, à un marqueur d'imagerie.

3. Procédé selon la revendication 2, dans lequel le marqueur d'imagerie est ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re ou ^{99m}Tc.

4. Procédé pour la préparation d'un anticorps ou fragment d'anticorps conjugué dans lequel on conjugue l'anticorps ou le fragment d'anticorps de la revendication 1 à un agent thérapeutique.

5. Procédé selon la revendication 4, dans lequel l'agent thérapeutique est un radionuclide, un médicament ou un modificateur de réponse biologique, une toxine ou un autre anticorps.

6. Procédé selon la revendication 5, dans lequel le radionuclide est ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I ou ¹¹¹In.

7. Procédé selon la revendication 5, dans lequel le médicament ou le modificateur de réponse biologique est le méthotrexate, l'adriamycine ou une lymphokine.

8. Procédé pour la préparation d'un vecteur d'expression recombinant qui comprend l'insertion d'une séquence d'ADN codant au moins pour une portion d'un anticorps, qui est capable de réagir sélectivement avec un antigène particulier ou une famille d'antigènes, et qui est contenue dans l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875), dans un vecteur d'expression.

9. Procédé selon la revendication 8, selon lequel la séquence code pour au moins une portion C_{H} d'un gène codant pour IgG₁₄, IgM, IgA, IgD ou IgE.

10. Procédé pour la préparation d'une composition thérapeutique comprenant l'anticorps ou fragment d'anticorps de la revendication 1 selon l'une des revendications 4 à 7 dans un support stérile, non toxique, pharmaceutiquement acceptable.

11. Procédé pour la préparation d'un hôte transformé qui comprend l'insertion d'un vecteur d'expression contenant la séquence d'ADN selon l'une des revendications 8 ou 9, dans un hôte convenable.

## Revendications (Revendications pour l'(les) Etat(s) contractant(s) suivant(s): GR)

1. Anticorps produit par l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875) ou fragment de celui-ci, qui est capable de réagir sélectivement avec un antigène particulier ou une famille d'antigènes.

2. Anticorps ou fragment d'anticorps conjugué comprenant un anticorps ou fragment d'anticorps selon la revendication 1 conjugué à un marqueur d'imagerie.

3. Anticorps ou fragment d'anticorps conjugué selon la revendication 2, dans lequel le marqueur d'imagerie est ¹²⁵I, ¹³¹I, ¹²³I, ¹¹¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re ou ^{99m}Tc.

4. Utilisation d'un anticorps ou fragment d'anticorps selon la revendication 1 pour la préparation d'un agent thérapeutique, caractérisée par le fait qu'on conjugue l'anticorps ou fragment d'anticorps à un agent thérapeutique.

5. Utilisation d'un anticorps ou fragment d'anticorps selon la revendication 4, dans laquelle l'agent thérapeutique est un radionuclide, un médicament ou un modificateur de réponse biologique, une toxine ou un autre anticorps.

6. Utilisation d'un anticorps ou fragment d'anticorps selon la revendication 5, dans laquelle le radionuclide est ¹³¹I, ⁹⁰Y, ¹⁰⁵Rh, ⁴⁷Sc, ⁶⁷Cu, ²¹²Bi, ²¹¹At, ⁶⁷Ga, ¹²⁵I, ¹⁸⁶Re, ¹⁸⁸Re, ¹⁷⁷Lu, ^{99m}Tc, ¹⁵³Sm, ¹²³I ou ¹¹¹In.

7. Utilisation d'un anticorps ou fragment d'anticorps conjugué selon la revendication 5, dans laquelle le médicament ou le modificateur de réponse biologique est le méthotrexate, l'adriamycine ou une lymphokine.

8. Séquence d'ADN codant au moins une portion d'un anticorps, qui est capable de réagir sélectivement avec un antigène particulier ou une famille d'antigènes, et qui est contenue dans l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875).

9. Séquence d'ADN selon la revendication 8, dans laquelle la séquence code pour au moins une portion C_{H} d'un gène codant pour IgG₁₋₄, IgM, IgA, IgD ou IgE.

10. Vecteur d'expression biologique fonctionnelle contenant la séquence d'ADN selon l'une des revendications 8 ou 9.

11. Cellule transformée avec le vecteur d'expression biologique fonctionnelle selon la revendication 10.

12. Cellule qui produit un anticorps, comme produit par l'une des lignées cellulaires CH44-1 (ATCC HB 9884), CH44-2 (ATCC HB 9880), CH44-4 (ATCC HB 9877), CH 88-1 (ATCC HB 9882), CH 88-2 (ATCC HB 9881), CH 88-3 (ATCC HB 9876), CH 88-4 (ATCC HB 9874), CH 84-1 (ATCC HB 9883), CH 84-2 (ATCC HB 9879), CH 84-3 (ATCC HB 9878) ou CH 84-4 (ATCC HB 9875).

13. Utilisation de l'anticorps ou fragment d'anticorps de la revendication 1 selon l'une des revendications 4 à 7 dans un support stérile, non toxique, pharmaceutiquement acceptable.

14. Procédé pour la préparation d'un vecteur d'expression recombinant dans lequel on insère la séquence d'ADN, comme définie selon l'une des revendications 8 ou 9, dans un vecteur d'expression.

15. Procédé pour la préparation d'un hôte transformé dans lequel on insère le vecteur d'expression, comme défini selon la revendication 10, dans un hôte convenable.
